# EUROPEAN PATENT SPECIFICATION

(11) **EP 1 812 426 B1**
(45) Date of publication and mention of the grant of the patent: **08.06.2011**
(21) Application number: 05812269.8
(22) Date of filing: 11.10.2005
(51) Int. Cl.: C07D 409/14, C07D 409/12, C07D 209/08, C07D 413/04, C07D 413/14, A61P 9/10, A61K 31/404

(54) **ARYL SULFONAMIDE PERI-SUBSTITUTED BICYCLICS FOR OCCLUSIVE ARTERY DISEASE**
PERI-SUBSTITUIERTE BICYCLISCHE ARYLSULFONAMIDE GEGEN ARTERIELLE VERSCHLUSSKRANKHEITEN
COMPOSÉS BICYCLIQUES DE TYPE ARYLSULFONAMIDE PÉRI-SUBSTITUÉS POUR LE TRAITEMENT DE L' ARTÉRIOPATHIE OBLITÉRANTE

(30) Priority: 12.10.2004 US 618202 P
(43) Date of publication of application: 01.08.2007
(73) Proprietor: DECODE GENETICS, EHF, 101 Reykjavik (IS)
(72) Inventor: SINGH, Jasbir, Woodridge, IL 60517 (US); GURNEY, Mark, Woodridge, IL 60517 (US); HATEGAN, Georgeta, Woodridge, IL 60517 (US)
(74) Representative: Hutchins, Michael Richard
(86) International application number: PCT/US2005/036558
(87) International publication number: WO 2006/044405

(56) References cited:
- EP-A- 1 431 267
- WO-A-2005/062795
- US-A- 5 981 533
- DATABASE CHEMABS CHEMICAL ABSTRACTS SERVICE, COLUMBUS, OHIO, US; 1987, KARPEISKII, A.M. ET AL.: "Pyridoxal analogs of nucleosides" XP002367469 Database accession no. 1988: 221466 & BIOORGANISCHESKAYA KHIMIYA, vol. 13, no. 8, 1987, pages 1059-1065,

## Description

### Field of the Invention

The invention relates to a chemical genus of peri-substituted, bicyclic aryl sulfonamides useful for the treatment and prophylaxis of occlusive artery disease and related prostaglandin-mediated disorders.

### Background of the Invention

Atherosclerosis is the pathology underlying several of mankind's most lethal diseases, such as myocardial infarction and peripheral arterial occlusive disease (PAOD). PAOD represents atherosclerosis of the large and medium arteries of the limbs, particularly to the lower extremities, and includes the aorta and iliac arteries. It often coexists with coronary artery disease and cerebrovascular disease. Persons with PAOD are at increased risk of other vascular events such as myocardial infarction or stroke [Waters, RE, Terjung RL, Peters KG & Annex BH. J. Appl. Physiol. 2004; Ouriel K. Lancet, 2001, 258:1257-64; Kroger, K. Angiology, 2004, 55:135-138]. Clinically significant lesions may gradually narrow the peripheral arteries leading to pain on walking usually relieved by rest (claudication), ischemic ulcers, gangrene, and sometimes limb amputation. Medical therapy is generally ineffective but operations bypassing or replacing the lesion with artificial or venous grafts improve blood flow distally, at least until they become restenosed [Haustein, K.O., Int. J. Clin. Pharmacol. Ther., 35:266 (1997)]. Recently, it has been discovered through human genetic linkage studies that DNA variants of the PTGER3 gene that encodes the prostaglandin E₂ receptor subtype 3 (known as EP3) increase the risk of an individual developing PAOD (see US published application 2003/0157599). Thus, antagonists of prostaglandin E₂ (PGE₂) binding to the EP3 receptor may provide effective treatment or prophylaxis for PAOD.

In response to various extracellular stimuli, prostaglandins are rapidly generated from free arachidonic acid through the consecutive action of the cyclo-oxygenases and synthases. The prostaglandins exert their action in close proximity to the site of their synthesis. To date, eight prostanoid receptors have been cloned and characterized. These receptors are members of the growing class of G-protein-coupled receptors. PGE₂ binds preferentially to the EP1, EP2, EP3, and EP4 receptors; PGD₂ to the DP and FP receptors; PGF_{2α} to the FP and EP3 receptors; PGI₂ to the IP receptor and TXA₂ to the TP receptor. PGE2 binding to the EP3 receptor has been found to play a key role in the regulation of ion transport, smooth muscle contraction of the GI tract, acid secretion, uterine contraction during fertilization and implantation, fever generation and hyperalgesia. The EP3 receptor has been detected in many organs such as the kidney, the gastrointestinal tract, the uterus and the brain. In the cardiovascular system, EP3 is expressed by vascular endothelium and smooth muscle, and at least four isoforms of EP3 are expressed on human platelets [Paul, B.Z., B. Ashby, and S.B. Sheth, Distribution of prostaglandin IP and EP receptor subtypes and isoforms in platelets and human umbilical artery smooth muscle cells. British Journal ofHaematology, 1998. 102(5): p. 1204-11.]

Prostanoids, acting through specific membrane receptors belonging to the superfamily of G protein-coupled receptors (GPCRs) have an essential role in vascular homeostasis, including platelet function regulation. Among the prostanoids, thomboxane A2 (TxA₂) is a potent stimulator of platelet aggregation, whereas prostaglandin (PG) I₂ inhibits their activation. On the other hand, prostaglandin E₂ (PGE₂) has been reported to have a biphasic effect on platelet response, potentiating their aggregation at low concentrations and inhibiting it at higher concentrations. It has been shown that the stimulatory effects of PGE₂ on platelet aggregation are exerted mainly through EP3 receptor, one of the four subtypes of receptors activated by PGE₂.

Local synthesis of prostaglandins in the arterial vessel wall may play a profound role in atherosclerosis. While only COX-1 is present in the healthy vessel wall, both COX-1 and COX-2 are present in arteriosclerotic plaque [Schonbeck, U., et al., Augmented expression of cyclooxygenase-2 in human atherosclerotic lesions. Am J Pathol, 1999. 155(4): p. 1281-91; Cipollone, F., et al., Overexpression of functionally coupled cyclooxygenase-2 and prostaglandin E synthase in symptomatic atherosclerotic plaques as a basis of PGE2-dependent plaque instability. Circulation, 2001. 104(8): p. 921-7]. Their increased expression, together with increased expression of prostaglandin E synthase, may account for the increased production of PGE₂ noted above. In genetically modified mice lacking the low density lipoprotein receptor (LDL-R), formation of atherosclerotic plaque can be reduced by treatment with rofecoxib, a selective inhibitor of COX-2, through reducing production of PGE₂ and other prostaglandins [Burleigh ME, Babaev VR, Oates JA, Harris RC, Gautam S, Riendeau D, Marnett LJ, Morrow JD, Fazio S, Linton MF. Cyclooxygenase-2 promotes early atherosclerotic lesion formation in LDL receptor-deficient mice.Circulation. 2002 Apr 16;105(15):1816-23].

Within the atherosclerotic plaque, vascular smooth muscle cells have been shown to express EP3 receptors and PGE₂ stimulates their proliferation and migration, a hallmark of atherosclerotic plaque formation [Blindt R, Bosserhoff AK, vom Dahl J, Hanrath P, Schror K, Hohlfeld T, Meyer-Kirchrath J. Activation of IP and EP(3) receptors alters cAMP-dependent cell migration.Eur J Pharmacol. 2002 May 24;444(1-2):31-7]. It is, therefore, plausible that chronically inflamed vessels produce sufficient quantities of PGE₂ to activate EP₃ receptors on vascular smooth muscles cells (contributing to atherosclerotic lesion formation) and on platelets (contributing to thrombosis). Locally produced PGE₂ (from platelets themselves, vessel wall components, and inflammatory cells) potentiates platelet aggregation by suboptimal amounts of prothrombotic tissue factors, which might not cause aggregation by themselves, through priming of protein kinase C. The intracellular events triggered by activation of the EP₃ receptor may enhance platelet aggregation by opposing the effect of PGI₂ and enhancing the effects of primary aggregating agents such as collagen. EP₃ receptor activation may therefore contribute to atherosclerosis and the risk of thrombosis observed in pathological states such as vasculitis and PAOD.

Current treatments for PAOD either address increased risk for cardiovascular events such as myocardial infarction and stroke, or provide symptomatic relief for claudication. All of these treatments affect platelet function. Treatments reducing risk for cardiovascular events include low dose asprin (sufficient to reduce platelet aggregation while still permitting the production of PGI₂ by the vessel wall) and inhibitors of the platelet adenosine diphosphate receptor inhibitor (clopidogrel). Binding of adenosine diphosphate to the platelet adenosine diphosphate receptor causes a drop in platelet cAMP with consequent platelet activation and aggregation. Treatments providing symptomatic relief from claudication include platelet phosphodiesterase type 3 inhibitors such as cilostazol which act to increase intracellular levels of cAMP. Inhibitors of the platelet adenosine diphosphate receptor or the platelet phosphodiesterase type 3 act directly or indirectly to increase the content of cAMP in platelets, thereby inhibiting platelet activation and consequent aggregation with thrombus formation. PGE₂ binding to EP3 acts to decrease cAMP, therefore an antagonist of PGE₂ binding to the EP3 receptor, by opposing the PGE₂ -dependent decrease in cAMP needed to induce platelet activation and consequent aggregation, or by opposing the PGE₂ -dependent decrease in vascular smooth muscle cell cAMP needed to stimulate migration, might be expected to provide therapeutic benefit in PAOD. Such an antagonist may also be disease-modifying by inhibiting or reducing atherosclerotic plaque formation.

Prostaglandins furthermore have been implicated in a range of disease states including pain, fever or inflammation associated with rheumatic fever, influenza or other viral infections, common cold, low back and neck pain, skeletal pain, post-partum pain, dysmenorrhea, headache, migraine, toothache, sprains and strains, myositis, neuralgia, synovitis, arthritis, including rheumatoid arthritis, degenerative joint diseases (osteoarthritis), gout and ankylosing spondylitis, bursitis, burns including radiation and corrosive chemical injuries, sunburns, pain following surgical and dental procedures, immune and autoimmune diseases; cellular neoplastic transformations or metastic tumor growth; diabetic retinopathy, tumor angiogenesis; prostanoid-induced smooth muscle contraction associated with dysmenorrhea, premature labor, asthma or eosinophil related disorders; Alzheimer's disease; glaucoma; bone loss; osteoporosis; Paget's disease;
peptic ulcers, gastritis, regional enteritis, ulcerative colitis, diverticulitis or other gastrointestinal lesions; GI bleeding; coagulation disorders selected from hypoprothrombinemia, hemophilia and other bleeding problems; and kidney disease.

While circulating levels of prostanoids are extremely low in healthy individuals [FitzGerald GA, Brash AR, Falardeau P & Oates JA. JCI 1981 68:12472-1275], the local concentration of PGE₂ can dramatically increase in inflammatory states. For example, the local production of PGE₂ was shown in vitro to increase more than 30-fold in aortoiliac occlusive disease [Reilly J, Miralles M, Wester W & Sicard G. Surgery, 1999, 126:624-628]. It is, therefore, plausible that chronically inflamed vessels produce sufficient quantities of PGE₂ to activate EP₃ receptors on platelets. In this environment, the intracellular events triggered by activation of the EP₃ receptor may enhance platelet aggregation by opposing the effect of PGI₂ and enhancing the effects of primary aggregating agents such as ADP. EP₃ receptor activation may therefore contribute to the thrombosis observed in pathological states such as vasculitis and atherosclerosis. Peripheral Arterial Occlusive Disease (PAOD) is an atherosclerotic illness that affects primarily the elderly as a consequence of occlusion of the lumen of peripheral arteries, mainly the femoral artery and it is associated with an increased risk of vascular events as myocardial infraction or stroke [Waters, RE, Terjung RL, Peters KG & Annex BH. J. Appl. Physiol. 2004; Ouriel K. Lancet, 2001, 258:1257-64; Kroger, K. Angiology, 2004, 55:135-138]. Several clinical studies have shown that treatment with prostaglandins improves PAOD symptoms [Reiter M, Bucek R, Stumpflen A & Minar E. Cochrane Database Syst. Rev. 2004, 1:CD000986; Bandiera G, Forletta M, Di Paola FM, Cirielli C. Int. Angiol. 2003, 22:58-63; Matsui K, Ikeda U, Murakami Y, Yoshioka T, Shimada K. Am. Heart J. 2003, 145:330-333] supporting the linkage between PAOD and prostanoid receptor function.

Ortho-substituted phenyl acylsulfonamides and their utility for treating prostaglandin-mediated disorders are described in US patent 6,242,493 and in two articles by Juteau et al. [BioOrg. Med. Chem. 9, 1977-1984 (2001)] and Gallant et al. [BioOrg. Med. Chem. Let. 12, 2583-2586 (2002)]. EP 1 431 267 A2 discloses carboxylic acid derivatives and their use as modulators of the EP₃ and/or EP₄ receptor.

### Summary of the Invention

In one aspect the invention relates to compounds of formula I

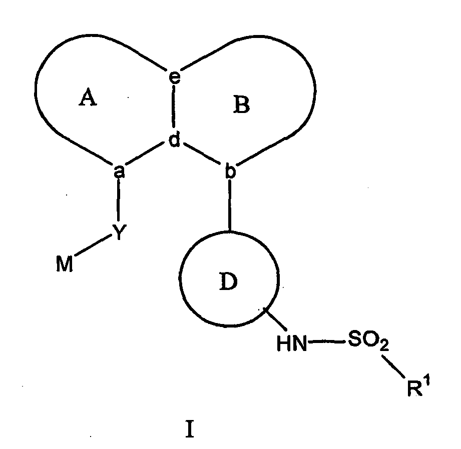

wherein A and B represent a pair of fused 5-, 6- or 7-membered rings. The fused A/B ring system may contain from zero to four heteroatoms chosen from nitrogen, oxygen and sulfur and may be additionally substituted with from zero to four substituents chosen independently from halogen, -OH, loweralkyl, - O-loweralkyl, fluoroloweralkyl, -O-lowerfluoroalkyl, methylenedioxy, ethylenedioxy, alkoxy-loweralkyl, hydroxyloweralkyl, oxo, oxide, -CN, nitro, -S-loweralkyl, amino, loweralkylamino, diloweralkylamino, diloweralkylaminoalkyl, carboxy, carboalkoxy, acyl, carboxamido, loweralkylsulfoxide, acylamino, phenyl, benzyl, *spiro*thiazolidinyl, phenoxy and benzyloxy. The nodes represented by a and b are the points of attachment of residues Y and D respectively, and a and b are in a peri relationship to one another on the fused A/B ring system. The nodes represented by d and e are points of fusion between ring A and ring B in the fused A/B ring system. Each of the nodes a, b, d and e may be either carbon or nitrogen.
D is an aryl or heteroaryl ring system, which may be additionally substituted with from zero to four substituents. The substiutents are chosen independently from halogen, -OH, loweralkyl, -O-loweralkyl, fluoroloweralkyl, -O-lowerfluoroalkyl, methylenedioxy, ethylenedioxy, alkoxy-loweralkyl, hydroxyloweralkyl, -CN, nitro, -S-loweralkyl, amino, loweralkylamino, diloweralkylamino, diloweralkylaminoalkyl, carboxy, carboalkoxy, acyl, carboxamido, loweralkylsulfoxide, acylamino, phenyl, benzyl, phenoxy and benzyloxy.
Y is a linker comprising from zero to 8 atoms in a chain.
M is chosen from aryl, substituted aryl, heterocyclyl and substituted heteroaryl;
R¹ is chosen from aryl, substituted aryl, heteroaryl, substituted heteroaryl and CF₃; and
when Y is a single atom linker, R¹ may additionally be lower alkyl.

In a second aspect the invention relates to pharmaceutical formulations comprising a pharmaceutically acceptable carrier and a compound as above, or an ester, a pharmaceutically acceptable salt or a hydrate of the compound.

The application further discloses methods for the treatment or prophylaxis of a prostaglandin-mediated disease or condition. The methods comprise administering to a mammal a therapeutically effective amount of a compound described herein. The disease or condition may be, for example, fever or inflammation associated with rheumatic fever, influenza or other viral infections, migraine, common cold, dysmenorrhea, sprains and strains, myositis, neuralgia, synovitis, arthritis, including rheumatoid arthritis, degenerative joint diseases (osteoarthritis), gout and ankylosing spondylitis, bursitis, burns including radiation and corrosive chemical injuries, sunburns, immune and autoimmune diseases and pain (e.g. low back and neck pain, skeletal pain, post-partum pain, headache, toothache, pain following surgical and dental procedures). EP3 antagonist compounds of the invention that penetrate the CNS are especially suited for pain management.

Compounds of the invention, which inhibit platelet aggregation and increase regional blood flow, are useful for treating primary thromboembolism, thrombosis and occlusive vascular diseases. The compounds can be used advantageously in combination with other platelet aggregation inhibitors and with inhibitors of cholesterol biosynthesis or uptake. The compounds can also be used advantageously in combination with a cyclooxygenase-2 inhibitor to treat inflammatory conditions.

Other diseases or conditions may also be treated, for example, cellular neoplastic transformations or metastic tumor growth; diabetic retinopathy, tumor angiogenesis; prostanoid-induced smooth muscle contraction associated with dysmenorrhea, premature labor, asthma or eosinophil related disorders; Alzheimer's disease; glaucoma; bone loss, osteoporosis or Paget's disease; peptic ulcers, gastritis, regional enteritis, ulcerative colitis, diverticulitis or other gastrointestinal lesions; GI bleeding; coagulation disorders selected from hypoprothrombinemia, hemophilia and other bleeding problems and kidney disease. The method aspect of the invention also includes methods for the promotion of bone formation, for cytoprotection and for reducing plaque in the treatment of atherosclerosis.

In a fourth aspect, the invention relates to methods for screening for selective prostanoid receptors, particularly EP3 ligands.

### Detailed Description of the Invention

Compounds of the genus represented by formula I above are antagonists at the EP3 receptor. As such they have utility in treating and preventing prostaglandin-mediated conditions, as described above, particularly for such conditions as occlusive vascular disease.

Compositions of the invention comprise an effective dose or a pharmaceutically effective amount or a therapeutically effective amount of a compound described above and may additionally comprise other therapeutic agents, such as platelet aggregation inhibitors (tirofiban, dipyridamole, clopidogrel, ticlopidine and the like); HMG-CoA reductase inhibitors (lovastatin, simvastatin, pravastatin, rosuvastatin, mevastatin, atorvastatin, cerivastatin, pitavastatin, fluvastatin and the like) and cyclooxygenase inhibitors. A further listing of non-limiting examples of antihyperlipidemic agents that may be used in combination with the compounds of the present invention may be found in columns 5-6 of US patent 6,498,156. Preferred cyclooxygenase-2 inhibitors are those that are selective for cyclooxygenase-2 over cyclooxygenase-1. Preferred cyclooxygenase-2 inhibitors include rofecoxib, meloxicam, celecoxib, etoricoxib, lumiracoxib, valdecoxib, parecoxib, cimicoxib, diclofenac, sulindac, etodolac, ketoralac, ketoprofen, piroxicam and LAS-34475, although the invention is not restricted to these or other known cyclooxygenase-2 inhibitors.

Methods of the invention parallel the compositions and formulations. The methods comprise administering to a patient in need of treatment a therapeutically effective amount of a peri-substituted, fused A/B ring compound according to the invention. The present invention is also directed to methods for screening for selective prostanoid receptor agonists and antagonists. Prostanoid receptors include EP1, EP2, EP3, EP4, IP and FP receptors. Selective EP3 ligands are of great interest, for which the method comprises bringing a labeled compound according to the invention into contact with a cloned human EP3 receptor and measuring its displacement by a test compound.

A genus according to the invention includes compounds of formula I: 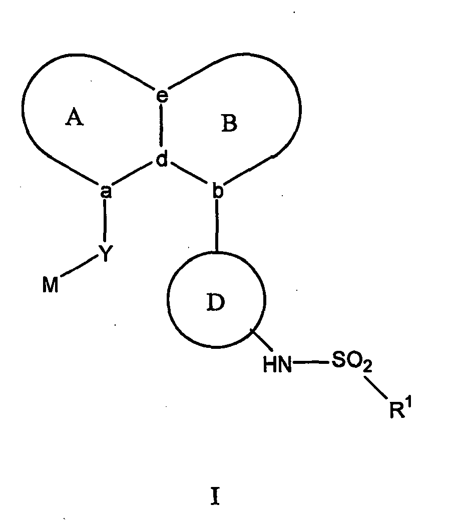 wherein A and B represent a pair of fused 5-, 6- or 7-membered rings and D is an aryl or heteroaryl ring system. In one subgenus, D is phenyl, which may be substituted or unsubstituted. In another subgenus, D is naphthyl, which may be substituted or unsubstituted. In a third subgenus, D is monocyclic heteroaryl, which may be substituted or unsubstituted. In a fourth subgenus, D is bicyclic heteroaryl, which may be substituted or unsubstituted. In one embodiment, R¹ is chosen from phenyl, substituted phenyl, 5-membered ring heteroaryl, substituted 5-membered ring heteroaryl and CF₃.

Each of A and B represents independently a 5-, 6- or 7-membered ring. The fused A/B ring system contains from zero to four heteroatoms chosen from nitrogen, oxygen and sulfur, and the rings are additionally substituted with from zero to four substituents. Suitable substituents include halogen, -OH, loweralkyl, -O-loweralkyl, fluoroloweralkyl, O lowerfluoroalkyl, methylenedioxy, ethylenedioxy, alkoxy-loweralkyl, hydroxyloweralkyl, oxo, oxide, -CN, nitro, -S-loweralkyl, amino, loweralkylamino, diloweralkylamino, diloweralkylaminoalkyl, carboxy, carboalkoxy, orthoesters, acyl, carboxamido, loweralkylsulfoxide, acylamino, phenyl, benzyl, spirothiazolidinyl, phenoxy and benzyloxy. Since the fused A/B ring system may include nitrogen or sulfur, the substituents may include oxides, e.g. N→O and S→O.

In one subgenus, the A/B ring system is a pair of fused 5-membered rings:

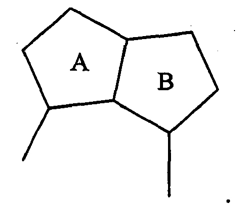

Examples of such 5/5 ring systems are:

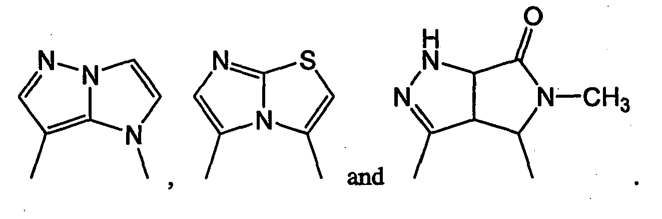

In another subgenus the A/B ring system is a pair of fused 6-membered rings:

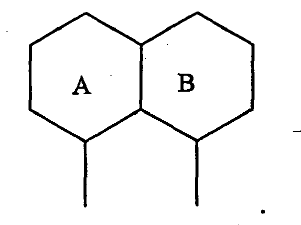

Examples of such 6/6 ring systems are: 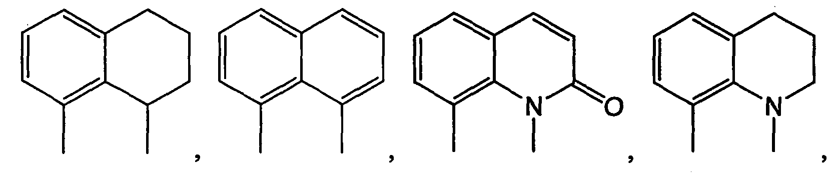 and

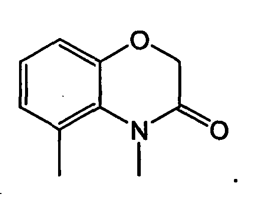

In another subgenus, the A/B ring system is a fused 5-and 6-membered ring pair:

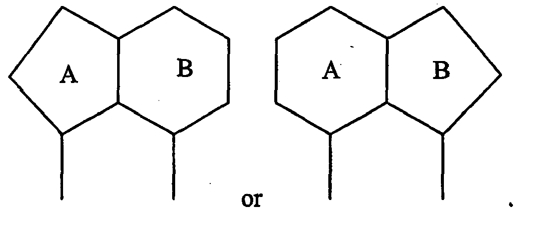

Examples of such 5/6 ring systems are indoles, indolines, indolones, isatins, benzimidazoles, benzoxazolinones, benzofurans and indazoles:

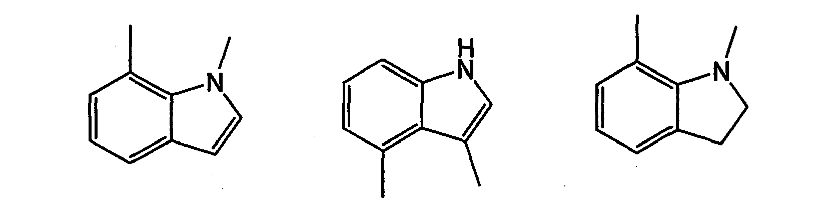

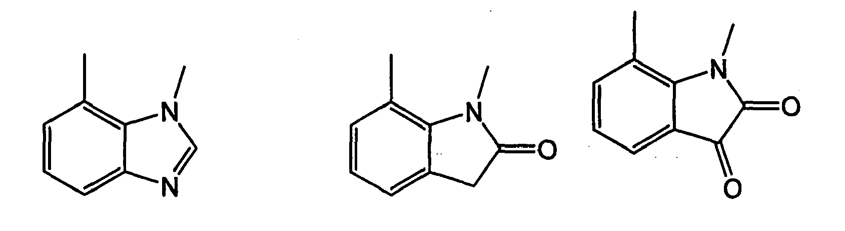

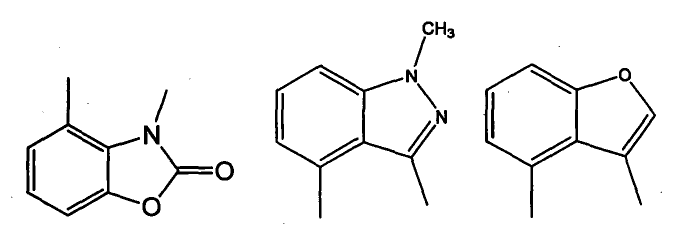

As indicated earlier, the ring systems may be substituted, for example:

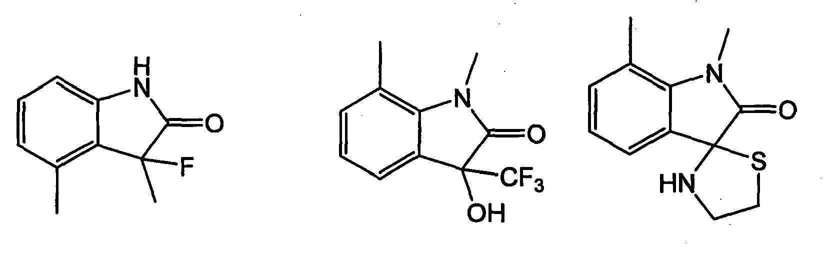

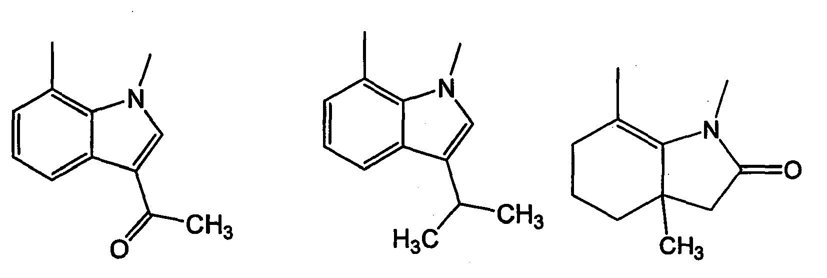

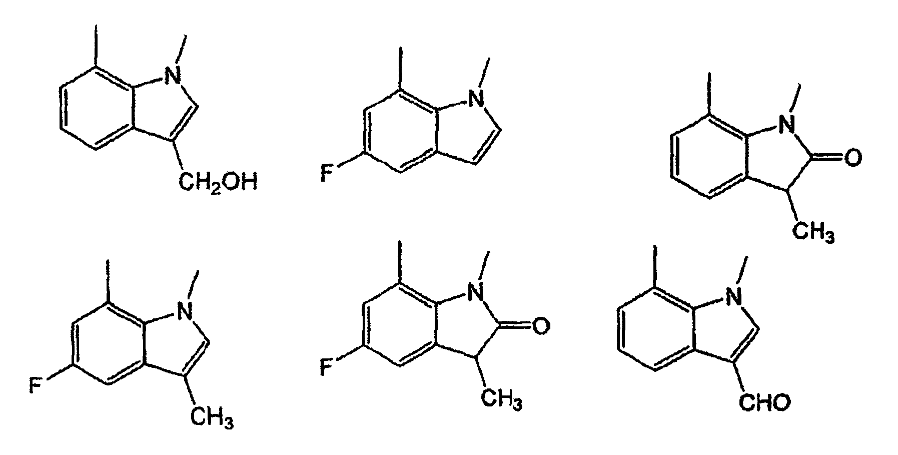

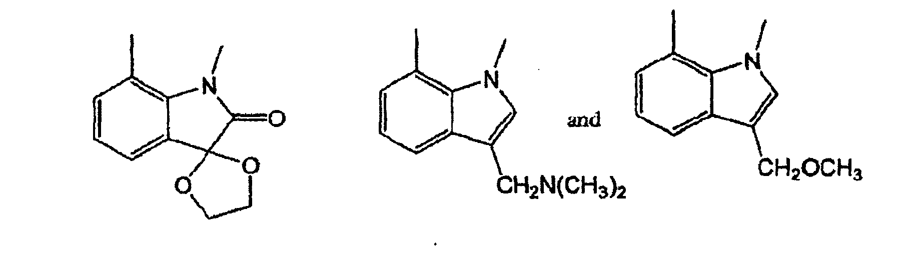

Y is a linker comprising from zero to 8 atoms in a chain. Preferably Y is from C₁ to C₈ alkyl in which one or two -CH₂- may be replaced by -O-, -C(=Q)-, -CH=CH-, -CF₂-, -S-, -SO-, -SO₂-, -NH- or -N(alkyl)-. More preferably, Y is a two-atom chain, i.e. C₁ or C₂ alkyl in which one or both -CH₂- may be replaced by the groups named above. In one embodiment, Y is chosen from -CH₂-, -O-, -OCH₂-, -S-, -SO-, and -SO₂-. The left-hand bond indicates the point of attachment to ring A or B.
M is chosen from aryl, substituted aryl, heterocyclyl and substituted heteroaryl, more preferably from phenyl, substituted phenyl, naphthyl, substituted naphthyl, heteroaryl and substituted heteroaryl.

The compounds may be presented as salts. The term "pharmaceutically acceptable salt" refers to salts whose counter ion derives from pharmaceutically acceptable non-toxic acids and bases. Suitable pharmaceutically acceptable base addition salts for the compounds of the present invention include, but are not limited to, metallic salts made from aluminum, calcium, lithium, magnesium, potassium, sodium and zinc or organic salts made from lysine, N,N-dialkyl amino acid derivatives (e.g. N,N-dimethylglycine, piperidine-1-acetic acid and morpholine-4-acetic acid), N,N'-dibenzylethylenediamine, chloroprocaine, choline, diethanolamine, ethylenediamine, meglumine (N-methylglucamine) and procaine. When the compounds contain a basic residue, suitable pharmaceutically acceptable base addition salts for the compounds of the present invention include inorganic acids and organic acids. Examples include acetate, benzenesulfonate (besylate), benzoate, bicarbonate, bisulfate, carbonate, camphorsulfonate, citrate, ethanesulfonate, fumarate, gluconate, glutamate, bromide, chloride, isethionate, lactate, maleate, malate, mandelate, methanesulfonate, mucate, nitrate, pamoate, pantothenate, phosphate, succinate, sulfate, tartrate, p-toluenesulfonate, and the like.
In another embodiment, the invention provides a compound of the formula: 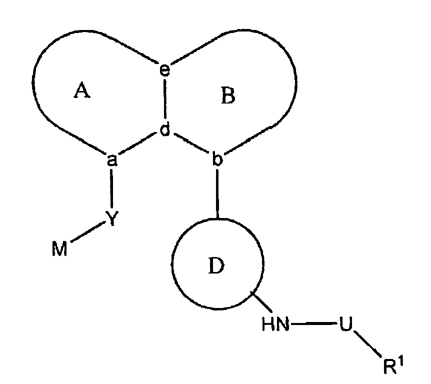 wherein
A and B represent a pair of fused 5-, 6- or 7-membered rings, said fused A/B ring system containing from zero to four heteroatoms chosen from nitrogen, oxygen and sulfur and said rings additionally substituted with from zero to four substituents chosen independently from halogen, -OH, (C₁-C₆)alkyl, (C₃-C₆)cycloalkyl, -O-(C₁-C₆)alkyl, -O-(C₃-C₆)cycloalkyl, fluoro(C₁-C₆)alkyl, fluoro(C₃-C₆)cycloalkyl, -O-(C₁-C₆)fluoroalkyl, -O-(C₃-C₆)cyclofluoroalkyl, methylenedioxy, ethylenedioxy, alkoxy-(C₁-C₆)alkyl, alkoxy-(C₃-C₆)cycloalkyl, hydroxy(C₁-C₆)alkyl, hydroxy(C₃-C₆)cycloalkyl, oxo, oxide, -CN, nitro, -S-(C₁-C₆)alkyl, -S-(C₃-C₆)cycloalkyl, amino, (C₁-C₆)alkylamino, (C₃-C₆)cycloalkylamino, di(C₁-C₆)alkylamino, di(C₃-C₆)cycloalkylamino, di(C₁-C₆)alkylaminoalkyl, di(C₁-C₆)alkylaminocycloalkyl, di(C₃-C₆)cycloalkylaminoalkyl, di(C₃-C₆)cycloalkylaminocycloalkyl, carboxy, carboalkoxy, acyl, carboxamido, (C₁-C₆)alkylsulfoxide, (C₃-C₆)cycloalkylsulfoxide, acylamino, phenyl, benzyl, *spiro*thiazolidinyl, phenoxy and benzyloxy;
a and b represent points of attachment of residues Y and D respectively and a and b are in a peri relationship to one another on said fused A/B ring system;
d and e represent points of fusion between ring A and ring B in said fused A/B ring system; U is C=O;
D is an aryl or heteroaryl ring system, said ring system additionally substituted with from zero to four substituents chosen independently from halogen, -OH, (C₁-C₆)alkyl, (C₃-C₆)cycloalkyl, -O-(C₁-C₆)alkyl, -O-(C₃-C₆)cycloalkyl, fluoro(C₁-C₆)alkyl, fluoro(C₃-C₆)cycloalkyl, -O-(C₁-C₆)fluoroalkyl, -O-(C₃-C₆)cyclofluoroalkyl, methylenedioxy, ethylenedioxy, alkoxy-(C₁-C₆)alkyl, alkoxy-(C₃-C₆)cycloalkyl, hydroxy(C₁-C₆)alkyl, hydroxy(C₃-C₆)cycloalkyl, -CN, nitro, -S-(C₁-C₆)alkyl, -S-(C₃-C₆)cycloalkyl, amino, (C₁-C₆)alkylamino, (C₃-C₆)cycloalkylamino, di(C₁-C₆)alkylamino, di(C₃-C₆)cycloalkylamino, di(C₁-C₆)alkylaminoalkyl, di(C₁-C₆)alkylaminocycloalkyl, di(C₃-C₆)cycloalkylaminoalkyl, di(C₃-C₆)cycloalkylaminocycloalkyl, carboxy, carboalkoxy, acyl, carboxamido, (C₁-C₆)alkylsulfoxide, (C₃-C₆)cycloalkylsulfoxide, acylamino, phenyl, benzyl, phenoxy and benzyloxy;
Y is -CH₂-;
M is chosen from aryl, substituted aryl, heterocyclyl and substituted heteroaryl;
R¹ is chosen from aryl, substituted aryl, heteroaryl, substituted heteroaryl, CF₃, (C₁-C₆)alkyl, and (C₃-C₆)cycloalkyl.
In one embodiment, the A/B ring system is an indole. Within this embodiment, M may be aryl or substituted aryl. Also within this embodiment, D may be phenyl or oxadiazolyl.
When D is phenyl or oxadiazolyl, R¹ may be chosen from phenyl, substituted phenyl, 5-membered ring heteroaryl, substituted 5-membered ring heteroaryl, CH₃ and CF₃. When D is Definitions

Throughout this specification the terms and substituents retain their definitions.

Alkyl is intended to include linear, branched, or cyclic hydrocarbon structures and combinations thereof. Lower alkyl refers to alkyl groups of from 1 to 6 carbon atoms. Examples of lower alkyl groups include methyl, ethyl, propyl, isopropyl, butyl, s-and t-butyl and the like. Preferred alkyl and alkylene groups are those of C₂₀ or below. Cycloalkyl is a subset of alkyl and includes cyclic hydrocarbon groups of from 3 to 8 carbon atoms. Examples of cycloalkyl groups include c-propyl, c-butyl, c-pentyl, norbornyl, adamantyl and the like.

C₁ to C₂₀ Hydrocarbon includes alkyl, cycloalkyl, alkenyl, alkynyl, aryl and combinations thereof. Examples include benzyl, phenethyl, cyclohexylmethyl, camphoryl and naphthylethyl.

Alkoxy or alkoxyl refers to groups of from 1 to 8 carbon atoms of a straight, branched, cyclic configuration and combinations thereof attached to the parent structure through an oxygen. Examples include methoxy, ethoxy, propoxy, isopropoxy, cyclopropyloxy, cyclohexyloxy and the like. Lower-alkoxy refers to groups containing one to four carbons.

Oxaalkyl refers to alkyl residues in which one or more carbons (and their associated hydrogens) have been replaced by oxygen. Examples include methoxypropoxy, 3,6,9-trioxadecyl and the like. The term oxaalkyl is intended as it is understood in the art [see Naming and Indexing of Chemical Substances for Chemical Abstracts, published by the American Chemical Society, ¶196, but without the restriction of ¶127(a)], i.e. it refers to compounds in which the oxygen is bonded via a single bond to its adjacent atoms (forming ether bonds). Similarly, thiaalkyl and azaalkyl refer to alkyl residues in which one or more carbons have been replaced by sulfur or nitrogen, respectively. Examples include ethylaminoethyl and methylthiopropyl. The term "oxo" referring to a substituent intends double-bonded oxygen (carbonyl). Thus, for example, a 2-oxoquinoline of the invention would be:

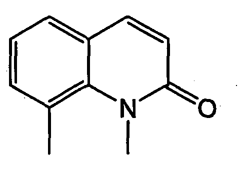

Acyl refers to groups of from 1 to 8 carbon atoms of a straight, branched, cyclic configuration, saturated, unsaturated and aromatic and combinations thereof, attached to the parent structure through a carbonyl functionality. One or more carbons in the acyl residue may be replaced by nitrogen, oxygen or sulfur as long as the point of attachment to the parent remains at the carbonyl. Examples include formyl, acetyl, propionyl, isobutyryl, t-butoxycarbonyl, benzoyl, benzyloxycarbonyl and the like. Lower-acyl refers to groups containing one to four carbons.

Aryl and heteroaryl mean a 5- or 6-membered aromatic or heteroaromatic ring containing 0-3 heteroatoms selected from O, N, or S; a bicyclic 9- or 10-membered aromatic or heteroaromatic ring system containing 0-3 heteroatoms selected from O, N, or S; or a tricyclic 13- or 14-membered aromatic or heteroaromatic ring system containing 0-3 heteroatoms selected from O, N, or S. Aromatic 6- to 14-membered carbocyclic rings include, *e.g*., benzene, naphthalene, indane, tetralin, and fluorene and the 5- to 10-membered aromatic heterocyclic rings include, *e.g*., imidazole, pyridine, indole, thiophene, benzopyranone, thiazole, furan, benzimidazole, quinoline, isoquinoline, quinoxaline, pyrimidine, pyrazine, tetrazole and pyrazole.

Arylalkyl means an alkyl residue attached to an aryl ring. Examples are benzyl, phenethyl and the like.

Substituted alkyl, aryl, cycloalkyl, heterocyclyl etc. refer to alkyl, aryl, cycloalkyl, or heterocyclyl wherein up to three H atoms in each residue are replaced with halogen, lower alkyl, haloalkyl, hydroxy, loweralkoxy, carboxy, carboalkoxy (also referred to as alkoxycarbonyl), carboxamido (also referred to as alkylaminocarbonyl), cyano, carbonyl, nitro, amino, alkylamino, dialkylamino, mercapto, alkylthio, sulfoxide, sulfone, acylamino, amidino, phenyl, benzyl, heteroaryl, phenoxy, benzyloxy, or heteroaryloxy. In the claims below, methylenedioxy and ethylenedioxy are mentioned as substituents. While methylenedioxy is attached at adjacent carbons on the ring, ethylenedioxy can be attached either at adjacent carbons on the ring or at the same carbon, forming a *spiro*dioxole (ketal), analogous to the *spiro*thiazolidinyl. The various options are illustrated in compounds 114, 144 and 160.

The term "halogen" means fluorine, chlorine, bromine or iodine.

The term "prodrug" refers to a compound that is made more active in vivo. Activation *in vivo* may come about by chemical action or through the intermediacy of enzymes. Microflora in the GI tract may also contribute to activation *in vivo.*

In the characterization of the variables, it is recited that A and B represent a pair of fused 5-, 6- or 7-membered rings and that the fused A/B ring system may contain from zero to four heteroatoms chosen from nitrogen, oxygen and sulfur. It is intended that these rings may exhibit various degrees of unsaturation from fully saturated to aromatic. Aromatic and partially unsaturated rings are preferred.

In the characterization of the variables, it is recited that the fused rings may be additionally substituted with from zero to four substituents chosen independently from a list of variable definitions. The structure below illustrates the intent of that language. In this example, the fused rings are substituted with three substituents: -CH₃, -OH and oxo:

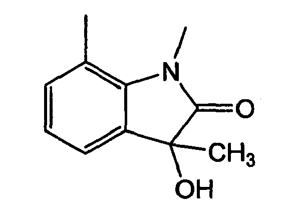

It will be recognized that the compounds of this invention can exist in radiolabeled form, i.e., the compounds may contain one or more atoms containing an atomic mass or mass number different from the atomic mass or mass number usually found in nature. Radioisotopes of hydrogen, carbon, phosphorous, fluorine, and chlorine include ²H, ³H, ¹³C, ¹⁴C, ¹⁵N, ³⁵S, ¹⁸F, and ³⁶Cl, respectively. Compounds that contain those radioisotopes and/or other radioisotopes of other atoms are within the scope of this invention. Tritiated, i.e. ³H, and carbon-14, i.e., ¹⁴C, radioisotopes are particularly preferred for their ease in preparation and detectability. Radiolabeled compounds of formula Ia of this invention and prodrugs thereof can generally be prepared by methods well known to those skilled in the art. Conveniently, such radiolabeled compounds can be prepared by carrying out the procedures disclosed in the Examples and Schemes by substituting a readily available radiolabeled reagent for a non-radiolabeled reagent.

As used herein, and as would be understood by the person of skill in the art, the recitation of "a compound" is intended to include salts, solvates, co-crystals and inclusion complexes of that compound.

The term "solvate" refers to a compound of Formula I in the solid state, wherein molecules of a suitable solvent are incorporated in the crystal lattice. A suitable solvent for therapeutic administration is physiologically tolerable at the dosage administered. Examples of suitable solvents for therapeutic administration are ethanol and water. When water is the solvent, the solvate is referred to as a hydrate. In general, solvates are formed by dissolving the compound in the appropriate solvent and isolating the solvate by cooling or using an antisolvent. The solvate is typically dried or azeotroped under ambient conditions. Co-crystals are combinations of two or more distinct molecules arranged to create a unique crystal form whose physical properties are different from those of its pure constituents. Pharmaceutical co-crystals have recently become of considerable interest for improving the solubility, formulation and bioavailability of such drugs as itraconazole [see Remenar et al. J.Am.Chem.Soc. 125, 8456-8457 (2003)] and fluoxetine. Inclusion complexes are described in Remington: The Science and Practice of Pharmacy 19th Ed. (1995) volume 1, page 176-177. The most commonly employed inclusion complexes are those with cyclodextrins, and all cyclodextrin complexes, natural and synthetic, with or without added additives and polymer(s), as described in US Patents 5,324,718 and 5,472,954, are specifically encompassed within the claims.

The terms "methods of treating or preventing" mean amelioration, prevention or relief from the symptoms and/or effects associated with lipid disorders. The term "preventing" as used herein refers to administering a medicament beforehand to forestall or obtund an acute episode. The person of ordinary skill in the medical art (to which the present method claims are directed) recognizes that the term "prevent" is not an absolute term. In the medical art it is understood to refer to the prophylactic administration of a drug to substantially diminish the likelihood or seriousness of a condition, and this is the sense intended in applicants' claims. As used herein, reference to "treatment" of a patient is intended to include prophylaxis. Throughout this application, various references are referred to.

The term "mammal" is used in its dictionary sense. Humans are included in the group of mammals, and humans would be the preferred subjects of the methods of treatment.

The compounds described herein may contain asymmetric centers and may thus give rise to enantiomers, diastereomers, and other stereoisomeric forms. Each chiral center may be defined, in terms of absolute stereochemistry, as (R)-or (S)-. The present invention is meant to include all such possible isomers, as well as, their racemic and optically pure forms. Optically active (R)- and (S)-, or (D)- and (L)- isomers may be prepared using chiral synthons or chiral reagents, or resolved using conventional techniques. When the compounds described herein contain olefinic double bonds or other centers of geometric asymmetry, and unless specified otherwise, it is intended that the compounds include both E and Z geometric isomers. Likewise, all tautomeric forms are also intended to be included.

The graphic representations of racemic, ambiscalemic and scalemic or enantiomerically pure compounds used herein are taken from Maehr J. Chem. Ed. 62, 114-120 (1985): solid and broken wedges are used to denote the absolute configuration of a chiral element; wavy lines and single thin lines indicate disavowal of any stereochemical implication which the bond it represents could generate; solid and broken bold lines are geometric descriptors indicating the relative configuration shown but denoting racemic character; and wedge outlines and dotted or broken lines denote enantiomerically pure compounds of indeterminate absolute configuration.

The configuration of any carbon-carbon double bond appearing herein is selected for convenience only and unless explicitly stated, is not intended to designate a particular configuration. Thus a carbon-carbon double bond depicted arbitrarily as E may be Z, E, or a mixture of the two in any proportion.

Terminology related to "protecting", "deprotecting" and "protected" functionalities occurs throughout this application. Such terminology is well understood by persons of skill in the art and is used in the context of processes which involve sequential treatment with a series of reagents. In that context, a protecting group refers to a group that is used to mask a functionality during a process step in which it would otherwise react, but in which reaction is undesirable. The protecting group prevents reaction at that step, but may be subsequently removed to expose the original functionality. The removal or "deprotection" occurs after the completion of the reaction or reactions in which the functionality would interfere. Thus, when a sequence of reagents is specified, as it is in the processes of the invention, the person of ordinary skill can readily envision those groups that would be suitable as "protecting groups". Suitable groups for that purpose are discussed in standard textbooks in the field of chemistry, such as Protective Groups in Organic Synthesis by T.W.Greene [John Wiley & Sons, New York, 1991]. Particular attention is drawn to the chapters entitled "Protection for the Hydroxyl Group, Including 1,2- and 1,3-Diols" (pages 10-86).

The abbreviations Me, Et, Ph, Tf, Ts and Ms represent methyl, ethyl, phenyl, trifluoromethanesulfonyl, toluenesulfonyl and methanesulfonyl respectively. A comprehensive list of abbreviations utilized by organic chemists (i.e. persons of ordinary skill in the art) appears in the first issue of each volume of the Journal of Organic Chemistry.

While it may be possible for the compounds of formula I to be administered as the raw chemical, it is preferable to present them as a pharmaceutical composition. According to a further aspect, the present invention provides a pharmaceutical composition comprising a compound of formula I, or a pharmaceutically acceptable salt or solvate thereof, together with one or more pharmaceutically carriers thereof and optionally one or more other therapeutic ingredients. The carrier(s) must be "acceptable" in the sense of being compatible with the other ingredients of the formulation and not deleterious to the recipient thereof.

The formulations include those suitable for oral, parenteral (including subcutaneous, intradermal, intramuscular, intravenous and intraarticular), rectal and topical (including dermal, buccal, sublingual and intraocular) administration. The most suitable route may depend upon the condition and disorder of the recipient. The formulations may conveniently be presented in unit dosage form and may be prepared by any of the methods well known in the art of pharmacy. All methods include the step of bringing into association a compound of formula I or a pharmaceutically acceptable salt or solvate thereof ("active ingredient") with the carrier, which constitutes one or more accessory ingredients. In general, the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder (including micronized and nanoparticulate powders) or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Molded tablets may be made by molding in a suitable machine a mixture of the powdered compound moistened with an inert liquid diluent. The tablets may optionally be coated or scored and may be formulated so as to provide sustained, delayed or controlled release of the active ingredient therein.

The pharmaceutical compositions may include a "pharmaceutically acceptable inert carrier", and this expression is intended to include one or more inert excipients, which include starches, polyols, granulating agents, microcrystalline cellulose, diluents, lubricants, binders, disintegrating agents, and the like. If desired, tablet dosages of the disclosed compositions may be coated by standard aqueous or nonaqueous techniques, "Pharmaceutically acceptable carrier" also encompasses controlled release means.

Compositions of the present invention may also optionally include other therapeutic ingredients, anti-caking agents, preservatives, sweetening agents, colorants, flavors, desiccants, plasticizers, dyes, and the like. Any such optional ingredient must, of course, be compatible with the compound of the invention to insure the stability of the formulation.

The dose range for adult humans is generally from 0.1 µg to 10 g/day orally. Tablets or other forms of presentation provided in discrete units may conveniently contain an amount of compound of the invention which is effective at such dosage or as a multiple of the same, for instance, units containing 0.1 mg to 500 mg, usually around 5 mg to 200 mg. The precise amount of compound administered to a patient will be the responsibility of the attendant physician. However, the dose employed will depend on a number of factors, including the age and sex of the patient, the precise disorder being treated, and its severity. The frequency of administration will depend on the pharmacodynamics of the individual compound and the formulation of the dosage form., which may be optimized by methods well known in the art (e.g. controlled or extended release tablets, enteric coating etc.)

Combination therapy can be achieved by administering two or more agents, each of which is formulated and administered separately, or by administering two or more agents in a single formulation. Other combinations are also encompassed by combination therapy. For example, two agents can be formulated together and administered in conjunction with a separate formulation containing a third agent. While the two or more agents in the combination therapy can be administered simultaneously, they need not be.

Approximately three hundred compounds representative of the overall concept have been synthesized. Their structures are shown in two copending applications filed of even date herewith under the titles "SULFONAMIDE PERI-SUBSTITUTED BICYCLICS FOR OCCLUSIVE ARTERY DISEASE" and "CARBOXYLIC ACID PERI-SUBSTITUTED BICYCLICS FOR OCCLUSIVE ARTERY DISEASE". Examples of the subgenus claimed in this application include B01, B03, B04 and B13:

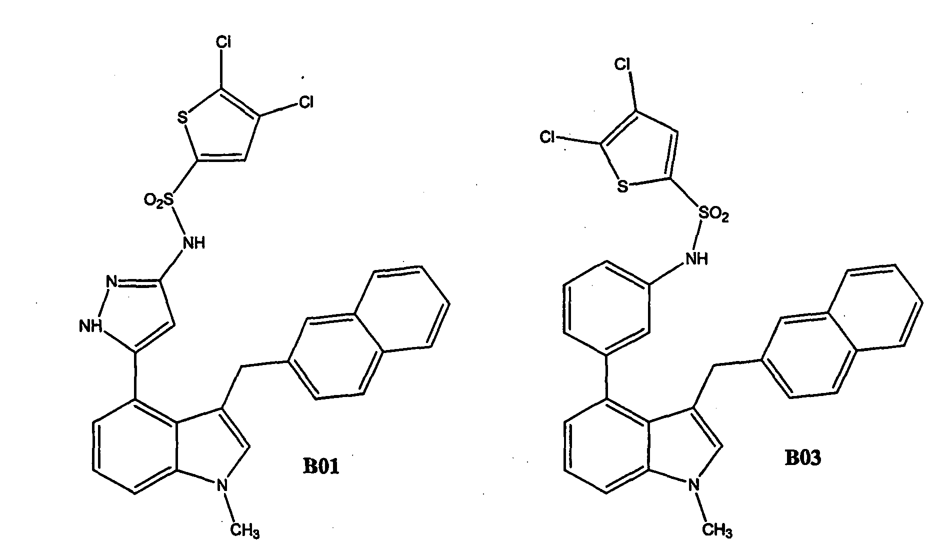

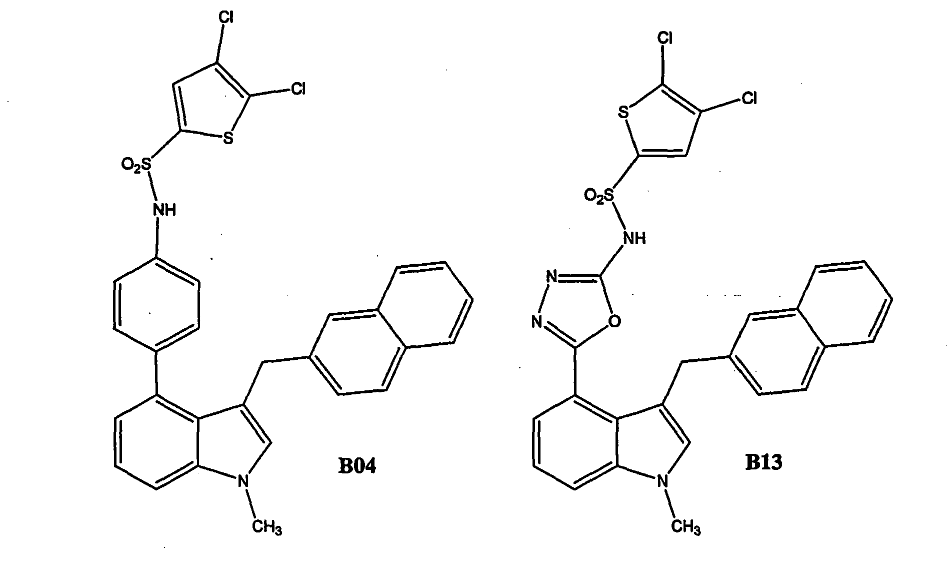

The compounds of the invention may be assayed for their binding on prostanoid EP3 receptors according to the method of Abramovitz et al. [Bioch. Biophys. Acta, 1473, 285-293 (2000)]. All of the examples in the tables below have been synthesized, characterized and tested for EP3 receptor binding.

The compounds of the invention may also be assayed for their effects on platelet aggregation *in vitro.* In experiments with human platelets, whole blood is extracted from overnight-fasted human donors. Each experiment is performed with blood from single individual. In experiments with rodent platelets, whole blood is gathered from the heart of female mice or male rats under isofluran (Abbott) anaesthesia. Blood is pooled from two or ten individual rodents for each experiment in the case of rat and mouse experiments, respectively. In all cases, blood is collected into 3.8 % sodium citrate tubes (Greiner Bio-one). Platelet-rich plasma (PRP) is obtained by centrifugation at 100 x g for 15 min at 25 °C for humans, at 150 x g for rats, or at 80 x g for 10 min for mice. Platelet-poor plasma is obtained by centrifugation of the remaining blood at 2,400 x g for 10 min at 25 °C. After counting in an Autocounter (Model 920 EO, Swelab) platelets are diluted when necessary to the desired stock concentrations (200,000-300,000 platelets/µl) using 0.9 % NaCl isotonic solution (Braun).

Platelet aggregation is determined by light absorbance using a platelet aggregometer with constant magnetic stirring (Model 490, Chronolog Cop., Havertown, Pennsylvania, USA), using a volume of 500 µl per cuvette. During the performance of the experiments, the platelet solution is continually agitated by mild horizontal shaking. Collagen (Sigma) and PGE₂ or sulprostone (Cayman Chemicals) are used as accelerants of platelet aggregation. Compounds used for this assay were dissolved and stored in a 100 % DMSO solution. After dilution, the final DMSO concentration in the assay is lower than 0.1 % v/v. It has been determined that this concentration of DMSO does not inhibit platelet aggregation in the assay. Acceleration agents and EP₃ test compounds are diluted in isotonic solution at the desired concentration. Sigmoidal non-lineal regression is used to calculate the concentration of test compound required to inhibit platelet aggregation by 50% (IC50). IC₅₀ values of test compounds are calculated using GraphPad Prism 3.02 for Windows (GraphPad Software, San Diego California USA).

Pulmonary Thromboembolism Assay: Conscious female C57BL/6 mice are dosed orally with the test compounds and 30 min later thromboembolism is induced by injection of arachidonic acid into a tail vein. Survival is evaluated one hour after the challenge with arachidonic acid, as mice that survive for that length of time usually recover fully. The arachidonic acid injection is given via a lateral tail vein in a mouse that has been warmed briefly under a heat lamp (dilation of the tail veins to facilitate the injection). Insulin syringe, 0.5 ml (from Becton Dickinson) is used for dosing. The dose volume given of both the test compound and the arachidonic acid is adjusted to the weight of the mouse (the dose volume p.o. for test copunds and i.v. for arachidonic acid solution is 10 µL and 5 µL per gram body weight, respectively). Survival rates for mice treated with test compounds (100 mg/kg, orally) in the thromboembolism model are obtained.

In general, the compounds of the present invention may be prepared by the methods illustrated in the general reaction schemes as, for example, described below, or by modifications thereof, using readily available starting materials, reagents and conventional synthesis procedures. In these reactions, it is also possible to make use of variants that are in themselves known, but are not mentioned here. The starting materials, in the case of suitably substituted fused A/B ring compounds, are either commercially available or may be obtained by the methods well known to persons of skill in the art.

Generally compounds of the Formula I, may be prepared from appropriately functionalized substituted bicyclo cores as shown in schemes 1 to 16. In particular when node "a" is a nitrogen atom, functionalization of this node followed by palladium mediated Suzuki coupling provides aryl amine derivative G3, which is subsequently derivatized to provide aryl linked amide, sulfonamide or phosphoramide G5, (Scheme 1). Alternatively, the N-functionalized intermediate is converted via palladium mediated Suzuki coupling to provide aryl ester derivative G6, which, following hydrolysis and reaction with Ph₂P(O)N₃ by in-situ generation of acyl azide, provide Curtius-rearranged product-aryl amine G8. One may also prepare the acyl acid from ester G6 using hydrazine followed by reaction of isoamyl nitrite to generate acyl azide intermediate. The amine G8 is then converted to G8, as shown in Scheme 2. The acid G7 may also be reacted with, for example, sulfonamide to provide acylsulfonamide G9. In the Schemes below, R¹ is the residue that appears in the claims as M and R² is the residue that appears in the claims as R¹.

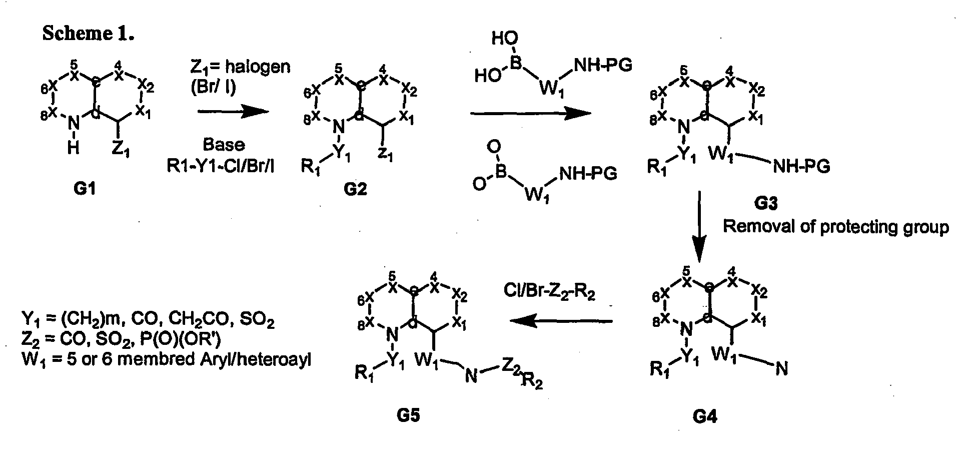

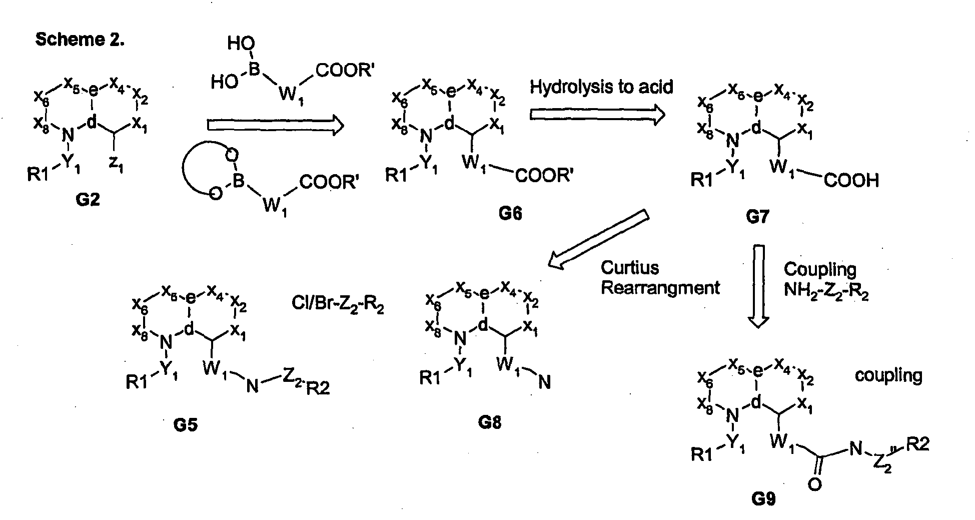

When the node "b" as carbon bears an ester or a nitrile functional group, reaction with in-situ generated anion from acetonitrile provides the corresponding β-hydroxy acrylonitrile G11, (Scheme 3) or β -amino acrylonitrile, G15 (Scheme 4), respectively. These intermediates then can be cyclized to provide nitrogen containing 5- (or 6-) membered heterocyclic amines (G12) which are the converted to amine-derivatized product G13. (Scheme 3 and 4). Alternatively, the aromatic halide bicyclic core via Heck reaction can provide the α,β-unsaturated nitrile which, upon reaction with hydrazine or amidine, provides dihydro-heterocycles which upon oxidative aromatization provide the heterocyclic amines G12, as shown in Scheme 5.

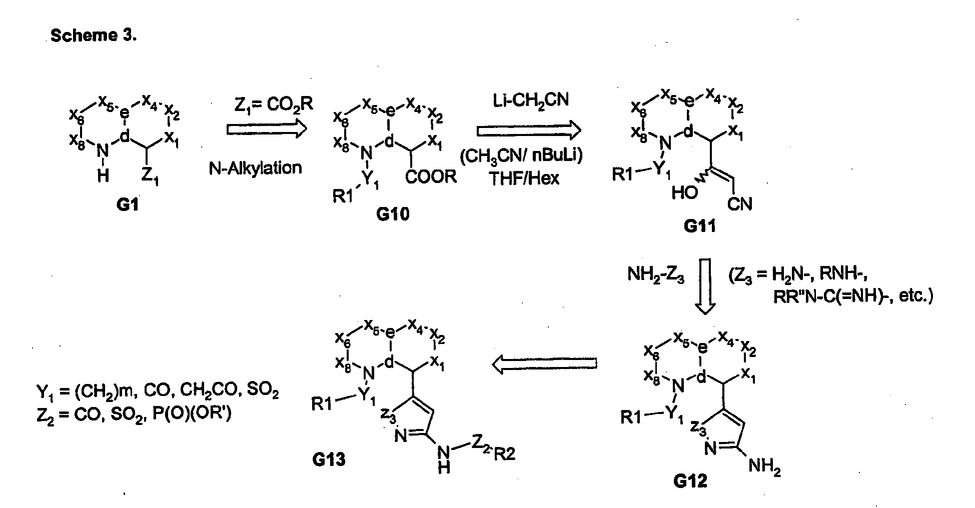

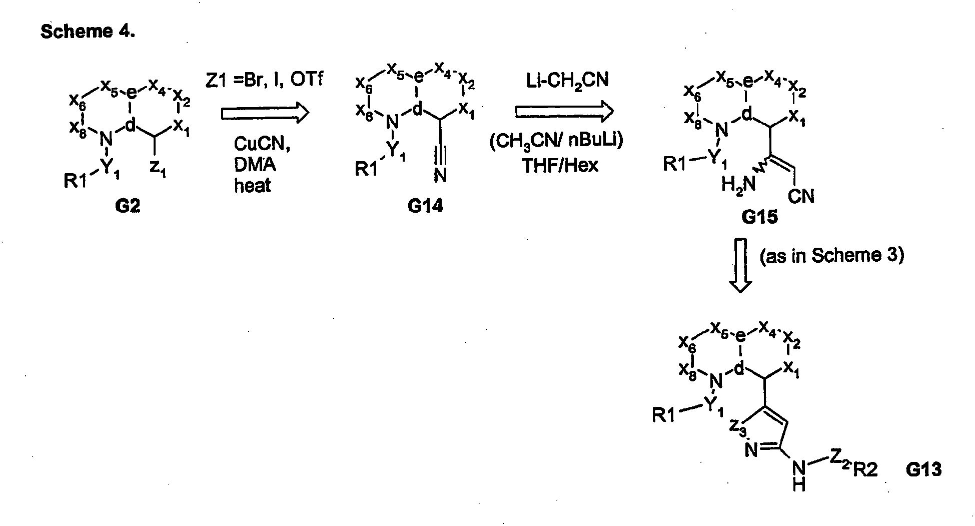

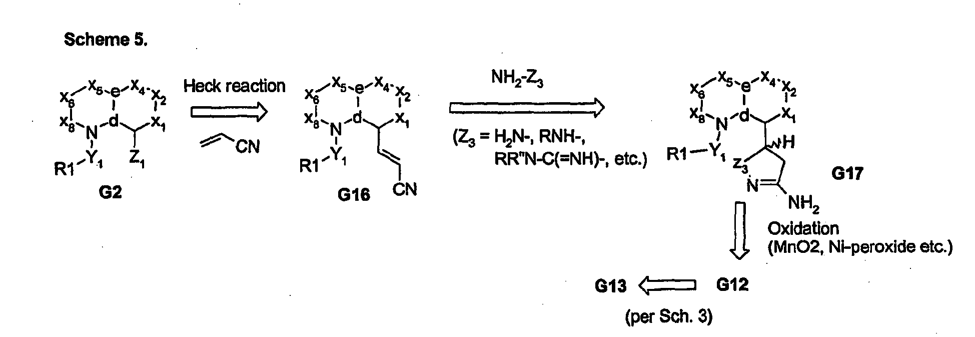

The reaction of the β-hydroxy or β-amino acrylonitrile derivatives (G11 and G15, respectively) with hydroxyl amine provide the amino-isoxazole derivative G18, leading to product G19 with regiospecificity shown (Scheme 6).

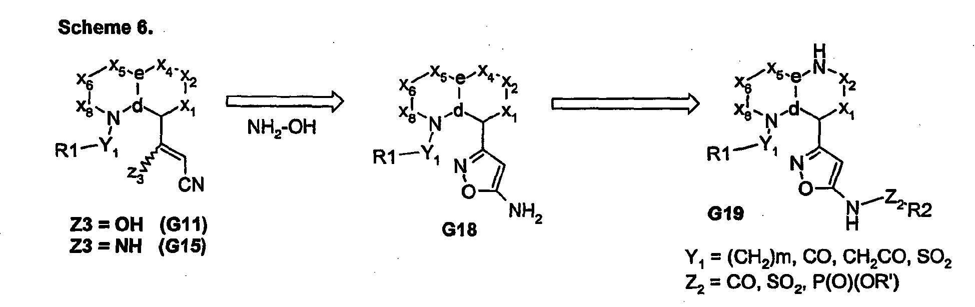

The bicyclic ester cores, following hydrolysis, provide corresponding carboxylic acids. The versatility of this intermediate, which provides entry to a wide variety of 5-membered azole derivatives, is shown in Scheme 7. The acid can be converted in a one pot reaction to amino-thiadiazole (G22, where Z₄=S). The corresponding amino-oxadiazole (G22, where Z₄-O), can be obtained form the corresponding hydrazide (G23) upon treatment with cyanogen bromide. Alternatively, the acid G20 may be reacted with semicarbazide to provide the intermediate G21 which may be converted to 5- or 6-membered heterocyclic amine, which can then be functionalized to provide products which are encompassed by the formula I.

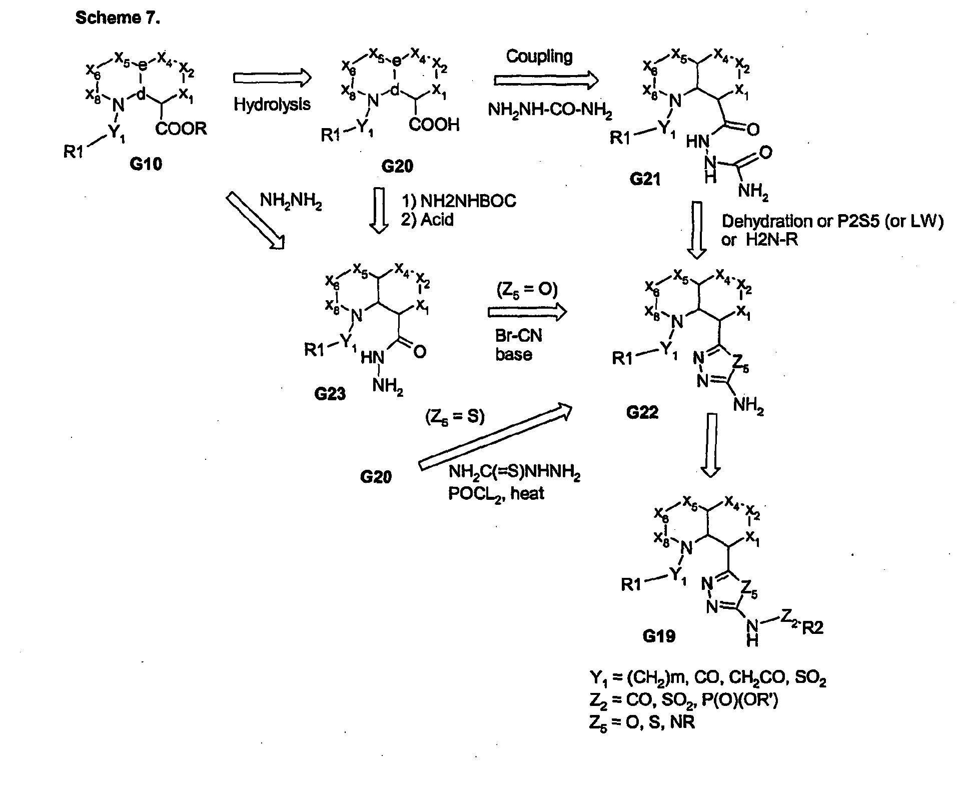

In the examples above, one of the peri-substitued linker arms has been introduced while the node "a=N". When both of the substituents on the bicyclic core are linked via carbon, the aryl linked amine and functionalized amine portion can be introduced as in the previous examples. For bicyclic systems that are electrophilic in nature, the second C-linked peri-substituents can be introduced to provide a wide diversity of substituents in which the attachment to the carbon node is thru a heteroatom. Compounds in which the attachment to carbon is through sulphur are shown in Scheme 8. Due to high nucleophilicity of the thiols, the use of cores such G24 permits the introduction of second peri-substituents. Formation of a thioether linker allows subsequent generation of sulphoxide or sulphone derived products, i.e. formation of biaryl derived analogs bearing sulphide, sulphoxide or sulphones a linkers. Scheme 9 provides a variation in which analogs to chemistry described in schemes 3 and 4 allow flexibility of input reagents and intermediates and thus diversity of products.

An example, which allows the introduction of an acyl fragment (bearing R2 group) via electrophilic reaction is shown in Scheme 16. This leads to preparation of analogs represented by G90 and G91. The benzylic carbonyl group present in G90 and G91 may be further derivatized, e.g. by reduction to alcohol or CH₂, formation of oxime, etc.

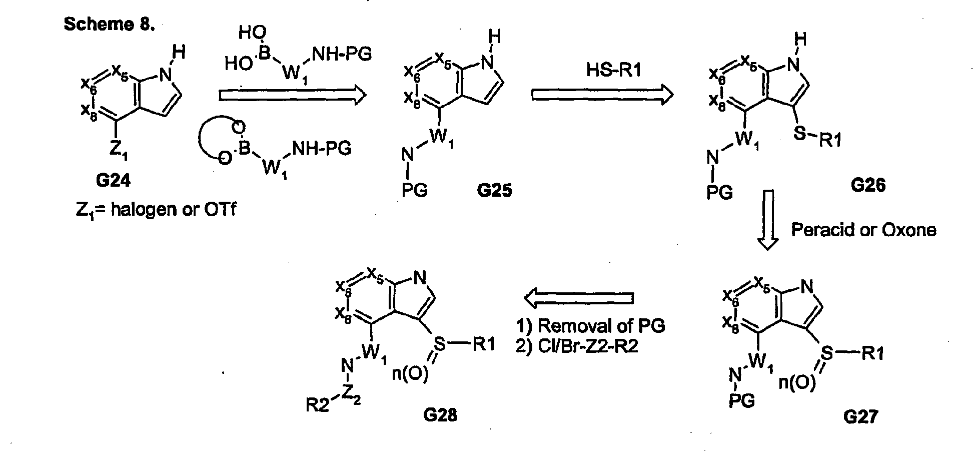

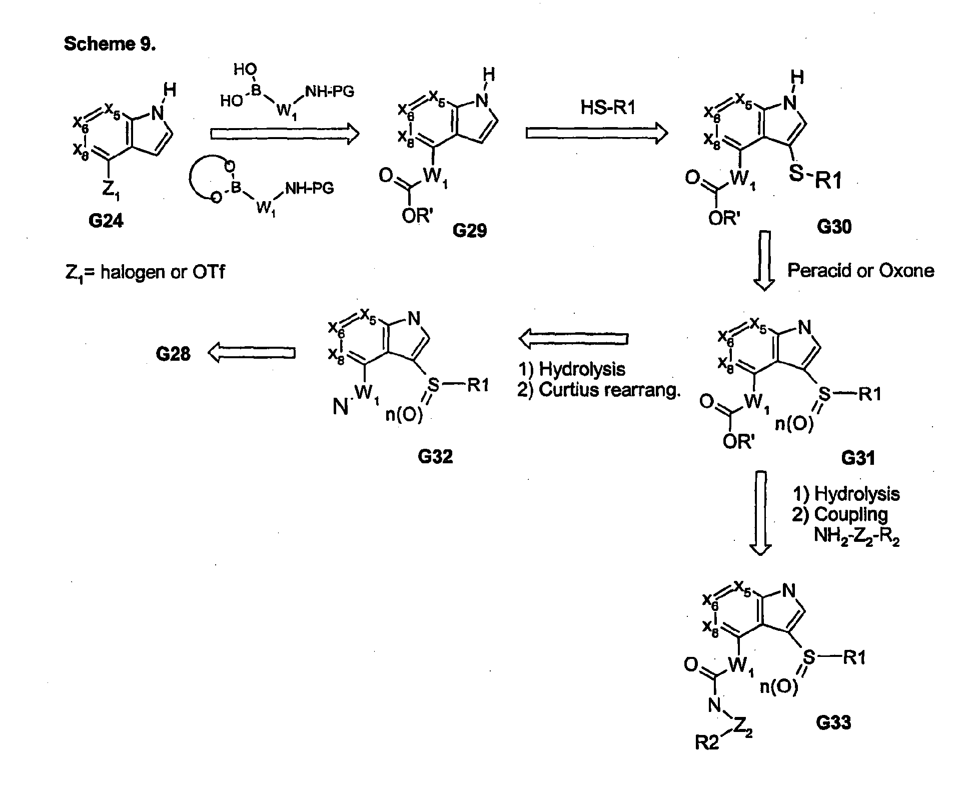

In order to prepare corresponding aza (or oxa) linked aryl/heteroaryl/alkyl groups (Rl), one may utilize reactive intermediates related to isatin, as shown by G37, which is derived from G34, (Scheme 10). As shown in scheme 10, the intermediate 37 provides access to a variety of aza-linked compounds, which are all derived by carbon linked attachment to the bicyclic core. Other variations of the isatin derived intermediates are shown in scheme 11. This approach provides peri-substituted aryl bicyclic compounds, allowing access to functionalities that are linked through carbon and nitrogen atoms of the core bicyclic system. In addition, access to the key intermediate G48, which contains a reactive carbonyl, distal to the peri-substituents ending in R1 and R2, allows the artisan to apply a range of chemistries to provide products highlighted in Scheme 11. These chemistries, e.g. ketal formation, addition to carbonyl and reaction with DAST provide access to analogs that bear diverse functionalities, as shown by G47-toG52. Analogs in scheme 10 and 11 also provide access to bicyclic cores, which contain one or both rings that are non-aromatic.

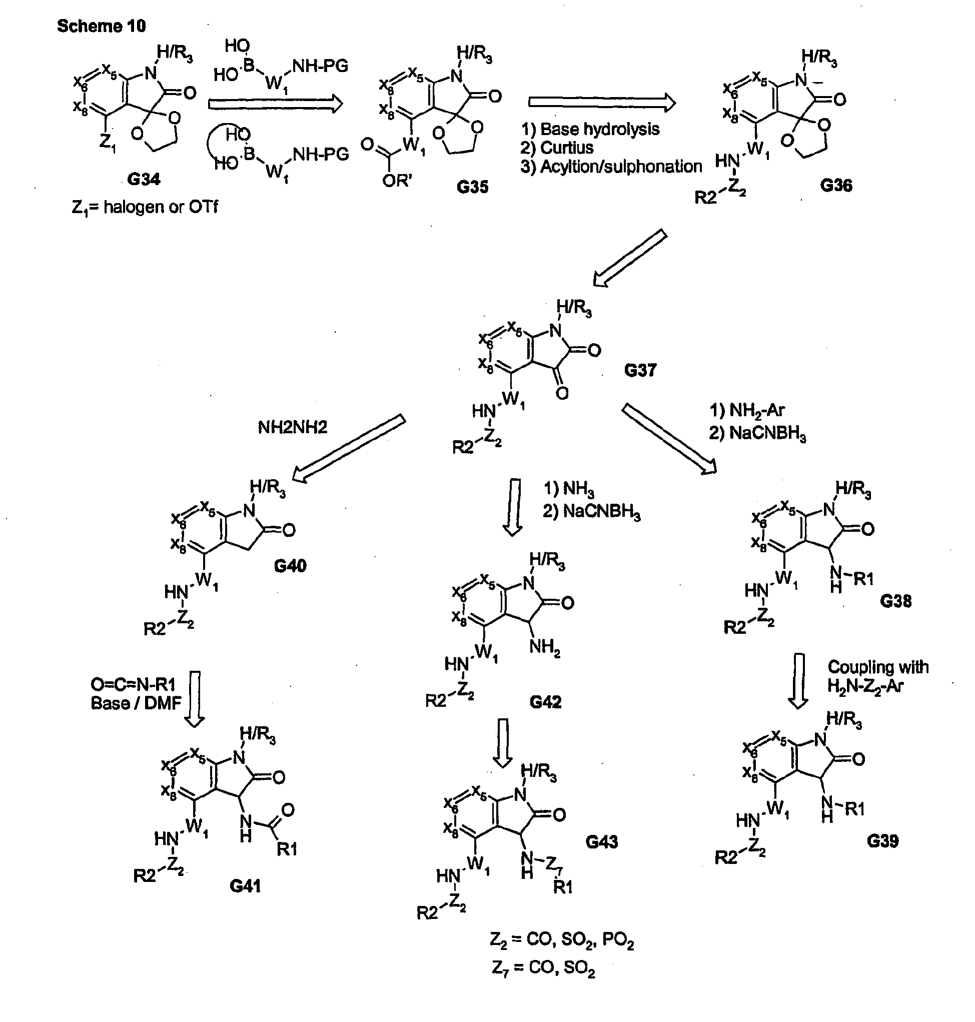

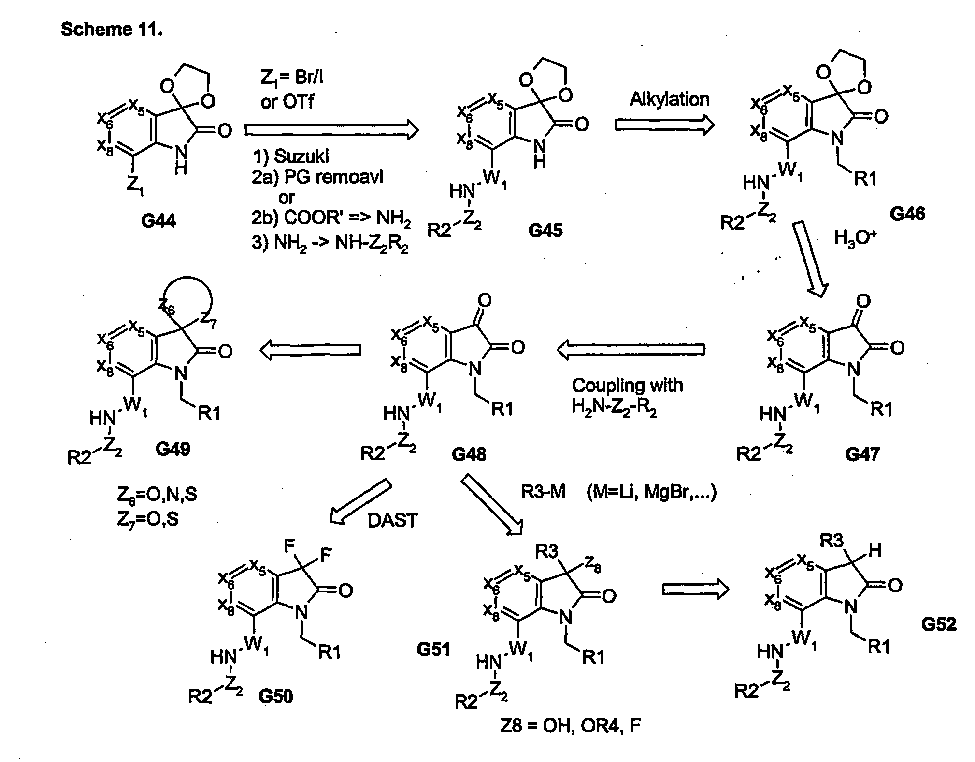

The synthetic routes outlined above, essentially all utilize a bicyclic core which is appropriately derivatized to obtain compounds described by formula I. The following chemistries provide for introduction of at least one of the perifragments as part of the construction of bicyclic core. The chemistry in Scheme 12 involves a three-component condensation reaction, whereby an α,γ-diketoester (G54), upon reaction with an aldehyde and a primary amine, provides a monocyclic product G63. The product G63, upon reaction with e.g. hydrazine (or mono substituted hydrazine), provides the peri-substituted bicyclic core (in this case a 5-5 ring system, as shown by G64), which then leads to the analog G56. The α,β-unsaturated ester can be transformed to corresponding α,β-unsaturated nitrile, which following chemistries outline in Scheme 5, provides a 5- (or six membered) heterocyclic system linked to a 5:5 bicyclic core to provide compounds represented by G58, which are encompassed by the formula I.

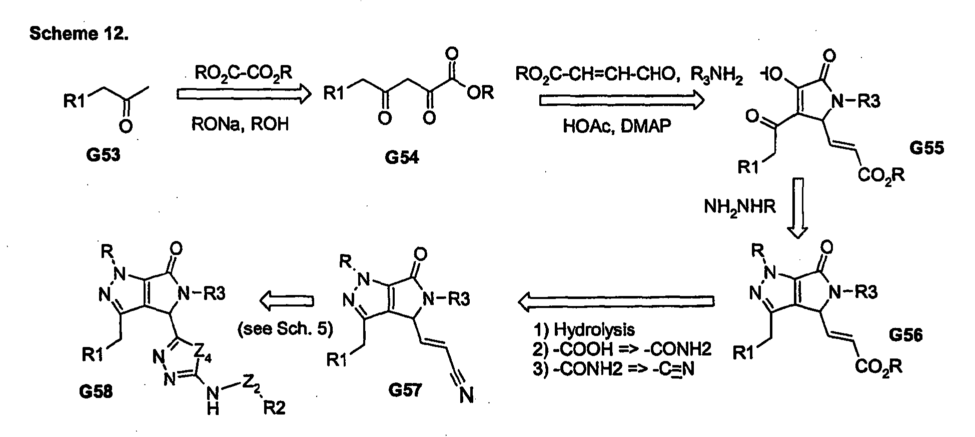

Other examples of chemistries that involve formation ofbicyclic cores are outlined in Schemes 13 and 14. These examples present syntheses of benzimidazole-based cores. In order to prepare a peri-substituted system, the R1 group is introduced regiospecifically at step G61-G62. In Scheme 14, the desired regiospecific introduction of the R1 group is accomplished by O => N acyl migration followed by reduction of amide to secondary amine. In this case ring closure also provides the desired peri-substituents, as in G70. The intermediates G62 and G70 subsequently can be derivatized following the sequence of steps described in Scheme 11 to provide the desired products G64 and G71 respectively.

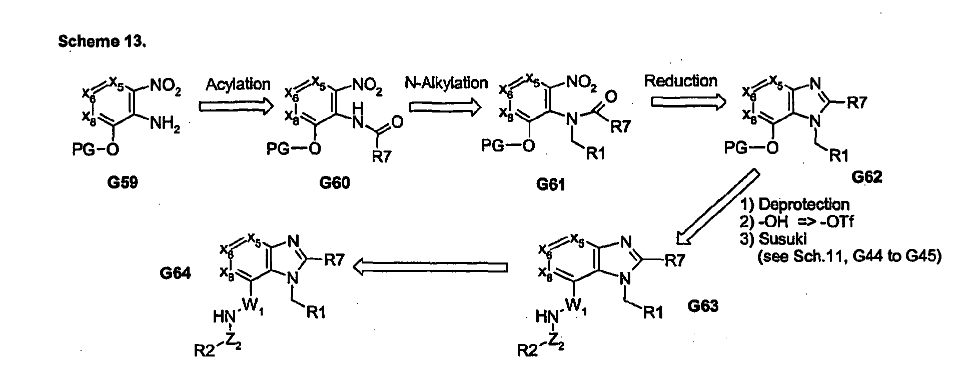

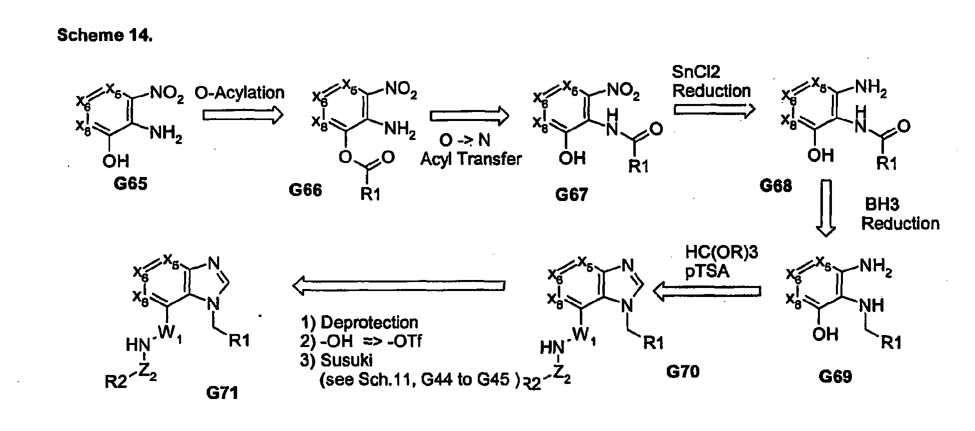

Another example of the chemistries involved in formation ofbicyclic cores with desired peri-functionalization is depicted in Scheme 15. Here, a thermal cyclization of an amine with a cyclic γ-keto acid G74 provides the required bicyclic intermediate G75. Bromination then provides the key intermediate which allows several routes for conversion to the motif with desired variations. One may utilize a Suzuki reaction pathway to provide G77, Alternatively, the vinyl bromide may be converted to the corresponding trisubstitued unsaturated ester or nitrile, which can then be derivatized following chemistries outlined in Scheme ¾ [Jasbir: what scheme should this be?] 6 and 7 to provide G80, G79 and G81, respectively. These chemistries allow synthesis of essentially non-aromatic ring systems and also provide for formation of bicyclic ring systems wherein the ring (a) is 5-membered. Ring (a) is produced during the cyclization reaction, whereas the size of the ring (b) is controlled by the use of the cyclic ketone at the initial step of the synthesis and thus allow for formation of "5-N" bicyclic system. In addition to the size; the substituent and presence of heteroatoms in the cyclic ketone also allow flexibility. The nature of the tertiary group may also be varied, and this may be introduced at the cyclic ketone stage, which allows significant control over its regiochemistry. The positions X5 /X8 may be heteroatoms and / or contain additional substituents as well.

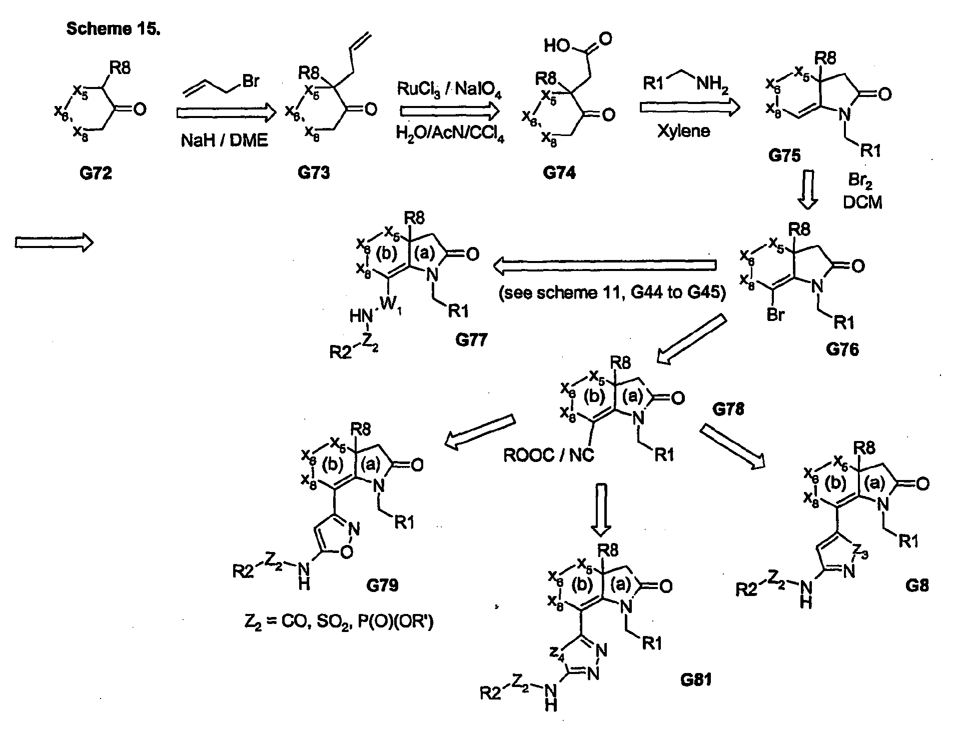

Scheme 16 provides for an alternate substitution pattern for carbon-linked bicyclic peri-substituents compared to that described earlier in Schemes 8 and 9. The reaction of an indole type of bicyclic core is with a cyclic ketone bearing an appropriately substituted ester or protected amine allows introduction of substituents at the C3 position. One may carry out functionalization or derivatization of the ester or amine to provide a non-aryl peri-substituents (G87 or the one derived form G86 as an acyl sulfonamide), or one may first conduct aromatization followed by derivatization of the amine/acid substituent to generate peri-substituted, bicyclic aryl sulfonamides, amides, phosphoramides etc., which are encompassed by the formula I.

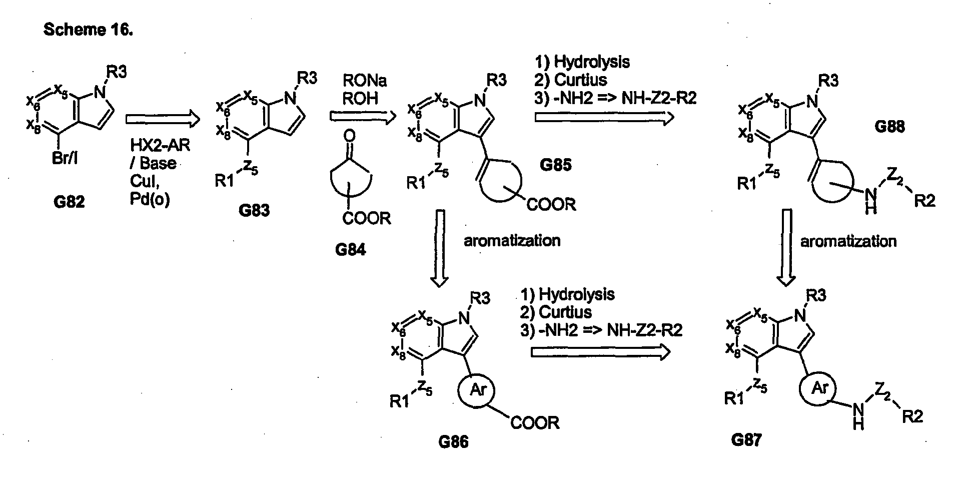

G10, where R = alkyl (Me for example), following with the dianion of 2-bromoacetic acid followed by decarboxylation provide α-bromoketone G89 (X = Br). Reaction of G89 with thiourea then delivers 2-aminothiazoles G90. Amino thiazole G90 can then be derivatized to yield G91 by methods similar to those described earlier. These series of reactions (dianion of bromoacetic acid and reaction with thiourea) can also be applied to other bicylco core derived eaters like G78 (Scheme 15) to provide corresponding 2-aminothiazoles, which are further elaborated.

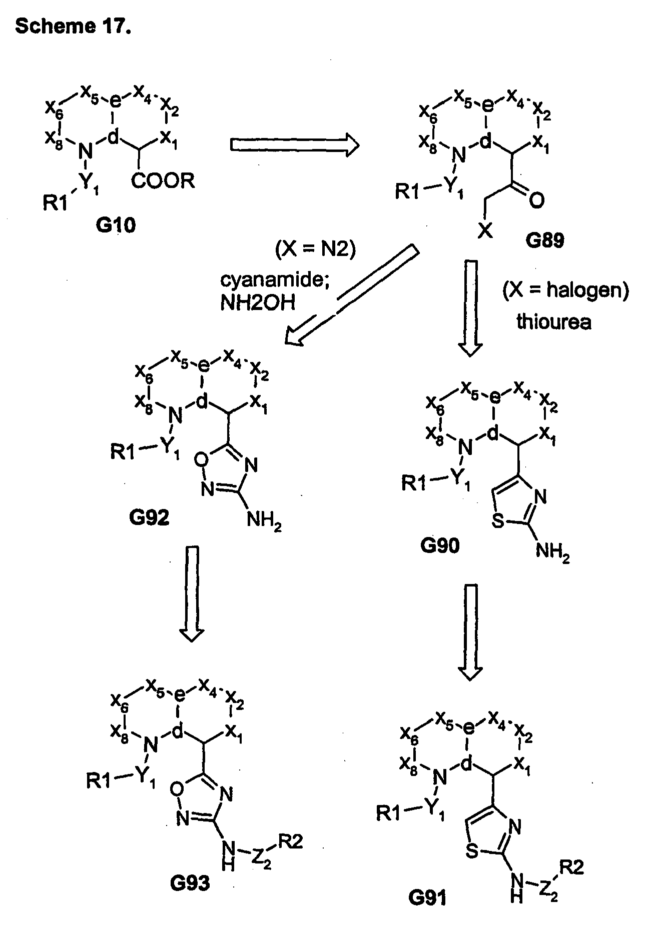

G10, where R = H (carboxylic acid) can be converted to the corresponding acid chloride which following reaction with diazomethane provide the diazoketone G89 (X = N2). The intermediates G89 can then be reacted with cyanamide followed by hydroxylamine to afford 3-amino-1,2,4-oxadiazoles G92. The amino group of G92 is then derivatized (with e.g. sulphonyl chloride) to provide G93 (sulphonamide).

The α-bromoketone functionality can also be incorporated onto the C-3 position of indoles derived core, such as G83, using bromoacetyl chloride. Reaction of the resulting α-bromoketone with thiourea provides a 4-(2-aminothiazole) [analogous to G91] appended to the C-3 position of the indole ring. The amino functionality of the resulting compounds can be further elaborated as described earlier. The methods described in the scheme 17 represent additional examples to build a diverse range of amino-heterocycles as key derivative to provide compounds related to the genus of the invention.

Finally, several appropriately functionalized bicylic cores are either commercially available or their syntheses are described in the published literature or could be inferred by one skill in the art. Examples of several of these are described as part of the Specific Examples. Some of these are summarized below.

For bicyclic systems wherein one of the nodes is nitrogen, indole derivatives serve as a readily accessible and useful core. The 4-bromo and 4-hydroxy indoles are commercially available. The 7 substituted indoles, e.g. 7-CO₂R, 7-alkoxy, 7-benzyloxy, etc. can be prepared by Batcho-Leimgruber chemistry from appropriately substituted 2-nitrotoluene, (Org Synthesis Co, Vol. 7). This approach also provides access to 7-Me, 7-CHO, 7-CN, and 7-OH indoles by functional group manipulations. Alternatively, the 7-halo indoles are accessible from 2-halo anilines via Bartoli chemistry (Bartoli, G. et.al. Tett. Letters, 1989, 30, 2129-2132). Diverse 7-substituted indoles may also be prepared via selective functionaliztion of indole via directed *ortho* metalation according to the procedure of Snieckus, [Snieckus V. et.al. Org Letters 2003, 1899-1902]. These various approaches also provide access to other substituted indole derivatives. The 8-hydroxytetrahydroquinolines, a [6:6]-based core, can be obtained from commercially available 8-hydroxy quinoline by reduction. 8-OH-1H-Quinolin-2-one, 8-OH-3,4 dihydro-1H-Quinolin-2-one. 2,6-dihydroxy anilines or related heterocycles may be transformed to 5-hydroxy-4H-benzo[1,4]oxazin-3-one, 5-hydroxy-4H-benzo[1,4]oxazin-2,3-dione, 4-hydroxy-3H-benzooxazol-2-one, bicyclic derivatives. Oxidation of indole based 1,7-disubstituted or 3,4-disubstituted bicyclo analogs provides corresponding oxyindole derivatives. Various anilines may be converted to isatin analogs using the literature procedures, and examples of these are described in the specific example section below. Synthesis of a series of [5:5] bicyclo cores (e.g. imidazothiazole and pyrrolopyarzolone) are described in the specific examples. A diverse group of [6:5] bicyclo cores can also be obtained analogous to literature syntheses of cores such as imidazopyridine and imidazopyrimidine [Katritzky A.R. et.al. JOC 2003, 68, 4935-37], pyrrolopyrimides [Norman M. et.al. JMC 2000, 43, 4288-4312]. These diverse bicyclo cores may then be derivatized to provide analogs of formula I.

Overall, the range of chemistries shown above allows for preparation of potent prostenoid antagonists / agonists. The chemistry allows manipulation of the core structure and introduction of optimal functional groups to provide a desired balance of hydrophobicity-hydrophilicity; it allows introduction of hydrogen bond donor and acceptors with desired topology; it allows adjustment of desired physical characteristics suitable for achieving desired pharmaceutical and ADME properties (e.g. membrane permeability, low plasma protein binding, desired metabolic profile etc.). The ability to adjust physical characteristics permits suitable formulation for oral bioavailability, which in turn allows for control over the size and frequency of dose administered to mammals to achieve desired pharmacological response. The ability to adjust metabolic profile allows for minimizing potential for drug-drug interactions. Thus the scope of this invention not only provides for preparation of potent prostenoid antagonists with proper isozyme selectivity to be useful tools for research, it also provides compounds are of value in therapy.

The following specific non-limiting examples, shown in Table 1 are illustrative of the invention. For entires in Table 1, 'X1'=CH except for B47 where it is C(=O); 'X2' is absent except for except for B43, B44, B45 where it is CH; 'g'=C; 'h' = C except for B02 where it is N; 'b' and 'd' are = C.

**Table 1**

| | | | | | | | | | | | | | | | |
|---|---|---|---|---|---|---|---|---|---|---|---|---|---|---|---|
| 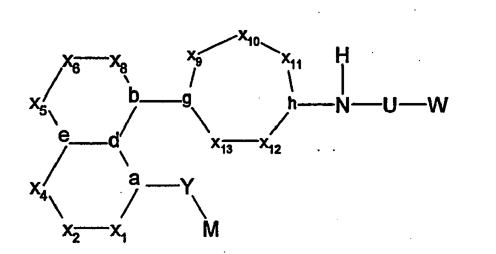 | | | | | | | | | | | | | | | |

| Cmpd No. B(x) | X4 | X5 | X6 | X8 | X9 | X10 | X11 | X12 | X13 | a | e | U | W | Y | M |
|---|---|---|---|---|---|---|---|---|---|---|---|---|---|---|---|
| B01 | N(CH3) | CH | CH | CH | - | NH | N | - | CH | C | C | SO2 | 4,5-diCl thio | CH2 | 2-Naphth |
| B02 | N(CH3) | CH | CH | CH | CH | C(NH2) | N | - | - | C | C | SO2 | 4,5-diCl thio | CH2 | 2-Naphth |
| B03 | N(CH3) | CH | CH | CH | CH | CH | CH | - | CH | C | C | SO2 | 4,5-diCl thio | CH2 | 2-Naphth |
| B04 | N(CH3) | CH | CH | CH | | CH | CH | CH | CH | C | C | SO2 | 4,5-diCl thio | CH2 | 2-Naphth |
| B05 | N(CH3) | CH | CH | CH | - | CH | CH | CH | CH | C | C | SO2 | CH3 | CH2 | 2-Naphth |
| B06 | C(CH3) | CH | CF | CH | - | CH | CH | CH | CH | N | C | SO2 | CH3 | CH2 | 3,4-diF2Ph |
| B07 | C(CH3) | CH | CF | CH | - | CH | CH | CH | CH | N | C | SO2 | CF3 | CH2 | 3,4-diF2Ph |
| B08 | N(CH3) | CH | CH | CH | - | CH | CH | CH | CH | C | C | SO2 | CF3 | CH2 | 2-Naphth |
| B09 | C(CH3) | CH | CF | CH | - | CH | CH | CH | CH | N | C | SO2 | CH3 | CH2 | 2,4-diCl2Ph |
| B10 | C(CH3) | CH | CF | CH | - | N | N | - | O | N | C | C(=O) | CF3 | CH2 | 2,4-diCl2Ph |
| B11 | C(CH3) | CH | CF | CH | - | N | N | - | O | N | C | SO2 | CH3 | CH2 | 2,4-diCl2Ph |
| B12 | C(CH3) | CH | CF | CH | - | N | N | - | O | N | C | SO2 | 2,4,5-TriF3Ph | CH2 | 2,4-diCl2Ph |
| B13 | C(CH3) | CH | CF | CH | - | N | N | - | O | N | C | SO2 | 4,5-diCl thio | CH2 | 2,4-diCl2Ph |
| B14 | C(CH3) | CH | CF | CH | - | N(CH3) | N | - | CH | N | C | SO2 | CH3 | CH2 | 3,4-diF2Ph |
| B15 | C(CH3) | CH | CF | CH | - | N(CH3) | N | - | CH | N | C | SO2 | 4,5-diCl thio | CH2 | 3,4-diF2Ph |
| B16 | C(CH3) | CH | CF | CH | - | O | N | - | CH | N | C | SO2 | CH3 | CH2 | 3,4-diF2Ph |
| B17 | N(CH3) | CH | CH | CH | - | CH | CH2 | CH | CH | C | C | C(=O) | CH3 | CH2 | 2-Naphth |
| B18 | N(CH3) | CH | CH | CH | - | CH | CH2 | CH | CH | C | C | C(=O) | CF3 | CH2 | 2-Naphth |
| B19 | C(CH3) | CH | CF | CH | - | N | N | - | O | N | C | SO2 | 3,4-diF2Ph | CH2 | 2,4-diCl2Ph |
| B20 | C(CH3) | CH | CF | CH | - | N | N | - | O | N | C | SO2 | 3,4-diCl2Ph | CH2 | 2,4-diCl2Ph |
| B21 | C(CH3) | CH | CF | CH | - | N | N | - | O | N | C | P(=O) | Ph2 | CH2 | 2,4-diCl2Ph |
| B22 | C(CH3) | CH | CF | CH | - | N | N | - | O | N | C | P(=O) | (O-2,4-diCl2Ph)2 | CH2 | 2,4-diCl2Ph |
| B23 | C(CH3) | CH | CF | CH | - | N | N | - | O | N | C | C(=O) | 4-FPh | CH2 | 2,4-diCl2Ph |
| B24 | C(CH3) | CH | CF | CH | - | N | N | - | O | N | C | C(=O) | 5-isoxazole | CH2 | 2,4-diCl2Ph |
| B25 | C(CH3) | CH | CF | CH | - | N | N | - | O | N | C | C(=O) | 3,5-diCl2Ph | CH2 | 2,4-diCl2Ph |
| B26 | C(CH3) | CH | CF | CH | - | N | N | - | O | N | C | C(=O) | 3,4-diF2Ph | CH2 | 2,4-Cl2Ph |
| B27 | C(CH3) | CH | CF | CH | - | N | N | - | O | N | C | C(=O) | 2,4-diF2Ph | CH2 | 2,4-diCl2Ph |
| B28 | C(CH3) | CH | OF | CH | - | N | N | - | O | N | C | C(=O) | 2,4-diCl2Ph | CH2 | 2,4-diCl2Ph |
| B29 | C(CH3) | CH | CF | CH | - | N | N | - | O | N | C | C(=O) | 3,4-OCF2O-Ph | CH2 | 2,4-Cl2Ph |
| B30 | C(CH3) | CH | CF | CH | - | N | N | - | O | N | C | C(=O) | 2-Fucanyl | CH2 | 2,4-diCl2Ph |
| B31 | C(CH3) | CH | CF | CH | - | O | N | - | CH | N | C | SO2 | 4,5-diClthio | CH2 | 3,4-diF2Ph |
| B32 | C(CH3) | CH | CF | CH | - | O | N | - | CH | N | C | SO2 | 3,4-diF2Ph | CH2 | 3,4-diF2Ph |
| B33 | C(CH3) | CH | CF | CH | - | O | N | - | CH | N | C | SO2 | 2,4,5-tnF3Ph | CH2 | 3,4-diF2Ph |
| B34 | C(CH3) | CH | CF | CH | - | O | N | - | CH | N | C | SO2 | 4,5-diClthio | CH2 | 2,4-diCl2Ph |
| B35 | C(CH3) | CH | CF | CH | - | O | N | - | CH | N | C | SO2 | 3,4-diF2Ph | CH2 | 2,4-diCl2Ph |
| B36 | C(CH3) | CH | CF | CH | - | O | N | - | CH | N | C | SO2 | 2,4,5-triF3Ph | CH2 | 2,4-diCl2Ph |
| B37 | C(CH3) | CH | CF | CH | - | O | N | - | CH | N | C | SO2 | 3,4-diCl2Ph | CH2 | 2,4-diCl2Ph |
| B38 | C(CH3) | CH | CF | CH | - | O | N | - | CH | N | C | SO2 | 3,4-diF2Ph | CH2 | 2-Naphth |
| B39 | C(CH3) | CH | CF | CH | - | O | N | - | CH | N | C | SO2 | 2,4,5-triF3Ph | CH2 | 2 Naphth |
| B40 | C(CH3) | CH | CF | CH | - | O | N | - | CH | N | C | SO2 | 3,4-diCl2Ph | CH2 | 2-Naphth |
| B41 | C(CH3) | CH | CF | CH | - | O | N | - | CH | N | C | SO2 | 4,5-diCl thio | CH2 | 2-Naphth |
| B42 | C(CH3) | CH | CF | CH | - | O | N | - | N | N | C | SO2 | 4,5-diCl thio | CH2 | 2,4-diCl2Ph |
| B43 | CH | N(CH3) | - | CH | - | CH | S | - | N | C | C | SO2 | 4,5-diCl thio | O | 2-Naphth |
| B44 | CH | N(CH3) | - | CH | - | CH | S | - | N | C | C | SO2 | 3,4-diF2Ph | O | 2-Naphth |
| B45 | CH | N(CH3) | - | CH | - | O | N | - | CH | C | C | SO2 | 3,4-diF2Ph | O | 2-Naphth |
| B46 | C(CH3) | CH | CF | CH | - | CH | N | CH | CH | N | C | SO2 | 4,5-diCl thio | CH2 | 2,4-diCl2Ph |
| B47 | CH2 | CH2 | CH2 | CH2 | - | CH | S | - | N | N | C(CH3) | SO2 | 3,4-diF2Ph | CH2 | 3-[OCIB]Ph |

### Example 1. Preparation of B01.

Synthesis of (4-Bromo-1H-indol-3-yl)-naphthalen-2-yl=methanone, 1-1: To a solution of 4-bromoindole (5g, 25.5 mmol) in anhydrous methylene chloride (100 mL) was added MeMgBr (3M solution in ether, 8.95 mL, 26.7 mmol) drop wise at 20°C. A slight exotherm was observed (maximum temperature observed was 28°C). The resulting orange solution was stirred for 10 min at rt, then the ZnCl₂ (1M solution in ether, 76.5 mL, 76.5 mmol) was added via addition funnel. The reaction mixture was stirred for 30 minutes. A solution of naphthoyl chloride (5.1g, 26.7 mmol) in methylene chloride (25 mL) was added during which a color change from light orange to dark red occurred. The resulting mixture was stirred at rt overnight. TLC (EtOAc/hexanes, 1:2) showed the reaction was complete and then the mixture was quenched with saturated NH₄Cl (100 mL). The resulting suspension was stirred for 15 min. The resulting solids were filtered off and washed several times with methylene chloride. The filtrate was washed with saturated NH₄Cl, water, brine, dried (MgSO₄) filtered and concentrated *in vacuo* to afford crude product (7 g). The solid was taken up into 10% aqueous HCl solution and extracted with ethyl acetate. The organic layer was washed with water, brine, dried over MgSO₄, filtered and concentrated to give 500 mg crude product. The combined crude product (7.5 g) was washed with MTBE (15 mL), the solvent was decanted, and then the solids were suspended in MTBE/hexane, 1:1 (10 mL) and filtered to afford 4.61g of pure title compound. The filtrate was concentrated and residue was purified by column chromatography (SiO₂), eluting with an ethyl acetate/hexane gradient (1:3 to 1:1) to give 2 g pure title compound, I-1, a total of 6.61g (74% yield). ¹H NMR (400 MHz, CDCl3) confirmed the structure.

Synthesis of (4-Bromo-1-methyl-1H-indol-3-yl)-naphthalen-2-yl-methanone, 1-2: Iodomethane (4.55 g, 32 mmol, 2 equiv.) was added to a stirred solution of I-1 (5.55 g, 15.9 mmol, 1 equiv.) and K₂CO₃ (5.48 g, 39.6 mmol, 2.5 equiv.) in acetone (110 mL). The reaction mixture was stirred overnight at rt. The reaction mixture was concentrated, diluted with water (100 mL) and extracted with ethyl acetate (3 x 100 mL). The combined organic layers were washed with water (50 mL), brine (50 mL), dried over MgSO₄, filtered, and concentrated to afford 5.45 g (94 %) of title compound 1-2 as a brown oil. ¹H-NMR (500 MHz, CDCl₃) confirmed the structure.

Synthesis of 4-Bromo-1-methyl-3-naphthalen-2-ylmethyl-1H-indole, 1-3: A 1 M solution of BH₃THF (16.3 mL, 16.3 mmol, 3.3 equiv.) in THF was added over 15 min to a stirred solution of 1-2 (1.8 g, 4.9 mmol, 1 equiv.) in THF (48 mL) at 0 °C and allowed to slowly warm to rt. The reaction mixture was then stirred at rt overnight. MeOH (3 mL) was added dropwise over 5 min, followed by additional MeOH (50 mL). The solvent was evaporated *in vacuo,* followed by the subsequent addition of MeOH (50 mL) and *in vacuo* evaporation. This was repeated twice to afford 2 g of yellow oil. The oil was dissolved in CH₂Cl₂ /hexane, I:4 (8 mL) at 40 °C, and allowed to cool to rt, and purified by chromatography on SiO₂ (27 g), eluting with a CH₂Cl₂/hexanes gradient (1:4 to 1:1) to afford 1-3 (1.04 g, 60%). ¹H-NMR (500 MHz, CDCl₃) confirmed the structure.

Synthesis of 1-Methyl-3-naphthalen-2-ylmethyl-1H-indole-4-carbonitrile, I-4: A solution of I-3 (200 mg, 0.571 mmol, 1 equiv.) and copper(I) cyanide (153 mg, 1.713 mmol, 3 equiv) in anhydrous dimethyl acetamide (0.83 mL) was degassed with argon for 15 min at rt and then heated at 210 °C in a closed vial for 2 h. Water and ethyl acetate (4 mL each) was added twice, and the resultant suspension filtered through celite. The residue was twice washed with ethyl acetate (2 mL) and filtered. The organic layer was separated, washed with water (4 x 4 mL), brine (4 mL), dried over MgSO₄, filtered, and *concentrated in vacuo* to afford 1-4 (167 mg, 99%) as brown oil, which crystallized upon standing. *R_{f}* 0.42 (EtOAc/hexanes, 1:3). MS (ESI⁻): 296 (M-1), ¹H-NMR (500 MHz, CDCl₃) confirmed the structure.

Synthesis of 3-Amino-3-(1-methyl-3-naphthalen-2-ylmethyl-1H-indol-4-yl)-acrylonitrile, I-5: Solution of n-BuLi (1.6 M, 1.7 mL, 2.7 mmol, 10 equiv.) in hexanes was added dropwise to a solution of I-4 (80 mg, 0.27 mmol, 1 equiv.) in anhydrous acetonitrile (111 mg, 2.7 mmol, 10 equiv.) and THF (2 mL) at -78 °C. The reaction mixture was allowed to warm to rt and stir for 1.5 h. The reaction was then quenched with saturated NH₄Cl, and extracted with ethyl acetate. The organic layer was washed with brine and evaporated to give crude I-5 (186 mg) as dark brown oil. *R_{f}=* 0.52 (EtOAc/hexanes, 1:1). MS (AP⁺): 338 (M+1). ¹H-NMR (500 MHz, CDCl₃) confirmed the structure.

Synthesis of 3-Hydroxy-3-(1-methyl-3-naphthalen-2-ylmethyl-1H-indol-4-yl)-acrylonitrile, I-6. A solution of crude 1-5 (186 mg) in CHCl₃ (2 mL) was stirred with 10% aqueous HCl (2 mL) at rt overnight. The organic layer was separated, filtered through celite, and washed with CHCl₃ (2 mL). Concentration of the filtrate afforded crude I-6 (106 mg, quantitative) as dark brown *oil. R_{f}=* 0.73 (EtOAc/hexanes, 1:1). MS (AP⁺): 338 (M+1). ¹H-NMR (500 MHz, CDCl₃) confirmed the structure.

Synthesis of 5-(1-Methyl-3-naphthalen-2-yhnethyl-1H-indol-4-yl)-1H-pyrazol-3-ylamine, I-7. To a solution of I-6 (46 mg, 0.136 mmol, 1 equiv.) and hydrazine hydrate (68 mg, 1.36 mmol, 10 equiv.) in ethanol (0.3 mL) was heated at 100 °C overnight, then 120 °C for 2 h. The reaction mixture was quenched with saturated NH₄Cl, and extracted with ethyl acetate. The organic layer was washed with water, brine and evaporated *in vacuo* to afford 46 mg of a crude product. The residue was chromatographed on SiO₂ (1 g), eluting with an ethyl acetate/hexanes gradient (1:4,1:3, 1:1), followed by pure ethyl acetate to afford I-7 (10 mg, 46 %) as yellow oil. *R_{f}*= 0.19 (EtOAc). MS (AP⁺): 353 (M+1). ¹H-NMR (500 MHz, CDCl₃) confirmed the structure.

Synthesis 4,5-Dichloro-thiophene-2-sulfonic acid [5-(1-methyl-3-naphthalen-2-ylinethyl-1H-indol-4-yl)-1H-pyrazol-3-yl]-amide, B01 and 1-(4,5-Dichloro-thiophene-2-sulfonyl)-5-(1-methyl-3-naphthalen-2-ylmethyl-1H-indol-4-yl)-1H-pyrazol-3-ylamine, B02: A solution of I-7 (12 mg, 0.034 mmol, 1 equiv.), 2,3-dichlorothiophene-5-sulfonylchloride (8.6 mg, 0.034 mmol, 1 equiv.) and DMAP (0.2 mg, 0.0017 mmol, 0.05 equiv.) in pyridine (0.2 mL) was stirred at rt for 1 h. The reaction was then quenched with 10% aqueous HCl and extracted with ethyl acetate. The combined organic layers were washed with water, brine, and dried over MgSO₄. The solution was concentrated *in vacuo* to afford a crude mixture of sulfonamides (23 mg) as a red solid. This crude product was combined with crude product from a previous reaction (9 mg, obtained from reaction of 7 mg, 0.02 mmol of I-7). The combined crude mixture was chromatographed on SiO₂ (2 g), eluting with an ethyl acetate/hexanes gradient (1:4 to 1:1) to afford less polar B02 (6.7 mg, 22%) as an orange solid; *R_{f}*= 0.26 (EtOAc/hexanes, 1:3); LC-MS (80 %): ESI⁺ Calcd. 567 (M) Found: 568.9 (M+1). ¹H NMR (CDCl₃) 3.72 (s, 3H), 4.05 (s, 2H), 4.70 (br s, 2H), 5.29 (s, 1H), 6.65 (br s, 1H), 7.04 (dd, J = 8.8, 0.8 Hz, 1H), 7.15 (dd, J = 8.8, 2.0 Hz, 1H), 7.21 (dd, J = 8.0, 7.2 Hz, 1H), 7.34 (dd, J = 8.4, 1.2 Hz), 7.35-7.42 (m, 3H), 7.61 (s, 1H), 7.66 (d, J = 8.4 Hz, 1H), 7.70-7.73 (m, 1H), 7.75-7.78 (m, 1H),and B01 (8 mg, 26%) as a red solid; *R_{f}*= 0.41 (EtOAc/hexanes, 1:1); LC-MS (92 %): ESI⁺ Calcd. 566 (M) Found: 567.3 (M+1). ¹H NMR (CDCl₃) 3.72 (s, 3H), 3.86 (s, 2H), 6.47 (s, 1H), 6.65 (br s, 1H), 7.05 (dd, J = 7.2,0.8 Hz, 1H), 7.10 (dd, J = 8.8, 2.0 Hz, 1H), 7.22 (s, 1H), 7.25 (d, J = 8.0 Hz, 1H), 7.27 (d, J = 8.8 Hz, 1H), 7.34 (br s, 1H), 7.36-7.41 (m, 3H), 7.62-7.66 (m, 2H), 7.73 (dd, J = 6.8, 2.8 Hz, 1H).

### Example 2. Preparation of B03.

Synthesis of 3-(l-Methyl-3-naphthalen-2-yhnethyl-1H-indol-4-yl)-phenylamine, I-8. A mixture of I-3 (175 mg, 0.5 mmol, 1 equiv.), 3-aminobenzene boronic acid hydrate (103 mg, 0.75 mmol, 1.5 equiv), barium hydroxide (103 mg, 0.75 mmol, 1.5 equiv.) and tetrakistriphenylphosphine palladium (58 mg, 0.05 mmol, 0.1 equiv.) in DME-H₂O (1:1, 7.2 mL) was heated at 110 °C for 4 h in a closed vial. Tetrakistriphenylphosphine palladium (25 mg, 0.022 mmol, 0.4 equiv.) and cesium carbonate (160 mg, 0.5 mmol, 1 equiv.) were added and the reaction was further heated at 110°C for 3 h. Tetrakistriphenylphosphine palladium (58 mg, 0.05 mmol, 0.1 equiv.) was added, and the reaction heated at 120 °C for 3 h. The reaction was partitioned between water/EtOAc (1:1), and the aqueous phase was extracted with EtOAc. The organic layer was filtered through small SiO₂-celite column to give 0.32 g of a crude product as an oil. Crude product was purified by chromatography on SiO₂ (5 g), eluting with a CH₂Cl₂/hexanes gradient (1:3 to 2:3) to afford 113 mg (as a yellow solid) of a crude product containing two spots according to TLC (EtOAc/hexanes, 1:3). This crude product was dissolved in MTBE (3 mL) then the impurity was precipitated by addition of hexane (∼6 mL). The mixture was cooled at -20 °C and the impurity was filtered off. The mother liquor was concentrated to afford I-8 (64 mg, 35 %) as yellow crystals. *R_{f}* = 0.17 (EtOAc/hexanes, 1:3); LC-MS (ESI⁺): 364 (M+1) (95%). ¹H-NMR (500 MHz, CDCl₃) confirmed the structure.

Synthesis of 4,5-Dichloro-thiophene-2-sulfonic acid[3-(1-methyl-3-naphthalen-2-ylmethyl-1H-indol-4-yl)-phenyl]-amide, B03: A solution of I-8 (20 mg, 0.055 mmol, 1 equiv.), 2,3-dichlorothiophene-5-sulfonyl chloride (14 mg, 0.055 mmol, 1 equiv.) and DMAP (0.3 mg, 0.0028 mmol, 0.05 equiv.) in pyridine (0.2 mL) was stirred at rt for 2 h . The reaction was then quenched with 10% aqueous HCl and extracted with ethyl acetate. The organic layer was washed with water, brine, and dried over MgSO₄. The solution was filtered, and concentrated *in vacuo* to afford crude product (35 mg) as a red oily solid. The crude product was purified by chromatography on SiO₂ (1 g), eluting with an ethyl acetate/hexanes gradient (3:17 to 1:1) to afford B03 (13 mg, 41%) as white foam. *R_{f}*= 0.30 (EtOAc/hexanes, 1:3). LC-MS (92 %): ESI⁻ Calcd. 576 (M) Found: 577.3 (M-1). ¹H-NMR (400 MHz, CDCl₃) 3.74 (s, 2H), 3.78 (s, 3H), 6.03 (br s, 1H), 6.76 (s, 1H), 6.78 (m, 1H), 6.83 (dd, J = 6.4, 1.2 Hz, 1H), 7.01 (dd, J = 8.4, 1.6 Hz, 1H), 7.07 (s, 1H), 7.15 (m, 1H), 7.18 (m, 1H), 7.20 (m, 1H), 7.25 (m, 1H), 7.34 (dd, J = 6.4,0.8 Hz, 1H), 7.41-7.44 (m, 2H), 7.63 (m, 1H), 7.65 (d, J = 7.6, 1H), 7.79 (m, 1H).

### Example 3. Preparation of B04.

Synthesis of 4-(1-Methyl-3-naphthalen-2-ylmethyl-1H-indol-4-yl)-phenylamine, I-9. A mixture of I-3 (175 mg, 0.5 mmol, 1 equiv.), 4-(4,4,5,5-tetramethyl)-1,3,2-dioxaborolan-2-yl) aniline (164 mg, 0.75 mmol, 1.5 equiv), tetrakistriphenylphosphine palladium (58 mg, 0.05 mmol, 0.1 equiv.) and cesium carbonate (244 mg, 0.75 mmol, 1.5 equiv.) in DME (3.8 mL) was heated at 120 °C for 3 h in a closed vial. The cooled reaction mixture was diluted with ethyl acetate and filtered through small SiO₂-celite column to give 0.34 g of a crude product as an oil. Crude product was purified by chromatography on SiO₂ (2 g), eluting with a CH₂Cl₂/hexanes gradient (1:3 to 1:1) to afford I-9 (88 mg, 49 %) as white foamy solid. *R_{f}*= 0.22 (EtOAc/hexanes, 1:3); LC-MS (ESI⁺): 364 (M+1) (96%). ¹H-NMR (500 MHz, CDCl₃) confirmed the structure.

Synthesis of 4,5-Dichloro-thiophene-2-sulfonic acid[4-(1-methyl-3-naphthalen-2-yhnethyl-1H-indol-4-yl)-phenyl]-amide, B04. A solution of I-9 (20 mg, 0.055 mmol, 1 equiv.), 2,3-dichlorothiophene-5-sulfonylchloride (14 mg, 0.055 mmol, 1 equiv.) and DMAP (0.3 mg, 0.0028 mmol, 0.05 equiv.) in pyridine (0.2 mL) was stirred at rt for 2 h. The reaction was quenched with 10% aqueous HCl and extracted with ethyl acetate. The organic layer was washed with water, brine, and then dried over MgSO₄. The solution was filtered, and concentrated *in vacuo* to afford a crude product (39 mg) as a pink oil. The crude product was purified by chromatography on SiO₂ (1 g), eluting with ethyl acetate/hexanes, 1:8 to afford B04 (8 mg, 25%) as off white foamy solid. *R_{f}*= 0.30 (EtOAc/hexanes, 1:3). ¹H-NMR (400 MHz, CDCl₃) 3.72 (s, 2H), 3.75 (s, 3H), 6.54 (br s, 1H), 6.66 (s, 1H), 6.91 (dd, J = 7.2, 1.2 Hz, 1H), 7.00-7.05 (m, 2H), 7.05 (s, 1H), 7.23 (s, 1H), 7.24-7.28 (m, 3H), 7.29 (m, 1H), 7.33 (dd, J = 7.2, 1.2 Hz, 1H), 7.40 (m, 2H), 7.60 (d, J = 8.4 Hz, 1H), 7.61-7.7.64 (m, 1H), 7.74-7.76 (m, 1H). LC-MS (89 %): ESI⁻ Calcd. 576 (M) Found: 577.3 (M-1).

### Example 4. Preparation of B17.

Synthesis of N-[4-(1-Methyl-3-naphthalen-2-ylmethyl-1H-indol-4-yl)-phenyl]-acetamide, B17. To a solution of aryl amine I-9 (0.06 mmol) in THF (0.2 mL) was added triethylamine (2 eq.), followed by 2 eq. of acetic anhydride at 0°C. The reaction mixture was stirred at rt for 4h. The reaction mixture was concentrated *in vacuo,* diluted with ethyl acetate and washed with 10% aqueous HCl. The organic layer was separated, washed with water, brine, dried to give the crude product. This material was purified by column chromatography to afford the N-acetyl product B17 in 73% yield. ¹H NMR (CDCl₃) 2.2 (s, 3H), 3.73 (s, 3H), 3.78 (s, 2H), 6.62 (s, 1H), 6.91 (dd, J = 6.8, 1.2 Hz, 1H), 7.06 (dd, J = 8.4, 1.6 Hz, 1H); 7.13 (br s, 1H), 7.21- 7.31 (m, 4H), 7.29 (s, 1H), 7.37- 7.41 (m, 4H), 7.61 (d, J = 8.4, 1H), 7.64- 7.65 (m, 1H), 7.73-7.75 (m, 1H). LCMS (APCI⁺): 405 (M+1), 94%.

### Example 5. Preparation of B18.

Synthesis of 2,2,2-Trifluoro-N-[4-(1-methyl-3-naphthalen-2-yhnethyl-1H-indol-4-yl)-phenyl]-acetamide, B18. To a solution of aryl amine I-9 (0.06 mmol) in THF (0.2 mL) was added triethylamine (2 eq.) and 2 eq. of trifluoroacetic anhydride at 0°C. The reaction mixture was stirred at rt for 4 h. The reaction mixture was concentrated *in vacuo,* diluted with ethyl acetate and washed with 10% aqueous HCl. The organic layer was separated, washed with water, brine, dried, filtered, and concentrated *in vacuo* to give the crude product. This crude product was purified by column chromatography to afford the N-trifluoroacetyl product in 51% yield. ¹H NMR (CDCl₃) 1.25 (s, 3H), 3.77 (s, 3H), 3.79 (s, 2H), 6.71 (s, 1H), 6.89 (dd, J = 6.4, 0.8 Hz, 1H), 7 (dd, J = 8.4, 1.2 Hz, 1H), 7.2 (br s, 1H), 7.23- 7.27 (m, 3H), 7.29 (s, 1H), 7.33- 7.39 (m, 4H), 7.57- 7.6 (m, 2H), 7.73-7.76 (m, 2H). LCMS (APCI⁻): 457 (M-1), 100%.

### Example 6. Preparation of B05.

Synthesis of N-[4-(1-Methyl-3-naphthalen-2-ylmethyl-1H-indol-4-yl)-phenyl] methanesulfonamide, B05. To a solution of I-9 (50 mg, 0.138 mmol) in pyridine (0.25 mL) cooled to 0 °C, was added methanesulfonyl chloride (31.6 mg, 2 eq.). The reaction mixture was stirred at rt for 3 h. The reaction mixture was concentrated *in vacuo,* and 10% aqueous HCl was added, and the aqueous layer was extracted with ethyl acetate (2 x 10 mL). The combined organic layers were washed with water, brine, dried (MgSO₄), filtered and concentrated *in vacuo* to afford crude product. The crude product was purified by column chromatography, eluting with ethyl acetate/hexanes (1:4) to afford 57 mg of product B05, 93.7% yield. ¹H NMR (CDCl₃) 2.99 (s, 3H), 3.76 (s, 3H), 3.8 (s, 2H), 6.54 (br s, 1H), 6.7 (s, 1H), 6.9 (dd, J = 7.2, 0.8 Hz, 1H), 7.02 (dd, J = 8.4, 1.6 Hz, 1H), 7.08 (dd, J = 8.4, 2 Hz, 2H), 7.21 (br s, 1H), 7.25- 7.27 (m, 1H), 7.28 (dd, J = 8.4, 2 Hz, 2H), 7.33 (dd, J = 8.4, 1.2 Hz, 1H), 7.37- 7.4 (m, 2H), 7.6 (d, J = 8.4 Hz, 1H), 7.62- 7.65 (m, 1H), 7.73-7.75 (m, 1H). LCMS (APCI-): 439 (M-1), 100%.

### Example 7. Preparation of B08.

Synthesis of C,C,C-Trifluoro-N-[4-(1-methyl-3-naphthalen-2-ylmethyl-1H-indol-4-yl)-phenyl]-methanesulfonamide, B08. To a solution of I-9 (50 mg, 0.138 mmol) and triethylamine (14 mg, 2 eq.) in methylene chloride (0.25 mL) cooled at -78°C, was added dropwise a solution of triflic anhydride (58 mg, 1.5 eq.) in methylene chloride (0.25 mL). The reaction mixture was slowly warmed to rt and stirred for 4 h. The reaction was quenched with 10% aqueous HCl and extracted with ethyl acetate (2 x 10 mL). The combined organic layers were washed with water, brine, dried (MgSO₄), filtered and concentrated *in vacuo.* The crude product was purified by column chromatography, eluting with ethyl acetate/hexanes (1:9) to afford 40 mg product B08, 58.8% yield. ¹H NMR (CDCl₃) 3.76 (s, 2H), 3.78 (s, 3H), 6.64 (br s, 1H), 6.75 (s, 1H), 6.88 (dd, J = 6.8, 1.2 Hz, 1H), 6.97 (dd, J = 8.4, 1.6 Hz, 1H), 7.1 (dd, J = 6.4, 1.6 Hz, 1H), 7.22 (br s, 1H), 7.25- 7.27 (m, 3H), 7.34 (dd, J = 8.4, 1.2 Hz, 1H), 7.36- 7.4 (m, 3H), 7.59 (d, J = 8, 1H), 7.61- 7.63 (m, 1H), 7.73-7.76 (m, 1H). LCMS (APCI⁻): 494 (M-1), 100%.

### Example 8. Preparation of B 10.

Synthesis of 7-Bromo-5-fluoro-3-methyl-1H-indole, I-10: This compound was prepared according to the known method (Dobbs, A., J. Org. Chem., 66, 638-641 (2001).

Synthesis of 7-Bromo-1-(2,4-dichloro-benzyl)-5-fluoro-3-methyl-1H-indole, I-11: NaH (60% in mineral oil, 526 mg, 13.15 mmol, 1.5 equiv.) was added to solution of I-10 (2 g, 8.77 mmol, 1 equiv.) in DMF (30 mL) at -10 °C. The reaction mixture was allowed to warm to rt and stirred for 30 min. A solution of 2,4-dichlorobenzyl chloride (2.06 g, 10.52 mmol, 1.2 equiv.) in DMF (10 mL) was added over 2.5 min at -10 °C. The reaction mixture was allowed to warm to rt and stir for 1 h. The reaction mixture was added to a stirred mixture of 10 % aqueous HCl/water/ether (1:1:2, 40 mL). The aqueous layer was extracted with ether (2 x 10 mL). The combined organic layers were washed with water (3 x 75 mL), brine (75 mL), dried over MgSO₄, filtered, and *concentrated in vacuo* to afford crude product as brown solid. Ether (4 mL) was added to the crude product and the resulting suspension was cooled to -78 °C and filtered to afford I-11 (2.49 g, 73%) as off-white solid. *R_{f}*= 0.70 (EtOAc/hexanes, 1:5). ¹H-NMR (500 MHz, CDCl₃) confirmed the structure.

Synthesis of 1-(2,4-Dichloro-benzyl)-5-fluoro-3-methyl-1H-indole-7-carboxylic acid ethyl ester, I-12. n-BuLi (1.6 M in hexanes, 0.97 mL, 1.55 mmol, 1.5 equiv.) was added over 7 min, under an Ar atmosphere to a solution of I-11 (400 mg, 1.03 mmol, 1 equiv.) in ether (7 mL) at -78 °C. The reaction mixture was stirred at -78 °C for additional 30 min. Ethyl chloroformate (0.2 mL, 2.07 mmol, 2 equiv.) was then added slowly to the reaction mixture and it was allowed to warm to rt (water bath) and stirred at rt for 30 min. The reaction mixture was quenched with 10% aqueous HCl (5 mL). The organic layer was washed with water (2 x 10 mL), brine (10 mL), dried over MgSO₄, filtered, and concentrated *in vacuo* to afford I-12 (386 mg, 98%) as a brown oil. *R_{f}*= 0.45 (EtOAc/hexanes, 1:19). MS (AP⁺): 380, 382 (M+1). ¹H-NMR (500 MHz, CDCl₃) confirmed the structure.

Synthesis of 1-(2,4-Dichloro-benzyl)-S-fluoro-3-methyl-1H-indole-7-carboxylic acid hydrazide, I-13. A solution of I-12 (114 mg, 0.3 mmol, 1 equiv.) and hydrazine (0.1 mL, 1.5 mmol, 10 equiv.) in ethanol (0.5 mL) was heated at 120 °C in a closed vial overnight. The reaction mixture was quenched by addition of 10% aqueous HCl at 0 °C, and then extracted with ethyl acetate. The organic layer was washed with water, brine, dried over MgSO₄, filtered, and concentrated *in vacuo* to afford a crude product (100 mg). The crude product was triturated with MTBE to afford pure I-13 (72 mg, 66 %) as a beige solid. *R_{f}*= 0.52 (EtOAc/hexanes, 1:1). MS (AP⁺): 366, 368 (M+1). ¹H-NMR (500 MHz, CDCl₃) confirmed the structure.

Synthesis of 5-[1-(2,4-Dichloro-benzyl)-5-fluoro-3-methyl-1H-indol-7-yl]-[1,3,4]oxadiazol-2-ylamine, I-14. A solution of sodium bicarbonate (16 mg, 0.188 mmol, 1 equiv.) in water (0.45 mL) was added to a solution of I-13 (69 mg, 0.18 mmol, 1 equiv.) in dioxane (0.5 mL) at rt and stirred for 5 min to afford a suspension. Cyanogen bromide (20 mg, 0.184 mmol, 1.02 equiv.) was added at rt and the reaction mixture was stirred at rt for 2 h. Hexanes (2 mL) was added and suspension was filtered to afford I-14 (54 mg, 73 %) as a beige solid. *R_{f}*= 0.45 (EtOAc/hexanes, 1:1). LC-MS (ESI⁺): 391, 393 (M+1) (97%). ¹H-NMR (500 MHz, CDCl₃₎ confirmed the structure.

Synthesis ofN- {5-[1-(2,4-Dichloro-benzyl)-5- fluoro-3-methyl-1H-indol-7-yl]-[1,3,4]oxadiazol-2-yl}-2,2,2-trifluoro-acetamide, B-10. Trifluoroacetic anhydride (13 mg, 0.061 mmol, 1.5 equiv.) was added to a suspension of I-14 (15 mg, 0.041 mmol, 1 equiv.) in triethylamine (8 mg, 0.082 mmol, 2 equiv.) and methylene chloride (0.2 mL) at -78 °C. The reaction mixture was allowed to warm to rt over 10 min. The reaction mixture was then quenched with 10% aqueous HCl and extracted with methylene chloride. The organic layer was washed with water, brine, dried over MgSO₄, filtered, and concentrated *in vacuo* to afford B10 (17 mg, 91 %) as a yellow solid. *R_{f}*= 0.17 (EtOAc/hexanes, 1:1). ¹H-NMR (400 MHz, DMSO-d₆) 2.24 (s, 3H), 5.60 (s, 2H), 6.04 (d, J = 8.4 Hz, 1H), 7.20 (dd, J = 8.4, 2.4 Hz, 1H), 7.34 (dd, J = 8.8, 2.4 Hz, 1H), 7.46 (br s, 1H), 7.48 (d, J = 2.0 Hz, 1H), 7.74 (dd, J = 8.8,2.4 Hz, 1H). LC-MS (90%): ESI⁻
Calcd. 486 (M) Found: 485.4 (M-1).

### Example 9. Preparation of B11.

Synthesis of N-{S-[1-(2,4-Dichloro-benzyl)-5-fluoro-3-methyl-1H-indol-7-yl]-[1,3,4]oxadiazol-2-yl}-methanesulfonamide, B11. Methane sulphonyl chloride (13 mg, 9 µL, 0.11 mmol, 2 equiv.) was added to a solution of I-14 (22 mg, 0.056 mmol, 1 equiv.) in pyridine (0.2 mL) at rt. The reaction mixture was stirred at rt overnight and then heated to 70 °C for 2 h. The reaction mixture was quenched with 10% aqueous HCl and extracted with ethyl acetate. The organic layer was washed with water, brine, dried over MgSO₄, filtered, and concentrated *in vacuo.* The resulting oil was chromatographed on SiO₂ (0.5 g), eluting with an ethyl acetate/hexanes gradient (1:3 to 1:1), followed by pure ethyl acetate to afford B11 (6.8 mg, 26 %) as an orange solid. *R_{f}*= 0.24 (EtOAc). ¹H-NMR (400 MHz, CDCl₃) 2.05 (s, 3H), 3.11 (s, 3H), 5.60 (s, 2H), 6.07 (d, J = 8.4 Hz, 1H), 7.00 (dd, J = 8.4, 2.4 Hz, 1H), 7.01 (s, 1H), 7.27 (dd, J = 8.4, 2.4 Hz, 1H), 7.40 (d, J = 2.0 Hz, 1H), 7.50 (dd, J = 8.4, 2.4 Hz, 1H). LC-MS (96 %): ESI⁻ Calcd. 470 (M) Found: 469.2 (M-1).

### Example 10. Preparation of B12.

Synthesis of N-{5-[1-(2,4-Dichloro-benzyl)-5-fluoro-3-methyl-1H-indol-7-yl]-[1,3,4]oxadiazol-2-yl}-2,4,5-trifluoro-benzenesulfonamide, B12. A solution of 2,4,5-trifluorobenzenesulfonyl chloride (92 mg, 0.4 mmol, 4 equiv.) in pyridine (0.3 mL) was added to a mixture of I-14 (39 mg, 0.1 mmol, 1 equiv.) and DMAP (49 mg, 0.4 mmol, 4 equiv.) at rt. The reaction mixture was heated to 90 °C for 2 h. The reaction mixture was then quenched with 10% aqueous HCl and extracted with ethyl acetate. The organic layer was washed with water, brine, dried over MgSO₄, filtered, and concentrated *in vacuo.* The resulting oil was chromatographed on SiO₂ (0.5 g), eluting with CH₂Cl₂ to afford B12 (10 mg, 17 %) as a yellow oil. *R_{f}*= 0.40 (EtOAc). ¹H-NMR (400 MHz, CDCl₃) 2.34 (d, J = 1.2 Hz, 3H), 5.64 (s, 2H), 6.05 (d, J = 8.4 Hz, 1H), 6.97 (dd, J = 8.4, 2.0 Hz, 1H), 7.03 (s, 1H), 7.08 (m, 1H), 7.29 (dd, J = 9.6, 2.4 Hz, 1H), 7.35 (d, J = 2.0 Hz, 1H), 7.51 (dd, J = 8.4, 2.4 Hz, 1H), 7.86 (m, 1H). LC-MS (95 %): ESI⁻
Calcd. 586 (M) Found: 585.1 (M-1).

### Example 11. Preparation of B 13.

Synthesis of 4,5-Dichloro-thiophene-2-sulfonic acid {5-[1-(2,4-dichloro-benzyl)-5-fluoro-3-methyl-1H-indol-7-yl]-[1,3,4]oxadiazol-2-yl}-amide, B13. A solution of 2,3-dichlorothiophene-5-sulfonyl chloride (75 mg, 0.3 mmol, 3 equiv.) in pyridine (0.20 mL) was added to a solution of I-14 (39 mg, 0.1 mmol, 1 equiv.) and DMAP (37 mg, 0.3 mmol, 3 equiv.) in pyridine (0.15 mL) at rt. The reaction mixture was heated to 70 °C for 2 h. The reaction mixture was then quenched with 10% aqueous HCl and extracted with ethyl acetate. The organic layer was washed with water, brine, dried over MgSO₄, filtered, and concentrated *in vacuo.* The resulting oil was chromatographed on SiO₂ (1 g), eluting with CH₂Cl₂ to afford B13 (17 mg, 27 %) as a white solid. *R_{f}=* 0.38 (EtOAc).
¹H-NMR (400 MHz, DMSO-d₆) 2.30 (d, J = 0.8 Hz, 3H), 5.59 (d, J = 0.4 Hz, 2H), 5.92 (d, J = 8.4 Hz, 1H), 7.15 (dd, J = 8.4, 2.0 Hz, 1H), 7.24 (dd, J = 9.6, 2.0 Hz, 1H), 7.27 (m, 1H), 7.45 (br s, 1H), 7.70 (dd, J = 8.4, 2.8 Hz, 1H), 7.71 (s, 1H). LC-MS (96 %): ESI⁻ Calcd. 606 (M) Found: 605.4 (M-1).

### Example 12. Preparation of B06.

Synthesis of 7-Bromo-l-(3,4-diftuoro-benzyl)-5-fluoro-3-methyl-1H-indole, I-15. To a suspension ofNaH (60% in mineral oil, 263 mg, 10.5 mmol, 1.5 equiv.) in DMF (20 mL) was added 7-bromo-5-fluoro-3-methyl-1H-indole, I-10 (1 g, 4.38 mmol, 1 equiv.) at -10 °C. The reaction mixture was allowed to warm to rt and stir for 30 min. 3,4-Difluorobenzyl bromide (0.95 g, 4.6 mmol, 1.05 .equiv.) was added over 2.5 min at -10 °C. The reaction mixture was allowed to warm to rt and stir for 1 h. The reaction mixture was added to stirring solution of 10 % aqueous HCl/water/ether (1:1:2, 40 mL). The layers were separated and the aqueous layer was extracted with ether (2 x 20 mL). The combined organic layers were washed with water (3 x 75 mL), brine (25 mL), dried over MgS0₄, filtered, and concentrated *in vacuo* to afford crude product as a brown oil. The crude product was purified via column chromatography, eluting with ethyl acetate/hexanes (2.5%) to afford 1.4 g of I-15 in 90% yield. ¹H-NMR (500 MHz, CDCl₃) confirmed the structure.

Synthesis of 4-[1-(3,4-Difluoro-benzyl)-5-fluoro-3-methyl-1H-indol-7-yl]-phenylamine, I-16. A mixture of I-15 (345 mg, 0.974 mmol), 4-(4,4,5,5-tetramethyl)-1,3,2-dioxaborane-2-yl) aniline (320 mg, 1.46 mmol), tetrakistriphenylphosphine palladium (60 mg, 0.048 mmol) and cesium carbonate (476 mg, 1.46 mmol) in DMF (4 mL) was heated at 120 °C for 3 h in a closed vial. Reaction mixture was cooled to rt, partitioned between water and EtOAc. The aqueous layer was extracted with EtOAc (2 x 20 mL). The combined organic layers were washed with water, brine, dried (MgSO4) and concentrated. The crude product was chromatographed on SiO₂ with 10%- 20% EtOAc/hexanes solvent mixture, to afford I-16 (180 mg, 50.6 % yield) as a white foam. ¹H-NMR (400 MHz, CDCl₃), 2.31 (s, 3H), 3.75 (br s, 2H), 4.86 (s, 2H), 6.19-6.22 (m, 1H), 6.27- 6.32 (m, 1H), 6.59 (dd, J = 8.4, 2 Hz, 2H), 6.71 (dd, J = 8.8, 2.4 Hz, 1H), 6.85 (s, 1H), 6.88- 6.91 (m, 1H), 6.94 (dd, J = 8.4, 2 Hz, 2H), 7.17
(dd, J = 8.8, 2.4 Hz, 1H).
LCMS (ESI⁺): 367(M+1), 91%.

Synthesis of N-{4-[1-(3,4-Difluoro-benzyl)-5-fluoro-3-methyl-1H-indol-7-yl]-phenyl}-methanesulfonamide, B06. To a solution of I-16 (50 mg, 0.136 mmol) in pyridine (0.25 mL) at 0 °C, was added methanesulfonyl chloride (31.3 mg, 2 eq.). The reaction mixture was stirred at rt for 3 h. The reaction mixture was *concentrated in vacuo,* and 10% aqueous HCl was added, followed by extraction of the aqueous layer with ethyl acetate (2 x 10 mL). The combined organic layers were washed with water, brine, dried (MgSO₄), filtered and concentrated *in vacuo.* The crude product was purified by column chromatography, eluting with ethyl acetate/hexanes (1:4) to afford 57 mg of B06, (50% yield). ¹H-NMR (400 MHz, CDCl₃), 2.32 (s, 3H), 3.1 (s, 3H), 4.83 (s, 2H), 6.1- 6.13 (m, 1H), 6.16- 6.21 (m, 1H), 6.5 (br s, 1H), 6.69 (dd, J= 9.6, 2.4 Hz, 1H), 6.84- 6.91 (m, 2H), 7.1- 7.14 (m, overlap, 4H), 7.23 (dd, J= 8.8, 2.4 Hz, 1H). LCMS (ESI-): 443 (M-1), 97%.

### Example 13. Preparation of B07.

Synthesis ofN-{4-[1-(3,4-Difluoro-benzyl)-5-fluoro-3-methyl-IH-indol-7-yl]-phenyl}-C,C,C-trifluoro-methanesulfonamide, B07. To a solution of I-16 (63 mg, 0.172 mmol) and triethylamine (35 mg, 2 eq.) in methylene chloride (0.7 mL) at -78°C, was added dropwise a solution of triflic anhydride (48.5 mg, 1.5 eq.) in methylene chloride (0.25 mL). The reaction mixture was slowly warmed to rt and stirred for 4 h. The reaction was quenched with 10% aqueous HCl and extracted with ethyl acetate (2 x 10 mL). The combined organic layers were washed with water, brine, dried (MgSO₄), filtered and concentrated *in vacuo.* The crude product was purified by column chromatography, eluting with ethyl acetate/hexanes (1:9) to afford 40 mg of product B07, 36.5% yield. ¹H-NMR (400 MHz, CDCl₃), 2.34 (s, 3H), 4.8 (s, 2H), 6.05- 6.06 (m, 1H), 6.1- 6.15 (m, 1H), 6.69 (dd, J = 9.2, 2.4 Hz, 1H), 6.83- 6.89 (m, 2H), 6.91 (s, 1H), 7.12- 7.2 (m, 4H), 7.25 (dd, J = 9.2,2.4 Hz, 1H). LCMS (APCF): 497 (M-1), 97%.

### Example 14. Preparation ofB14.

Synthesis of 1-(3,4-Difluoro-benzyl)-5-fluoro-3-methyl-1H-indole-7-carbonitrile, I-17. A solution of I-15 (1.1 g, 3.106 mmol, 1 equiv.) and copper(I) cyanide (834 mg, 9.32 mmol, 3 equiv) in anhydrous dimethyl acetamide (3.5 mL) was degassed with argon for 15 min at rt and then heated at 210 °C in a closed vial for 1.5 h. Water and EtOAc (30 mL each) was added and mixture was filtered. The solid residue was washed with ethyl acetate. The organic layer was separated, washed with water (3 x 50 mL), brine (30 mL), dried over MgSO₄, filtered and concentrated to afford I-17 (903 mg, 97%) as a solid compound. ¹H-NMR (500 MHz, CDCl₃) confirmed the structure.

Synthesis of (Z)-3-Amino-3-[1-(3,4-difluoro-benzyl)-5-fluoro-3-methyl-1H-indol-7-yl]-acrylonitrile, I-18. n-BuLi (1.6 M, 5.8 mL, 9.324 mmol, 4 equiv.) was added dropwise to a solution of diisopropylamine (1.3 ml, 9.324 mmol, 4 equiv.) in anhydrous THF (4 mL) at -78 °C. A solution of I-17 in anhydrous acetonitrile (0.49 mL) and THF (1.8 mL) was added. The reaction mixture was allowed to warm to rt and stir for 1.5 h. The reaction was quenched with saturated NH₄Cl (20 mL), and extracted with ethyl acetate (20 mL). The organic layer was washed with brine, dried and concentrated *in vacuo* to give crude I-18 (754 mg) as dark brown oil, which crystallized upon standing at rt. ¹H-NMR (500 MHz, CDCl₃) confirmed the structure.

Synthesis of 5-[1-(3,4-Difluoro-benzyl)-5-fluoro-3-methyl-1H-indol-7-yl]-2-methyl-2H-pyrazol-3-ylamine, I-19. To a mixture of I-18 (150 mg, 0.438 mmol) in isopropanol (0.2 mL) and acetic acid (0.2 mL) was added methylhydrazine (100 mg, 0.115 ml, 2.19 mmol, 5 equiv.) at rt. The reaction mixture was heated to 100 °C overnight. The reaction mixture was concentrated *in vacuo* and partitioned between water and ethyl acetate. The organic layer was washed with water, brine, dried (MgSO₄) filtered, and concentrated *in vacuo* to give 100 mg of crude product. Purification by column chromatography on silica gel, eluting with methylene chloride afforded 40 mg of I-19, 25% yield. ¹H-NMR (500 MHz, CDCl₃) confirmed the structure.

Synthesis of N-{5-[1-(3,4-Difluoro-benzyl)-5-fluoro-3-methyl-1H-indol-7₋yl]-2-methyl-2H-pyrazol-3-yl}-methanesulfonamide, B14. To a mixture of I-19 (18 mg, 0.048 mmol) in pyridine (0.1 mL) was added methanesulfonyl chloride (12 mg, 2 equiv.) at 0 °C. The mixture was stirred at rt for 2 h, then heated at 60 °C for 6 h. The reaction mixture was concentrated *in vacuo,* and diluted with ethyl acetate (10 mL). The organic layer was washed with 10% aqueous HCl (2 mL), water, brine, dried (MgSO₄), filtered and concentrated *in vacuo* to afford 20 mg of crude product. The crude product was triturated with a mixture of ether/hexanes (2:1) and filtered to afford 14 mg of B14. ¹H NMR (CDCl₃) 2.32 (s, 3H), 2.99 (s, 3H), 3.92 (s, 3H), 5.29 (s, 2H), 6.07 (s, 1H), 6.13 (br s, 1H), 6.27-6.32 (m, 1H), 6.32- 6.36 (m, 1H), 6.83 (dd, J = 9.6, 2.4 Hz, 1H), 6.9 (dd, J= 8.4, 2 Hz, 1H), 6.95 (s, 1H), 7.25 (dd, J= 8.8, 2.8 Hz, 1H). LCMS (ESI-): 448 (M-1), 89%.

### Example 15. Preparation of B15.

Synthesis of 4,5-Dichloro-thiophene-2-sulfonic acid {5-[1-(3,4-difluoro-benzyl)-5-fluoro-3-methyl-1H-indol-7-yl]-2-methyl-2H-pyrazol-3-yl}-amide, B15. A mixture of I-19 (15 mg, 0.04 mmol) and 2,3-dichorothiophene-5-sulphonylchloride (12.2 mg, 0.048 mmol) in pyridine (0.1 mL) was heated to 60 °C overnight. TLC analysis showed only ~50% conversion of the reaction. DMAP (9.8 mg, 2 eq.) was added and the mixture heated to 60°C again overnight. The reaction mixture was concentrated *in vacuo,* diluted with ethyl acetate and washed with 10% aqueous HCl. The organic layer was washed with water, brine, dried (MgSO₄) filtered and concentrated *in vacuo* to afford 20 mg of crude product. The crude product was purified by preparative TLC using 1% MeOH/methylene chloride to afford 10 mg of B15. ¹H NMR (CDCl₃) 2.24 (s, 3H), 3.65 (s, 3H), 5.31 (s, 2H), 5.98 (br s, 1H), 6.29- 6.32 (m, 1H), 6.46- 6.51 (m, 1H), 6.79 (dd, J = 9.6, 2.4 Hz, 1H), 7.05- 7.14 (m, 2H), 7.29 (dd, J = 9.2, 2.4 Hz, 1H), 7.34 (s, 1H), 7.50 (br s, 1H). LCMS (ESI-): 585 (M-1), 91%.

### Example 16. Preparation of B16.

Synthesis of 1-(3,4-Difluoro-benzyl)-5-fluoro-3-methyl-1H-indole-7-carboxylic acid, ethyl ester, I-20. n-BuLi (1.6 M in hexanes, 0.64 mL, 1.01 mmol, 1.2 equiv.) was added over 7 min under an Ar atmosphere to a solution of 7-Bromo-1-(3,4-difluoro-benzyl)-5-fluoro-3-methyl-1H-indole, I-15 (300 mg, 0.847 mmol, 1 equiv.) in diethyl ether (15 mL) at -78°C. The reaction mixture was stirred at - 78 °C for an additional 30 min. Ethyl chloroformate (0.09 mL, 1 mmol, 1.2 equiv.) was added dropwise to the reaction mixture and the mixture was allowed to warm to rt and stir for 30 min. The reaction mixture was quenched with 10% aqueous HCl (5 mL) and diluted with ether (15 mL). The organic layer was separated, washed with water (2x10 mL), brine (10 mL), dried over MgSO₄, filtered, and concentrated *in vacuo* to afford crude ester as a brown oil. The residue was purified via column chromatography, eluting with ethyl acetate/hexanes (1:19) to afford 260 mg of I-20. ¹H-NMR (500 MHz, CDCl₃) confirmed the structure.

Synthesis of 3-[1-(3,4-Difluoro-benzyl)-5-fluoro-3-methyl-1H-indol-7-yl]-3-oxo-propionitrile, I-21. Dry acetonitrile (50 µL, 1.1 equiv.) was added to a solution of n-BuLi (2.5 M in hexane, 0.375 ml, 0.93 mmol, 1.25 eq.) in anhydrous THF (1.5 mL) at -78°C. This mixture was stirred for 30 min, followed by the dropwise addition of a solution of I-20 in THF (1.5 mL). The reaction mixture was allowed to warm to rt over 3 h. The reaction was quenched with water, followed by the addition of 10% aqueous HCl. This mixture was stirred for 10 min, then extracted with ethyl acetate (3 x 20 mL). The combined organic layers were washed with water, brine, dried, filtered and concentrated *in vacuo* to afford 280 mg crude I-27. ¹H-NMR (500 MHz, CDCl₃) confirmed the structure. The product I-21 was used without further purification for the next step.

Synthesis of 5-[1-(3,4-Difluoro-benzyl)-5-fluoro-3-methyl-1H-indol-7-yl]-isoxazol-3-ylamine, I-22. To a mixture of I-21 (160 mg, 0.46 mmol) and hydroxylamine hydrochloride (86 mg, 1.21 mmol, 2.6 eq.) in ethanol (2.8 mL) was added a solution of sodium hydroxide (48 mg, 1.21 mmol, 2.6 eq.) in water (0.6 mL). The resulting mixture was refluxed for 1 h. The reaction mixture was diluted with water (2 mL), methylene chloride (5 mL), and the pH was adjusted to 1 with 10% aqueous HCl. The organic layer was separated and the pH of the aqueous layer was adjusted to 8 by addition of solid NaHCO3 and was extracted with ethyl acetate (2x10 mL). The combined organic layers were washed with water, brine and concentrated *in vacuo* to afford 80 mg of crude intermediate. This residue was mixed with 2 N aqueous HCl (0.2 mL) and heated to 100°C for 3 h. The mixture was cooled to rt, and the pH was adjusted to 8 using saturated NaHCO3. The aqueous mixture was extracted several times with methylene chloride, and the combined organic layers were washed with water, brine, dried, filtered and concentrated *in vacuo* to afford 100 mg of a crude mixture of isomers 3-amino and 5-aminoisoxazole. The crude material was purified by column chromatography on silica gel, eluting with methylene chloride to afford 35 mg of I-22. ¹H-NMR (500 MHz, CDCl₃) confirmed the structure.

Synthesis of N-{5-[1-(3,4-Difluoro-benzyl)-5-fluoro-3-methyl-1H-indol-7-yl]-isoxazol-3-yl}-methanesulfonamide, B16. To a solution of I-22 (30 mg, 0.084 mmol) in pyridine (0.2 mL) was added dropwise methanesulphonyl chloride (19 mg, 0.168 mmol, 2 eq.). The resulting mixture was heated at 60°C for 6 h. The mixture was concentrated *in vacuo,* diluted with ethyl acetate and washed with 10% aqueous HCl. The organic layer was washed with water, brine, dried, filtered and concentrated *in vacuo* to afford 30 mg of crude product. The crude product was purified by preparative TLC using 1% MeOH/methylene chloride to afford 10 mg of B16. ¹H-NMR (400 MHz, CDCl₃), 2.33 (s, 3H), 3.15 (s, 3H), 5.18 (s, 2H), 6.19 (s, 1H), 6.39 (m, 1H), 6.5 (m, 1H), 6.93- 6.99 (m, 2H), 7.0 (s, 1H), 7.2 (br s, 1H), 7.39 (dd, J= 8.8, 2.4 Hz, 1H). LCMS (ESI-): 435 (M-1), 88%.

### Example 17. Preparation of B19.

Synthesis of (N-{5-[1-(2,4-Dichloro-benzyl)-5-fluoro-3-methyl-1H-indol-7-yl]-[1,3,4]oxadiazol-2-yl}-3,4-difluoro-benzenesulfonamide, B19. A solution of 3,4-difluorobenzenesulfonyl chloride (159 mg, 0.75 mmol, 2.5 equiv.) in pyridine (0.5 mL) was added to a solution of I-14 (117 mg, 0.3 mmol, 1 equiv.) and DMAP (92 mg, 0.75 mmol, 2.5 equiv.) in pyridine (0.8 mL) at rt. The reaction mixture was stirred and heated at 80°C for 0.5 h. The reaction mixture was quenched with 10% aqueous HCl (4 mL) and extracted with EtOAc (4 mL). The organic layer was washed with water (3 x 4 mL), brine (2 mL), dried over MgSO₄, filtered, and concentrated. The resulting oil (154 mg) was triturated with hexane (4 mL) and filtered to afford 145 mg of a solid. The solid was chromatographed on SiO₂ (Flash, 2 g) with CH₂Cl₂ (50 mL), EtOAc/hexanes, 1:3 (30 mL), EtOAc/hexanes, 1:1 (30 mL) to yield a brown oil. The oil was triturated with hexane (2 mL) to afford a title compound B-19 (33 mg, 19 %) as a brown solid. R_{f}=0.40 (EtOAc), ¹H-NMR (400 MHz, DMSO-d₆) 2.24 (s, 3H), 5.54 (br s, 2H), 5.86 (d, J = 8.0 Hz, 1H), 7.08 (dd, J = 8.0, 2.4 Hz, 1H), 7.15 (dd, J = 10.4, 2.4 Hz, 1H), 7.18 (d, J = 2.0 Hz, 1H), 7.38 (s, 1H), 7.57-7.64 (m, 2H), 7.68 (br s, 1H), 7.86 (m, 1H).
LC-MS (85 %): ESI⁻ Calcd. 566 (M) Found: 565.3 (M-1).

### Example 18. Preparation of B20

Synthesis of (3,4-Dichloro-N-{5-[1-(2,4-dichloro-benzyl)-5-fluoro-3-methyl-1H-indol-7-yl]-[1,3,4]oxadiazol-2-yl}-benzenesulfonamide, B20. A solution of freshly prepared LDA (0.525 mmol, 2.1 equiv.) in THF (0.5 mL) was added dropwise over 5 min to a solution of I-14 (98 mg, 0.25 mmol, 1 equiv.) and HMPA (87 mg, 0.50 mmol, 2.1 equiv.) in THF (0.5 mL) at -78°C. The reaction mixture was stirred for 15 min at -78°C. A solution of 3,4-dichlorobenzenesulfonyl chloride (153 mg, 0.625 mg, 2.5 equiv.) in THF (0.5 mL) was added dropwise over 3 min and the reaction mixture was slowly warmed in 1 h to -0°C, stirred for 1 h at -0°C and slowly warmed in 1 h to rt. The reaction mixture was cooled to -78°C, quenched by slow addition of 10% aqueous HCl (4 mL) and extracted with EtOAc (2 x 4 mL). The combined organic phases were washed with water (2 x 4 mL), brine (4 mL), dried over MgSO₄ filtered, and concentrated to yield crude product (140 mg) as an orange oil. Purification by chromatography on SiO₂ (Flash, 2 g) with CH₂Cl₂ yielded a crude product (10 mg) as a yellow oil. The oil was washed with hexane to afford a title compound B20, (10 mg, 7 %) as a yellowish solid. R_{f} 0.18 (EtOAc). ¹H-NMR (400 MHz, DMSO-d₆) 2.34 (s, 3H), 5.57 (s, 2H), 6.00 (d, J = 8.4 Hz, 1H), 6.94 (dd, J = 8.4, 2.0 Hz, 1H), 7.01 (s, 1H), 7.22 (dd, J = 6.4, 2.0 Hz, 1H), 7.50 (dd, J = 8.4, 2.4 Hz, 1H), 7.62 (d, J = 8.8 Hz, 1H), 7.78 (dd, J = 8.4, 2.0 Hz, 1H), 8.50 (d, J = 2.0 Hz, 1H). LC-MS (91 %): BSI⁻ Calcd. 598 (M) Found: 599.1 (M+1).

### Example 19. Preparation of B21.

Synthesis of B21. A solution of diphenylphosphinic chloride (35 mg, 0.15 mmol, 1.5 equiv.) in pyridine (0.1 mL) was added to a solution of I-14 (39 mg, 0.1 mmol, 1 equiv.) and DMAP (1.2 mg, 0.01 mmol, 0.1 equiv.) in pyridine (0.3 mL) at 60 °C. The reaction mixture was stirred and heated at 60 °C for 16 h. The reaction mixture was quenched with 10% aqueous HCl (2 mL) and extracted with EtOAc (2 x 2 mL). The combined organic layers were washed with water (3 x 4 mL), brine (4 mL), dried over MgSO₄, filtered, and concentrated. The resulting oil (59 mg) was triturated subsequently with hexane (2 x 1 mL) and ether (1.5 mL) and filtered to afford B21 (29 mg, 49 %) as a white solid. R_{f} = 0.37 (EtOAc/hexanes, 1:1). ¹H-NMR (400 MHz, DMSO-d₆) 2.29 (s, 3H), 5.60 (br s, 2H), 5.91 (d, J = 8.4 Hz, 1H), 7.00 (br s, 1H), 7.16 (dd, J = 8.4, 2.0 Hz, 1H), 7.40 (br s, 1H), 7.43 (s, 1H), 7.46-7.58 (m, 7H), 7.66 (dd, J = 8.8, 2.4 Hz, 1H), 7.75-7.80 (m, 4H). LC-MS (91 %): ESI⁻ Calcd. 592 (M) Found: 591.2 (M-1).

### Example 20. Preparation of B22.

Synthesis of {5-[1-(2,4-Dichloro-benzyl)-5-fluoro-3-methyl-1H-indol-7-yl]-[1,3,4]oxadiazol-2-yl}-phosphoramidic acid bis-(2,4-dichloro-phenyl) ester, B-22. A solution of bis(2,4-dichlorophenyl) chlorophosphate (73 mg, 0.18 mmol, 1.2 equiv.) in pyridine (0.2 mL) was added to a solution of 1-14 (59 mg, 0.15 mmol, 1 equiv.) and DMAP (1.8 mg, 0.015 mmol, 0.1 equiv.) in pyridine (0.2 mL) at rt. The reaction mixture was stirred and heated at 60 °C for 2 h and 70°C for 1 h. The reaction mixture was cooled to -78°C and quenched by addition of 10% aqueous HCl (4 mL) and extracted with EtOAc (2 x 2 mL). The combined organic layers were washed with water (3 x 2 mL), brine (2 mL), dried over MgSO₄, filtered, and concentrated. The resulting oil (130 mg) was triturated subsequently with hexane (2 mL) and MTBE (1 mL) and filtered to afford a title compound B-22 (29 mg, 25 %) as a white solid. R_{f}= 0.22 (EtOAc/hexanes, 1:1). ¹H-NMR (400 MHz, DMSO-d₆) 2.30 (d, J = 0.8 Hz, 3H), 5.62 (s, 2H), 5.95 (d, J =8.4 Hz, 1H), 7.16 (dd, J = 8.4, 2.0 Hz, 1H), 7.20 (dd, J = 9.6, 1.6, 1H), 7.30 (dd, J = 8.8, 2.4, 1H), 7.44 (d, J = 2.4 Hz, 1H), 7.45 (s, 1H), 7.47 (d, J = 2.8 Hz, 1H), 7.51 (d, J = 2.4 Hz, 1H), 7.53 (d, J =1.2 Hz, 1H), 7.55 (d, J = 0.8 Hz, 1H), 7.69 (dd, J = 2.4, 0.8 Hz, 1H), 7.73 (dd, J = 8.8, 2.8 Hz, 1H). LC-MS (87 %): ESI⁻
Calcd. 762 (M) Found: 761.1 (M-1).

### Example 21. Preparation of B23.

General Procedure A-1. A solution of the corresponding acyl chloride (0.30 mmol, 1.2 equiv.) in THF (0.15 mL) was added over 1 min to a solution of I-14 (98 mg, 0.25 mmol, 1 equiv.) and cat. DMAP (1.5 mg, 0.0125 mmol, 0.05 equiv.) in pyridine (0.6 mL) at rt and the reaction mixture was stirred at rt from 3-16 h. The reaction mixture was cooled to ~-70 °C (dry ice-acetone bath) and 10% aqueous HCl (4 mL) was added. The mixture was extracted with EtOAc (2 x 2 mL). The combined organic phase was washed with water (3 x 4 mL), brine (4 mL), dried over MgSO₄, filtered, and concentrated to yield crude product as an oil. The oil was crystallized by addition of hexane (2 mL). The resulted solid was washed with ether/hexane, 1:1 (2 mL) to afford the title compound.

Synthesis of (N-{5-[1-(2,4-Dichloro-benzyl)-5-fluoro-3-methyl-1H-indol-7-yl]-[1,3,4]oxadiazol-2-yl}-4-fluoro-benzamide, B23. Following general procedure A-1, 70 mg (55%) of B23 was isolated as a white solid, R_{f} 0.15 (EtOAc/hexanes, 1:1). ¹H-NMR (400 MHz, DMSO-d₆) 2.32 (d, J = 0.8 Hz, 3H), 5.66 (br s, 2H), 6.00 (d, J = 8.4 Hz, 1H), 7.18 (dd, J = 6.4, 2.0 Hz, 1H), 7.33 (dd, J = 9.6, 2.4 Hz, 1H), 7.41 (t, J = 8.8 Hz, 2H), 7.45 (d, J = 2.0 Hz, 1H), 7.49 (br s, 1H), 7.73 (dd, J = 8.8, 2.4 Hz, 1H), 8.06-8.09 (m, 2H). LC-MS (92 %): ESI⁻
Calcd. 514 (M) Found: 513.3 (M-1).

### Example 22. Preparation of B24.

Synthesis of (Isoxazole-5-carboxylic acid {5-[1-(2,4-dichloro-benzyl)-5-fluoro-3-methyl-1H-indol-7-yl]-[1,3,4]oxadiazol-2-yl}-amide, B24. Following general procedure A-1, 41 mg (34%) of B24 was isolated as a white solid, R_{f} 0.17 (EtOAc/hexanes, 1:1).
¹H-NMR (400 MHz, DMSO-d₆) 2.32 (d, J = 0.8 Hz, 3H), 5.65 (br s, 2H), 6.01 (d, J=8.4Hz, 1H), 7.17 (dd, J = 8.4, 2.4 Hz, 1H), 7.34 (dd, J=9.6, 2.8 Hz, 1H), 7.36-7.46 (m, 2H), 7.46 (d, J = 2.0 Hz, 1H), 7.49 (s, 1H), 7.74 (dd, J = 8.8, 2.4 Hz, 1H), 8.86 (br s, 1H).
LC-MS (90 %): ESI⁻ Calcd. 485 (M) Found: 484.3 (M-1).

### Example 23. Preparation of B25.

Synthesis of (3,5-Dichloro-N-{5-[1-(2,4-dichloro-benzyl)-5-flluoro-3-methyl-1H-indol-7-yl]-[1,3,4]oxadiazol-2-yl}-benzamide, B25. Following general procedure A-1, 52 mg (37%) of B25 was isolated as a white solid, R_{f} 0.31 (EtOAc/hexanes, 1:2). ¹H-NMR (400 MHz, DMSO-d₆) 2.32 (d, J =1.2 Hz, 3H), 5.64 (br s, 2H), 6.01 (d, J = 8.4 Hz, 1H), 7.18 (dd, J = 8.0, 2.0 Hz, 1H), 7.33 (dd, J = 9.6, 2.0 Hz, 1H), 7.44 (d, J = 2.0 Hz, 1H), 7.49 (br s, 1H), 7.74 (dd, J = 8.4, 2.4 Hz, 1H), 7.86 (d, J = 2.0 Hz, 1H), 7.95 (br s, 1H), 8.0 (d, J =1.6 Hz, 2H). LC-MS (78 %): ESI⁻
Calcd. 564 (M) Found: 563.1 (M-1).

### Example 24. Preparation of B26.

Synthesis of (N-{5-[1-(2,4-Dichloro-benzyl)-5-fluoro-3-methyl-1H-indol-7-yl]-[1,3,4]oxadiazol-2-yl}-3,4-difluoro-benzamide, B26. Following general procedure A-1, 76 mg (57%) of B26 was isolated as a white solid, R_{f} 0.54 (EtOAc/hexanes, 1:1). ¹H-NMR (400 MHz, DMSO-d₆) 2.32 (d, J = 0.8 Hz, 3H), 5.65 (brs, 2H), 6.01 (d, J = 8.4 Hz, 1H), 7.17 (dd, J=8.4,2.0Hz, 1H), 7.33 (dd, J = 9.2, 2.4 Hz, 1H), 7.45 (d, J = 2.0 Hz, 1H), 7.49 (br s, 1H), 7.66 (q, J = 8.4 Hz, 1H), 7.23 (dd, J = 8.8, 2.4 Hz, 1H), 7.90 (br s, 1H), 8.02-8.08 (m, 1H), 12.28 (br s, 1H). LC-MS (92 %): ESI⁻ Calcd. 532 (M) Found: 531.1 (M-1).

### Example 25. Preparation of B27.

Synthesis of (N-{5-[1-(2,4-Dichloro-benzyl)-5-fluoro-3-methyl-1H-indol-7-yl]-[1,3,4]oxadiazol-2-yl}-2,4-difluoro-benzamide, B27. Following general procedure A-1, 55 mg (41%) of B27 was isolate as a white solid, R_{f} 0.80 (EtOAc/hexanes, 1:1). ¹H-NMR (400 MHz, DMSO-d₆) 2.32 (d, J = 0.8 Hz, 3H), 5.67 (s, 2H), 5.98 (d, J = 8.4 Hz, 1H), 7.17 (dd, J = 8.4, 2.0 Hz, 1H), 7.25 (dt, J = 8.4, 2.0 Hz, 1H), 7.31 (dd, J = 8.4, 2.4 Hz, 1H), 7.43-7.46 (m, 1H), 7.49 (s, 1H), 7.49 (d, J = 2.4 Hz, 1H), 7.73 (dd, J = 8.8, 2.4 Hz, 1H), 7.79 (q, J = 7.2 Hz, 1H), 12.33 (br s, 1H). LC-MS (100 %): APCI⁺ Calcd. 530 (M) Found: 531.0 (M+1).

### Example 26. Preparation of B28.

Synthesis of (2,4-Dichloro-N-{5-[1-(2,4-dichloro-benzyl)-5-fluoro-3-methyl-1H-indol-7-yl]-[1,3,4]oxadiazol-2-yl}-benzamide, B28. Following general procedure A-1, 105 mg (74%) of B28 was isolated as a white solid, R_{f} 0.60 (EtOAc/hexanes, 1:1). ¹H-NMR (400 MHz, DMSO-d₆) 2.32 (d, J = 0.8 Hz, 3H), 5.67 (s, 2H), 5.94 (d, J = 8.4 Hz, 1H), 7.17 (dd, J = 8.4, 2.0 Hz, 1H), 7.28 (br d, J = 8.4 Hz, 1H), 7.48 (br s, 1H), 7.52 (d, J =1.6 Hz, 1H), 7.60 (dd, J = 8.0, 2.0 Hz, 1H), 7.65 (d, J= 8.0 Hz, 1H), 7.73 (dd, J = 8.8, 2.4 Hz, 1H), 7.80 (d, J = 2.0 Hz, 1H), 12.52 (br s, 1H). LC-MS (100 %): APCI⁺ Calcd. 563 (M) Found: 564.0 (M+1).

### Example 27. Preparation of B29.

Synthesis of (2,2-Difluoro-benzo[1,3]dioxole-5-carboxylic acid {5-[1-(2,4-dichloro-benzyl)-5-fluoro-3-methyl-1H-indol-7-yl]-[1,3,4]oxadiazol-2-yl}-amide, B29. Following general procedure A-1, 60 mg (35%) of B29 was isolated as a yellowish solid, R_{f} 0.27 (EtOAc/hexanes, 1:2). ¹H-NMR (400 MHz, DMSO-d₆) 2.32 (d, J = 0.8 Hz, 3H), 5.65 (br s, 2H), 6.00 (d, J = 8.4 Hz, 1H), 7.18 (dd, J = 8.0,2.0 Hz, 1H), 7.36 (br q, J = 8.0 Hz, 2H), 7.45 (d, J = 2.0 Hz, 1H), 7.49 (br s, 1H), 7.67 (br t, J = 8.0 Hz, 2H), 7.74 (dd, J = 8.8, 2.4 Hz, 1H), 12.42 (br s, 1H). LC-MS (100 %): APCI⁺ Calcd. 574 (M) Found: 575.2 (M+1).

### Example 28. Preparation of B30.

Synthesis of (Furan-2-carboxylic acid {5-[1-(2,4-dichloro-benzyl)-5-fluoro-3-methyl-1H-indol-7-yl]-[1,3,4]oxadiazol-2-yl}-amide, B30. Following general procedure A-1, 80 mg (55%) of B30 was isolated as a yellowish solid, R_{f} 0.23 (EtOAc/hexanes, 1:1). ¹H-NMR (400 MHz, DMSO-d₆) 2.32 (d, J = 0.8 Hz, 3H), 5.66 (br s, 2H), 5.99 (d, J = 8.4 Hz, 1H), 6.75 (dd, J = 3.6, 2.0 Hz, 1H), 7.17 (dd, J = 8.4, 2.0 Hz, 1H), 7.33 (dd, J = 8.8, 2.8 Hz, 1H), 7.44 (d, J = 2.0 Hz, 1H), 7.48 (s, 1H), 7.57 (d, J = 3.2 Hz, 1H), 7.72 (dd, J = 8.8, 2.8 Hz, 1H), 8.03 (d, J = 0.8 Hz, 1H), 12.15 (br s, 1H). LC-MS (100 %): APCI⁺ Calcd. 484 (M) Found: 485.2 (M+1).

### Example 29. Preparation of B31.

Synthesis of 4,5-Dichloro-thiophene-2-sulfonic acid {5-[1-(3,4-difluoro-benzyl)-5-fluoro-3-methyl-1H-indol-7-yl]-isoxazol-3-yl}-amide, B31. To a suspension of I-22 (42mg, 0.117 mmol) in pyridine (0.2 mL) was added DMAP (28 mg, 0.23 mmol, 2 eq.). This mixture was heated at 70 °C until solution was achieved, and 2,3-dichlorothiophene-5-sulphonyl chloride (58 mg, 0.23 mmol, 2 eq.) was added. The reaction mixture was stirred at this temperature for 2 h. The cooled reaction mixture was concentrated to an oil and 10% aqueous HCl (1 mL) was added. The mixture was extracted with EtOAc (2 x 5 mL). The combined organic layers were washed with 10% aqueous HCl (1mL), water (2 x 3 mL), brine (2 x 3 mL), dried over MgSO₄, filtered and concentrated to give 55 mg of crude product. Purification by column chromatography using 40% to 10% hexane/ methylene chloride gave 12 mg of B31 (18% yield). ¹H-NMR (400 MHz, CDCl₃), 2.32 (s, 3H), 5.09 (s, 2H), 6.31 (s, 1H), 6.41 (m, 1H), 6.44- 6.49 (m, 1H), 6.92- 6.99 (m, overlap, 2H), 6.96 (s, 1H), 7.4 (dd, J= 8.8,2.4 Hz, 1H), 7.47 (s, 1H), 7.97 (br s, 1H). LC/MS (ESI-): 572 (M-1), 96%.

### Example 30. Preparation of B32

General Procedure (A-2) for Sulfonation of 3-aminoisoxazoles. A 5 mL vial was charged with the corresponding 3-aminoisoxazole (1 equiv.), pyridine (1 mL/0.80 mmol), DMAP (2 equiv.). The reaction mixture was heated to 75°C and sulfonyl chloride (2-3.5 equiv.) was added neat after 2-3 min. A suspension formed immediately and the reaction mixture was stirred and heated at 75 °C for 1 h. The reaction mixture was cooled to rt and 10% aqueous HCl (10 mL/0.80 mmol) was added. The mixture was extracted with EtOAc (10 mL). Organic phase was washed with water (2 x 10 mL), brine (10 mL), dried over MgSO₄, filtered, and concentrated to yield crude product as an oil. The product was purified by SiO₂ flash chromatography (1 g per 0.05 mmol of starting 3-aminoisoxazole using CH₂Cl₂ as eluent) to afford the designated product as a solid.

Synthesis of (N-{5-[1-(3,4-Difluoro-benzyl)-5-fluoro-3-methyl-1H-indol-7-yl]-isoxazol-3-yl}-3,4-difluoro-benzenesulfonamide, B32. The title compound was obtained from I-22 (143 mg, 0.40 mmol) and 3,4-difluorobenzenesulfonyl chloride (212 mg, 1.00 mmol) following general procedure A-2 to afford 93 mg (44%) as a yellow solid (hexane). R_{f} 0.18 (CH₂Cl₂-MeOH, 19:1). ¹H-NMR (400 MHz, CDCl₃) 2.32 (d, J =1.2 Hz, 3H), 5.10 (s, 2H), 6.25 (s, 1H), 6.34-6.39 (m, 1H), 6.40-6.45 (m, 1H), 6.86-6.95 (m, 2H), 6.97 (s, 1H), 7.33 (m, 1H), 7.39 (dd, J = 8.4, 2.4 Hz, 1H), 7.67-7.72 (m, 1H), 7.74-7.78 (m, 1H), 8.10 (br s, 1H). LC-MS (96 %): ESI⁻ Calcd. 532.9 (M-1) Found: 532.6.

### Example 31. Preparation of B33

Synthesis of (N-{5-[1-(3,4-Difluoro-benzyl)-5-fluoro-3-methyl-1H-indol-7-yl]-isoxazol-3-yl}-2,4,5-trifluoro-benzenesulfonamide, B33. The title compound was obtained from I-22 (143 mg, 0.40 mmol) and 2,4,5-trifluorobenzenesulfonyl chloride (323 mg, 1.40 mmol) follwing general procedure A-2 to afford 35 mg (16%) as a yellow solid (hexane). R_{f} 0.13 (CH₂Cl₂-MeOH, 19:1). ¹H-NMR (400 MHz, CDCl₃) 2.32 (d, J = 0.8 Hz, 3H), 5.10 (s, 2H), 6.23 (s, 1H), 6.33-6.41 (m, 1H), 6.41-6.46 (m, 1H), 6.87-6.94 (m, 2H), 6.97 (s, 1H), 7.12 (m, 1H), 7.38 (dd, J = 8.8, 1.6 Hz, 1H), 7.80 (m, 1H), 8.25 (br s, 1H). LC-MS (97 %): ESI⁻ Calcd. 550.5 Found: 550.7.

### Example 32. Preparation of B34.

Synthesis of 3-[1-(2,4-Dichloro-benzyl)-5-fluoro-3-methyl-1H-indol-7-yl]-3-oxo-propionitrile, I-23. To a mixture of n-BuLi (2.5 M in hexane, 13.7 mL, 2.25 eq.) in 90 ml anhydrous THF, at -78°C was added acetonitrile (1.6 ml, 30.26 mmol, 2 eq.) over a 5 min period. The suspension was stirred at this temperature for 0.5 h, then a solution of I-12 (5.75 g, 15.13 mmol) in anhydrous THF (40 mL) was added over a 20 min period. The mixture was allowed to warm to 10°C and was quenched by slow addition of 10% aqueous HCl. The mixture was extracted with EtOAc (2 x 100 mL). The combined organic layers were washed with water (2 x 50 mL), brine (50 mL), dried over Na₂SO₄, filtered and concentrated *in vacuo* to afford 5.9 g of I-23 as an oil. This was used for next step without purification. ¹H-NMR (500 MHz, CDCl₃) confirmed the structure.

Synthesis of 5-[1-(2,4-Dichloro-benzyl)-5-fluoro-3-methyl-1H-indol-7-yl]-isoxazol-3-ylamine, I-24. To a solution of crude I-23 (1 g, 2.66 mmol) in a mixture of EtOH/ water (1:1, 54 mL) was added NaOH (124 mg, 3.06 mmol) and hydroxylamine sulfate (486 mg, 2.93 mmol). This mixture was heated at 80°C for 22 h. The reaction mixture was cooled to rt, concentrated to half its original volume and extracted with ethyl acetate (2 x 50 mL). The combined organic layers were washed with water (2 x 20 mL), brine (20 mL), dried (over MgSO₄), filtered and concentrated to give 900 mg of a brown oil. Purification of this residue by column chromatography using 20% to 30% EtOAc/hexanes afforded 290 mg of product, I-24 (29% yield). ¹H-NMR (500 MHz, CDCl₃) confirmed the structure.

Synthesis of 4,5-Dichloro-thiophene-2-sulfonic acid {5-[1-(2,4-dichlorobenzyl)-5-fluoro-3-methyl-1H-indol-7-yl]-isoxazol-3-yl}-amide, B34. To a suspension of I-24 (180 mg, 0.447 mmol) in pyridine (0.5 mL) was added DMAP (81 mg, 0.67 mmol, 1.5 eq.). This mixture was heated at 70°C until solution was achieved and 2,3-dichlorothiophene-5-sulphonyl chloride (140 mg, 0.536 mmol, 1.2 eq.) was added. The reaction mixture was stirred at this temperature for 3 h. The cooled reaction mixture was concentrated to an oil and diluted with EtOAc (15 mL). The organic layer was washed with 10% aqueous HCl (2 x 3 mL), water (2 x 3 mL), brine (2 x 3 mL), dried over MgSO₄ filtered and concentrated to give 280 mg of a crude residue. Purification of this residue by column chromatography using 20% to 50% EtOAc/hexanes gave 100 mg of product B34 (35% yield). ¹H-NMR (400 MHz, CDCl₃), 2.32 (s, 3H), 5.05 (s, 2H), 6.24 (d, J = 8 Hz, 1H), 6.34 (s, 1H), 6.9 (s, 1H), 6.97 (dd, J = 8.8, 2.8 Hz, 1H), 7.03 (dd, J = 8.8, 2 Hz, 1H), 7.3 (d, J = 2, 1H), 7.41 (dd, J = 8.8, 2.8 Hz,1H), 7.45 (s, 1H), 7.55 (br s,1H). LC/MS (ESI-) 604, 97%.

### Example 33. Preparation of B35.

Synthesis of N-{5-[1-(2,4-Dichloro-benzyl)-5-fluoro-3-methyl-1H-indol-7-yl]-isoxazo 1-3-yl}-3,4-difluoro-benzenesulfonamide, B35. To a suspension of I-24 (94 mg, 0.24 mmol) in pyridine (0.3 mL) was added DMAP (44 mg, 0.36 mmol, 1.5 eq.). This mixture was heated at 70 °C until solution was achieved and 3,4-difluorobenzenesulphonyl chloride (64.4 mg, 0.0.28 mmol, 1.2 eq.) was added. The reaction mixture was stirred at this temperature for 3 h. The cooled reaction mixture was concentrated to an oil and 10 % aqueous HCl (2 mL) was added. The mixture was extracted with EtOAc (3x10 mL). The combined organic layers were washed with water (2x5 mL), brine (5 mL), dried over MgSO₄, filtered and concentrated to give 100 mg of a residue. Purification of this residue by column chromatography using 20% to 50% EtOAc/hexanes gave 20 mg of product B35 (15% yield). ¹H-NMR (400 MHz, CDCl₃), 2.32 (s, 3H), 5.05 (s, 2H), 6.18 (d, J = 8.4 Hz, 1H), 6.29 (s, 1H), 6.89 (s, 1H), 6.93 (dd, J = 9.2,2.4 Hz, 1H), 7.01 (dd, J = 8.4, 2 Hz, 1H), 7.27- 7.31 (m, overlap, 1H), 7.28 (d, J = 2 Hz, 1H), 7.4 (dd, J = 8.8, 2.4 Hz, 1H), 7.47 (br s, 1H), 7.64- 7.66 (m,1H), 7.7- 7.74 (m, 1H). LC/MS (APCI-) 565, 91%,

### Example 34. Preparation of B36.

Synthesis of (N-{5-[1-(2,4-Dichloro-benzyl)-5-fluoro-3-methyl-1H-indol-7-yl]-isoxazol-3-yl}-2,4,5-trifluoro-benzenesulfonamide, B36. The title compound was obtained from I-24 (156 mg, 0.40 mmol) and 2,4,5-trifluorobenzenesulfonyl chloride (185 mg, 0.80 mmol) following general procedure A-2 to afford 64 mg (27%) as a yellow solid (hexane). R_{f} 0.15 (CH₂Cl₂-MeOH, 19:1). ¹H-NMR (400 MHz, CDCl₃) 2.31 (d, J = 1.2 Hz, 3H), 5.03 (s, 2H), 6.17 (d, J = 8.4 Hz, 1H), 6.26 (s, 1H), 6.89 (s, 1H), 6.92 (dd, J = 8.8, 2.8 Hz, 1H), 7.02 (dd, J = 8.4, 2.4 Hz, 1H), 7.11 (m, 1H), 7.28 (d, J = 2.4 Hz, 1H), 7.39 (dd, J = 8.4, 2.8 Hz, 1H), 7.75 (m, 1H), 8.00 (br s, 1H). LC-MS (96 %): ESI⁻ Calcd. 585 (M) Found: 584.1 (M-1).

### Example 35. Preparation of B37.

Synthesis of (3,4-Dichloro-N-{5-[1-(2,4-dichloro-benzyl)-5-fluoro-3-methyl-1H-indol-7-yl]-isoxazol-3-yl}-benzenesulfonamide, B37. The title compound was obtained from I-24 (156 mg, 0.40 mmol) and 3,4-dichlorobenzenesulfonyl chloride (196 mg, 0.80 mmol) following general procedure A-2 to afford 103 mg (43%) as a yellow solid (hexane). R_{f} 0.18 (CH₂Cl₂-MeOH, 19:1). ¹H-NMR (400 MHz, CDCl₃) 2.32 (d, J = 0.8 Hz, 3H), 5.02 (s, 2H), 6.19 (d, J = 8.4 Hz, 1H), 6.31 (s, 1H), 6.90 (s, 1H), 6.94 (dd, J = 9.2, 2.4 Hz, 1H), 7.02 (dd, J = 8.0, 2.0 Hz, 1H), 7.27 (d, J = 2.0 Hz, 1H), 7.40 (dd, J = 8.8, 1.6 Hz, 1H), 7.57 (d, J = 8.4 Hz, 1H), 7.67 (dd, J = 8.4, 2.0 Hz, 1H), 7.92 (br s, 1H), 7.97 (d, J = 2.0 Hz, 1H). LC-MS (98 %): ESI⁻ Calcd. 599 (M) Found: 598.3 (M-1).

### Example 36. Preparation of B38.

Synthesis of 7-Bromo-5-fluoro-3-methyl-(1-naphthalen-2-ylmethyl)-1H-indole, I-25. Compound I-25 was obtained from I-10 (4.8 g, 21.04 mmol), NaH (1.26 g, 31.57 mmol), 2-(bromomethyl) naphthalene (5.58 g, 25.25 mmol) and DMF (90 mL) in a manner similar to that for the conversion of I-10 to I-11 to afford 7.00 g (90 %) as a light brown solid (hexane). *R_{f}* 0.33 (hexanes/acetone, 9:1). ¹H-NMR (400 MHz, CDCl₃) confirmed the structure.

Synthesis of 5-Fluoro-3-methyl-1-naphthalen-2-yl methyl-1H-indole-7-carboxylic acid ethyl ester, I-26. Compound I-26 was obtained from I-25 (7.00 g, 19.01 mmol), 2.5 N BuLi (11.4 mL, 28.50 mL), ethyl chloroformate (3.63 mL, 38.02 mmol), anhydrous ether (120 mL) in a manner similar to the preparation of I-12 from I-11 to afford 7.09 g (quantitative) of I-26 as a brown oil. *R_{f}* 0.36 (hexanes/acetone, 9:1). ¹H-NMR (400 MHz, CDCl₃) confirmed the structure.

Synthesis of 3-(5-Fluoro-3-methyl-1-naphthalen-2-ylmethyl-1H-indol-7-yl)-3-oxo-propionitrile, I-27. Compound I-27 was obtained from I-26 (7.06 g, 19.53 mmol) in a manner similar to that described for the conversion of I-12 to I-23. The resulting crude oil (7.16 g, quant.) was triturated with hexane (15 mL) to provide a solid which was filtered and washed with hexane (2 x 5 mL) to afford I-27 (5.56 g, 80 %) as a light brown solid. Rf 0.06 (hexanes/acetone, 9:1). 1H-NMR (400 MHz, CDC13) confirmed the structure.

Synthesis of 5-(5-Fluoro-3-methyl-1-naphthalen-2-ylmethyl-1H-indol-7-yl)-isoxazol-3-ylamine, I-28. Compound I-28 was obtained from I-27 (4.43 g, 12.43 mmol) in a manner similar to that described for the conversion of I-23 to I-24 to afford I-28 (1.69 g, 37 %) as an orange solid. Rf 0.33 (CH₂Cl₂). ¹H-NMR (400 MHz, CDC13) 2.32 (d, J = 0.8 Hz, 3H), 5.32 (s, 2H), 5.45 (s, 1H), 6.89 (dd, J = 9.6, 2.4 Hz, 1H), 6.91 (dd, J = 8.8, 1.6 Hz, 1H), 7.01 (s, 1H), 7.21 (br s, 1H), 7.34 (dd, J = 8.8, 2.4 Hz, 1H), 7.39-7.44 (m, 2H), 7.65 (d, J = 8.0 Hz, 1H), 7.65-7.69 (m, 1H), 7.73-7.77 (m, 1H).

Synthesis of (3,4-Difluoro-N-[5-(5-fluoro-3-methyl-1-naphthalen-2-ylmethyl-1H-indol-7-yl)-isoxazol-3-yl]-benzenesulfonamide, B38. The title compound was obtained from I-28 (297 mg, 0.80 mmol) and 3,4-difluorobenzenesulfonyl chloride (340 mg, 1.60 mmol) following general procedure A-2 to afford B38 (106 mg, 24%) as an orange solid. Rf 0.14 (CH₂Cl₂). ¹H-NMR (400 MHz, CDC13) 2.31 (d, J = 0.8 Hz, 3H), 5.22 (s, 2H), 6.19 (s,1H), 6.84 (dd, J = 8.4, 1.6 Hz, 1H), 6.91 (dd, J = 9.2, 2.8 Hz, 1H), 6.99 (s, 1H), 7.07 (dq, J = 8.0, 1.6 Hz, 1H), 7.14 (s, 1H), 7.41 (dd, J = 5.6, 2.4 Hz, 1H), 7.42-7.45 (m, 2H), 7.51-7.55 (m, 1H), 7.62-7.70 (m, 3H), 7.74-7.76 (m, 1H), 7.92 (br s, 1H). LC-MS (98 %): ESI- Calcd. 547.56 Found: 546.4 (M-1).

### Example 37. Preparation of B39.

Synthesis of (2,4,5-Trifluoro-N-[5-(5-fluoro-3-methyl-1-naphthalen-2-ylmethyl-1H-indol-7-yl)-isoxazol-3-yl]-benzenesulfonamide, B39. The title compound was obtained from I-28 (149 mg, 0.40 mmol) and 2,4,5-trifluorobenzenesulfonyl chloride (185 mg, 0.80 mmol) following general procedure A-2 to afford B39 (42 mg, 19%) as an off-white solid. Rf 0.26 (CH₂Cl₂-MeOH, 19:1). ¹H-NMR (400 MHz, CDCl3) 2.31 (d, J = 0.8 Hz, 3H), 5.22 (s, 2H), 6.18 (s, 1H), 6.85 (dd, J = 8.8, 2.0 Hz, 1H), 6.89 (dd, J = 9.2, 2.4 Hz, 1H), 6.95 (dd, J = 9.2, 4.8 Hz, 1H), 6.99 (s, 1H), 7.14 (s, 1H), 7.38 (dd, J = 8.8, 2.4 Hz, 1H), 7.43-7.46 (m, 2H), 7.63-7.77 (m, 4H), 8.09 (br s, 1H). LC-MS (94 %): ESI- Calcd. 565.55
Found: 564.6 (M-1).

### Example 38. Preparation of B40.

Synthesis of (3,4-Dichloro-N-[5-(5-fluoro-3-methyl-1-naphthalen-2-ylmethyl-1H-indol-7-yl)-isoxazol-3-yl]-benzenesulfonamide, B40. The title compound was obtained from I-28 (149 mg, 0.40 mmol) and 3,4-dichlorobenzenesulfonyl chloride (196 mg, 0.80 mmol) following general procedure A-2 to afford B40 (76 mg, 33%) as an off-white solid. Rf 0.31 (CH₂Cl₂-MeOH, 19:1). ¹H-NMR (400 MHz, CDCl3) 2.31 (d, J = 0.8 Hz, 3H), 5.22 (s, 2H), 6.20 (s, 1H), 6.84 (dd, J = 8.4, 1.6 Hz, 1H), 6.91 (dd, J = 8.4, 1.6 Hz, 1H), 6.99 (s, 1H), 7.14 (s, 1H), 7.35 (d, J = 8.8 Hz, 1H), 7.40 (dd, J = 8.4, 2.4 Hz, 1H), 7.42-7.45 (m, 2H), 7.56 (dd, J = 8.4, 2.0 Hz, 1H), 7.63 (d, J = 7.6 Hz, 2H), 7.74-7.76 (m, 1H), 7.95 (d, J = 2.4 Hz, 1H), 8.00 (br d, J = 4.5 Hz, 1H). LC-MS (93 %): ESI- Calcd. 581 (M)
Found: 580.3 (M-1).

### Example 39. Preparation of B41.

Synthesis of (4,5-Dichloro-thiophene-2-sulfonic acid 5-(5-fluoro-3-methyl-1-naphthalen-2-ylmethyl-1H-indol-7-yl)-isoxazol-3-yl]-amide, B41. The title compound was obtained from I-28 (149 mg, 0.40 mmol) and 2,3-dichlorothiophene-5-sulfonyl chloride (201 mg, 0.80 mmol) following general procedure A-2 to afford B41 (132 mg, 33%) as an off-white solid. R_{f} 0.10 (CH₂Cl₂-MeOH, 19:1). ¹H-NMR (400 MHz, CDCl₃) 2.32 (d, J = 0.8 Hz, 3H), 5.26 (s, 2H), 6.23 (s, 1H), 6.86 (dd, J = 8.4, 2.0 Hz, 1H), 6.95 (dd, J = 9.2, 2.8 Hz, 1H), 7.00 (s, 1H), 7.19 (br s, 1H), 7.39-7.43 (m, 3H), 7.63-7.66 (m, 2H), 7.74-7.76 (m, 1H), 7.98 (s, 1H). LC-MS (99 %): ESI⁻ Calcd. 587 (M) Found: 586.2 (M-1).

### Example 40. Preparation of B42.

Synthesis of 1-(2,4-Dichloro-benzyl)-5-fluoro-3-methyl-1H-indole-7-carboxylic acid, I-29. A solution of compound I-11 (1.08 g, 2.84 mmol, 1 equiv.) in 2 N aqueous NaOH (7.1 mL, 14.20 mmol, 5 equiv.), methanol (3 mL) and THF (3 mL) was stirred and heated in a closed vial at 85 °C for 1.5 h. The reaction mixture was cooled to -70 °C and quenched through the addition of 10% aqueous HCl (20 mL). The mixture was extracted with EtOAc (50 mL), the organic layer washed with water (3 x 50 mL), brine (50 mL), dried over MgSO₄, filtered, and concentrated. The resulted solid was filtered and washed with hexane to afford I-29 (694 mg, 69%) as an off-white solid. *R_{f}* 0.22 (EtOAc/hexanes, 1:3). ¹H-NMR (400 MHz, CDCl₃) confirmed the structure.

Synthesis of 1-(2,4-Dichloro-benzyl)-5-fluoro-3-methyl-1H-indole-7-carboxylic acid iminomethyleneamide, I-30. Oxalyl chloride (0.99 mL, 1.98 mmol, 1.2 equiv.) was added to a solution of I-29 (580 mg, 1.65 mmol) in THF (7 mL) at rt under an Ar atmosphere. The reaction mixture was stirred at rt for 30 min and then it was concentrated to yield yellow crystals. A solution of 2N aqueous NaOH (1.65 mL, 3.29 mmol, 2 equiv.) was added to a solution of cyanamide (138 mg, 3.294 mmol, 2 equiv.) in THF (7 mL), stirred at rt for 20 min and then added over 2 min to a suspension obtained from I-29 and oxalyl chloride in THF (2 mL). The reaction mixture was stirred at rt for 30 min. The reaction mixture was concentrated, water (4 mL) was added, followed by 10% aqueous HCl (2 mL) and the aqueous phase was extracted with EtOAc (8 mL). The organic phase was washed with water (2 x 6 mL), dried over MgSO₄, filtered and concentrated *in vacuo* to yield (400 mg) of an orange oil. The oil was washed with hexane (4 mL, 2 mL) to afford a title compound I-30 (325 mg, 52 %) as a yellowish powder. *R_{f}* 0.30 (EtOAc). MS: ESI⁻ Calcd. 375 (M) Found: 374.3 (M-1). ¹H-NMR (400 MHz, CDCl₃) confirmed the structure.

Synthesis of (5-[1-(2,4-Dichloro-benzyl)-5-fluoro-3-methyl-1H-indol-7-yl]-[1,2,4]oxadiazol-3-ylamine, I-31. Pyridine (0.5 mL) was added to a mixture of I-30 (113 mg, 0.3 mmol, 1 equiv.) and hydroxylamine (21 mg, 1 equiv.) and the reaction mixture was stirred and heated at 45 °C for 16 h, and at 60 °C for 1 h. The reaction mixture was cooled to rt and poured into a mixture of 10% aqueous HCl (4 mL) and EtOAc (4 mL). The organic phase was washed with water (3 x 6 mL), brine (4 mL), dried over MgSO₄, filtered, and concentrated to yield crude product (136 mg) as an orange oil. Purification by chromatography on SiO₂ (Flash, 2 g) with CH₂Cl₂/hexanes, 1:1 (20 mL), CH₂Cl₂ (20 mL) yielded a crude product (45 mg) as an oil. The oil was triturated with hexane to afford a title compound I-31 (30 mg, 26 %) as a white powder. *R_{f}* 0.78 (EtOAc/hexanes, 1:1). ¹H-NMR (400 MHz, DMSO-d₆) 2.31 (d, J = 0.8 Hz, 3H), 5.70 (s, 2H), 5.89 (d, J = 8.4 Hz, 1H), 6.35 (s, 2H), 7.16 (dd, J = 8.8, 2.0 Hz, 1H), 7.32 (dd, J = 9.6, 2.4 Hz, 1H), 7.47 (s, 1H), 7.54 (d, J = 2.4 Hz, 1H), 7.71 (dd, J = 8.8, 2.4 Hz, 1H). LC-MS (99 %): (ESI+) Calcd. 390 (M) Found: 391.2 (M+1).

Synthesis of (5-Dichloro-thiophene-2-sulfonic acid {5-[1-(2,4-dichlorobenzyl)-5-fluoro-3-methyl-1H-indol-7-yl]-[1,2,4]oxadiazol-3-yl}-amide, B42. A solution of freshly prepared LDA (0.537 mmol, 2.1 equiv.) in THF (0.5 mL) was added dropwise over 2 min to a solution of I-31 (100 mg, 0.256 mmol, 1 equiv.) and HMPA (96 mg, 0.537 mmol, 2.1 equiv.) in THF (0.5 mL) at -78°C. The reaction mixture was stirred for 10 min at -78°C. A solution of 2,3-dichlorothiophene-5-sulfonyl chloride (161 mg, 0.639 mg, 2.5 equiv.) in THF (0.5 mL) was added dropwise over 2 min and the reaction mixture was slowly warmed over 1 h to -18°C, stirred for 1 h at -18 °C and slowly warmed over 1 h to rt. The reaction mixture was poured into a mixture of 10% aqueous HCl (4 mL) and EtOAc (4 mL). The organic phase was washed with water (3 x 4 mL), brine (4 mL), dried over MgSO₄, filtered, and concentrated to yield crude product (134 mg) as an orange oil. Purification of this oil by chromatography on SiO₂ (Flash, 5 g) with CH₂Cl₂/hexanes, 1:2 (30 mL), CH₂Cl₂/hexanes, 1:1 (10 mL), CH₂Cl₂ (10 mL), EtOAc (10 mL) yielded a crude product (40 mg) as a yellow oil. The oil was purified by chromatography on SiO₂ (Flash, 2 g) with EtOAc/hexanes, 1:4 (30 mL), and yielded a partially purified product (35 mg) as a yellow oil. The oil was recrystallized from CH₂Cl₂-hexanes, 2:1 to afford a title compound B42 (15 mg, 9 %) as a white solid. *R_{f}* 0.10 (EtOAc/hexanes, 1:1). ¹H-NMR (400 MHz, DMSO-d₆) 2.31 (s, 3H), 5.59 (s, 2H), 5.89 (d, J = 8.8 Hz, 1H), 7.13 (dd, J = 8.4, 2.4 Hz, 1H), 7.26 (d, J =1.6 Hz, 1H), 7.37 (dd, J = 9.6,2.4 Hz, 1H), 7.46 (s, 1H), 7.68 (s, 1H), 7.74 (dd, J = 8.8, 2.4 Hz, 1H). LC-MS (93 %): ESI⁻ Calcd. 604 (M) Found: 603.1 (M-1).

### Example 41. Preparation of B43.

Synthesis of 4-Bromo-1-methyl-1H-indole, I-32. To a solution of NaH (60% in mineral oil, 600 mg, 15 mmol) in DMF (20 mL), 4-bromo-1H-indole (1.96 g, 10 mmol) was added at -10°C. The stirring mixture was allowed to warm to rt for 10 min, recooled to -10 °C and then iodomethane (6.7 g, 50 mmol) was added at -10°C. The reaction mixture was stirred at rt for 3 h and diluted with CH₂Cl₂ (~200 mL). The reaction mixture was washed with water (3 x 200 mL), brine and dried over sodium sulfate. After filtration and removal of the solvent, 3 g of crude product I-32 was obtained. This compound was directly used in next step reaction without further purification.
¹H-NMR (500 MHz, CDCl₃) confirmed the structure.

Synthesis of 1-Methyl-4-(naphthalen-2-yloxy)-1H-indole, I-33. A mixture of I-32 (2.4 g, 11.42 mmol), CuI (217 mg, 1.142 mmol), N,N-dimethylglycine HCl salt (480 mg, 3.42 mmol), 2-naphthol (2.47 g, 17.14 mmol) and Cs₂CO₃ (7.42 g, 22.84 mmol) in dioxane (22 mL) was stirred under Ar at 105°C for 2 d. The reaction mixture was diluted with ethyl acetate and washed with water, brine and dried over sodium sulfate. After removal of solvent, the residue was purified by column chromatography on silica gel with 2% ethyl acetate/hexane as an eluent to give 2.16 1-Methyl-4-(naphthalen-2-yloxy)-1H-indole, I-33 (83% yield) ¹H-NMR (500 MHz, CDCl₃) confirmed the structure.

Synthesis of 2-Bromo-1-[1-methyl-4-(naphthalen-2-yloxy)-1H-indol-3-yl]-ethanone, I-34. To a solution of I-33 (500 mg, 1.83 mmol) in anhydrous methylene chloride (10 mL) at -70 °C was added diethylaluminum chloride (1 M solution in hexane, 2.74 mL, 2.74 mmol) at such rate to maintain the temperature below -65 °C. After the diethylaluminum chloride addition, the dry-ice-acetone bath was replaced with a water-salt-ice bath and the solution was warmed to -10 °C. At this temperature, bromoacetyl chloride (0.23 mL, 2.74 mmol) was added. The reaction mixture was stirred at this temperature for 1 h. TLC analysis showed the reaction was complete. Water (9 mL) was added slowly while stirring. The aqueous phase was extracted with methylene chloride (3 x 15 mL). The combined organic extracts were washed with water, brine, dried, concentrated to give 500 mg crude product. Trituration with ether afforded 450 mg of I-34 (62% yield). ¹H-NMR (500 MHz, CDCl₃) confirmed the structure.

Synthesis of 4-[-Methyl-4-(naphthalen-2-yloxy)-1H-indol-3-yl]-thiazol-2-ylamine, I-35. A suspension of I-34 (220 mg, 0.558 mmol) and thiourea (51 mg, 0.67 mmol) in ethanol (5 mL) was heated at reflux for 2 h. After completion, the reaction mixture was cooled to rt, diluted with water and basified with saturated aqueous NaHCO₃. The suspension was filtered off, washed with water and dried. Trituration with ether afforded 200 mg of I-35 as a white solid, 96% yield. ¹H-NMR (400 MHz, CDCl₃), 3.83 (s, 3H), 4.76 (br s, 2H), 6.7 (dd, J= 7.2, 1.2 Hz, 1H), 7.01 (s, 1H), 7.12- 7.25 (m, 2H), 7.28- 7.3 (m, 2H), 7.34- 7.43 (m, 2H), 7.6 (s, 1H), 7.64 (d, J = 8 Hz, 1H), 7.78- 7.81 (m, 2H). LC/MS (ESI+) 372: 98%.

### General Procedure for Sulfonamide Synthesis, (A-3).

To a solution of I-35 (0.1 mmol) in anhydrous THF (0.3 mL) was added NaH (2 eq., 60% dispersion in oil). The reaction mixture was stirred at rt for 15 min, then the corresponding sulfonyl chloride (2 eq.) was added. After completion, the mixture was acidified with 10% aqueous HCl and extracted with EtOAc (2 x 5 mL). The combined organic layers were washed with water, brine, dried and concentrated to give crude product. Purification by preparative TLC using 5% MeOH/ methylene chloride gave the target product.

Synthesis of 4,5-Dichloro-thiophene-2-sulfonic acid 14-[1-methyl-4-(naphthalen-2-yloxy)-1H-indol-3-yl]-thiazol-2-yl}-amide, B43. Compound B43 was synthesized following general procedure A-3. ¹H-NMR (400 MHz, CDCl₃), 3.89 (s, 3H), 6.32 (s, 1H), 6.81 (dd, J = 7.6, 1.2 Hz, 1H), 6.96 (m, 1H), 7.19-7.38 (m, 4H), 7.38 (s, 1H), 7.41- 7.45 (m, 2H), 7.61 (d, J = 7.6 Hz, 1H), 7.73 (d, J = 8.8 Hz, 1H), 7.79- 7.81 (m, 1H), 10.6 (br s, 1H). LC/MS (ESI-) 586: 98%.

### Example 42. Preparation of B44.

Synthesis of 3,4-Difluoro-N-{4-[1-methyl-4-(naphthalen-2-yloxy)-1H-indol-3-yl]-thiazol-2-yl}-benzenesulfonamide, B44. Compound B43 was synthesized following general procedure A-3. ¹H-NMR (400 MHz, CDCl₃), 3.87 (s, 3H), 6.28 (s, 1H), 6.79 (dd, J = 7.6, 1.2 Hz, 1H), 7.21- 7.27 (m, 4H), 7.3 (d, J = 2.4 Hz, 1H), 7.38 (s, 1H), 7.39- 7.45 (m, 2H), 7.52- 7.56 (m, 1H), 7.59- 7.61 (m, 1H), 7.65- 7.69 (m, 1H), 7.71 (s, 1H), 7.78- 7.8 (m, 1H), 10.57 (br s 1H). LC/MS (AP+) 547: 98%,

### Example 43. Preparation of B45.

Synthesis of 3-[1-Methyl-4-(naphthalen-2-yloxy)-1H-indol-3-yl]-3-oxo-propionitrile, I-36. A mixture of cyanoacetic acid (130 mg, 1.51 mmol), acetic anhydride (1.5 g, 1.5 mL, 15.1 mmol) and I-33 (412 mg, 1.51 mmol) was heated at 50 °C for 15 min. TLC analysis showed no starting material. The mixture was cooled to rt and solid precipitated out. The mixture was diluted with ether (5 mL) and filtered off. The solid was triturated with ether (10 mL). After filtration and air drying, 346 mg (67% yield) of I-36 was obtained as a slightly yellow compound. ¹H-NMR (500 MHz, CDCl₃) confirmed the structure.

Synthesis of 5-[1-Methyl-4-(naphthalen-2-yloxy)-1H-indol-3-yl]-isoxazole-3-ylamine, I-37. A suspension of I-36 (360 mg, 1.05 mmol), hydroxylamine sulfate (104 mg, 1.15 mmol) and sodium hydroxide (50.4 mg, 1.26 mmol) in a mixture of ethanol/ water (1: 1, 5 mL) was heated at 80 °C for 24 h. The reaction was not completed and more sodium hydroxide (50 mg) and hydroxylamine sulfate (100 mg) were added. The mixture was heated at 100 °C for 24 h. The reaction mixture was concentrated to half its initial volume and 36% HCl (0.25 mL) was added. The reaction mixture was heated at 100 °C for 3 h. The mixture was cooled to rt, concentrated to an oil and diluted with ethyl acetate (10 mL). The solution was washed with 10% aqueous NaOH. The basic aqueous phase was extracted with ethyl acetate (3x10 mL). The combined extracts were washed with water, brine, dried over magnesium sulfate, filtered and concentrated to a give a brown solid (400 mg). This crude material was purified by silica gel column chromatography using 30% ethyl acetate/ hexane to afford 120 mg of I-37 (32% yield). ¹H-NMR (500 MHz, CDCl₃) confirmed the structure.

Synthesis of 3,4-Difluoro-N-{5-[1-methyl-4-(naphthalen-2-yloxy)-1H-indol-3-yl]-isoxazol-3-yl}-benzenesulfonamide, B45. To a solution of I-37 (90 mg, 0.253 mmol) in anhydrous THF (0.8 mL) was added NaH (21 mg, 0.51 mmol, 60% dispersion in oil). The reaction mixture was stirred at rt for 15 min, and then 3,4-difluorobenzene sulfonyl chloride (83 mg, 0.38 mmol) was added. The reaction mixture was stirred at rt for 24 h. After completion, the mixture was acidified with 10% aqueous HCl and extracted with EtOAc. The combined extracts were washed with water, brine, dried and concentrated to give crude product. This crude product was purified by column chromatography using 10, 15,20% ethyl acetate/hexane and gave 39 mg (37% yield) of B45. ¹H-NMR (400 MHz, CDCl₃), 3.89 (s, 3H), 6.41- 6.47 (m, 1H), 6.79 (dd, J= 7.6, 0.8 Hz, 1H), 6.99 (s, 1H), 7.02 (m, 1H), 7.18 (d, J = 8, 0.8 Hz, 1H), 7.26 (t, J = 8.4, 1H), 7.38- 7.48 (m, 5H), 7.68 (s,1H), 7.7 (d, J = 8 Hz, 1H), 7.84 (d, J = 8, 1H), 7.90 (d, J = 8.8, 1H), 8.39 (br s, 1H). LC/MS (APCI+) 532: 100%,

### Example 44. Preparation of B46.

Synthesis of 5-Bromo-2-(2,5-dimethyl-pyrrol-1-yl)-pyridine, I-38. A mixture of 5-bromo-pyridin-2-ylamine (3.28 g, 19 mmol), acetonylacetone (2.17 g, 19 mmol) and p-toluenesulfonic acid monohydrate (0.95 g) in toluene (20 mL) was refluxed using a Dean-Stark trap overnight. The reaction mixture was concentrated *in vacuo,* diluted with EtOAc (50 mL) and washed with water (2 x 10 mL), 10% aqueous NaHCO₃, water, brine, dried over MgSO4, filtered and concentrated to give 4.2 g of a residue. Purification of this residue by column chromatography using silica gel and 2% to 4% EtOAc/hexanes gave 3 g product I-38. ¹H-NMR (500 MHz, CDCl3) confirmed the structure.

Synthesis of 2-(2,5-Dimethyl-pyrrol-1-yl)-5-(4,4,5,5-tetramethyl-[1,3,2]dioxaborolan-2-yl)-pyridine, I-39. To a solution of I-38 (220 mg, 0.876 mmol) in anhydrous THF (10 mL) at -78°C was added n-BuLi (2.5 M in hexane, 0.43 mL, 1.095 mmol). The reaction mixture was stirred at this temperature for 15 min, then 2-isopropoxy-4,4,5,5-tetramethyl-1,3,2-dioxaborolane (0.36 mL, 1.75 mmol) was added dropwise. The reaction mixture was stirred at -78 °C for 1 h, then the acetone-dry-ice bath was removed and the mixture was allowed to warm to 0 °C and quenched at this temperature with saturated aqueous NH₄Cl. The mixture was stirred at rt for 15 min, then extracted with EtOAc (2 x 10 mL). The combined organic extracts were washed with water, brine, dried over Na₂SO₄, filtered and concentrated to give 300 mg of I-39. This material was deemed of sufficient purity to be used in the next step. ¹H-NMR (500 MHz, CDCl₃) confirmed the structure.

Synthesis of 1-(2,4-Dichloro-benzyl)-7-[6-(2,5-dimethyl-pyrrol-1-yl)-pyridin-3-yl]-5-fluoro-3-methyl-1H-indole, I-40. To a solution of I-39 (300 mg, 1 mmol) in DME (4 mL) was added I-11 (258 mg, 0.66 mmol), then cesium carbonate (326 mg, 1 mmol). After the suspension was degassed by bubbling argon through the mixture for 5 min, the catalyst Pd (Ph₃P)₄ (46 mg, 0.04 mmol) was added and the reaction mixture was stirred at 100 °C for 3.5 h. The reaction was cooled to rt and diluted with water. The mixture was extracted with EtOAc (2 x 15 mL). The combined organic extracts were washed with water, brine, dried over Na₂SO₄, filtered and concentrated to give 400 mg of a residue. Purification by silica gel column chromatography provided 100 mg of I-40.
¹H-NMR (500 MHz, CDCl₃) confirmed the structure

Synthesis of 5-[1-(2,4-Dichloro-benzyl)-5-fluoro-3-methyl-1H-indol-7-yl]-pyridin-2-ylamine,I-41. A mixture of I-40 (95 mg, 0.198 mmol), triethylamine (110 µL, 0.792 mmol), hydroxyl amine hydrochloride (158 mg, 2.28 mmol) in a mixture of solvents: EtOH (1.2 mL), water (0.4 mL), chloroform (0.2 mL) was heated at 90 °C for 24 h in a closed vial. TLC analysis showed the reaction was not complete. Additional hydroxylamine hydrochloride (130 mg) was added and mixture was heated at 100 °C for 1 d. The reaction mixture was cooled to rt, concentrated, then 10% aqueous HCl was added until a pH = 2 was reached and the mixture was extracted with ether. The aqueous layer was basified to pH = 9 using 6N aqueous NaOH, and extracted with ethyl acetate (3x10 mL). The combined extracts were washed with water, brine, dried over Na₂SO₄, filtered and concentrated to give 120 mg of a residue. Purification of this residue by silica gel column chromatography using 10% to 50% ethyl acetate/hexane provided 50 mg of I-40 (starting material) and 30 mg of I-41.

Synthesis of 4,5-Dichloro-thiophene-2-sulfonic acid {5-[1-(2,4-dichlorobenzyl)-5-fluoro-3-methyl-1H-indol-7-yl]-pyridin-2-yl}-amide, B46. To a mixture of I-41 (12 mg, 0.03 mmol) in pyridine (0.15 mL), 2,3-dichlorothiophene-5-sulfonyl chloride (12 mg, 0.045 mmol), was added at rt. The reaction mixture was stirred at rt for 5 h. TLC analysis shows no product formed. At this point DMAP (4 mg) was added and mixture was stirred at rt for 24 h. The pyridine was removed *in vacuo,* 10% aqueous HCl (1 mL) was added and the mixture was extracted with ethyl acetate (2 x 4 mL). The combined extracts were washed with brine, dried over Na₂SO₄, filtered and concentrated to give 20 mg of a residue. Trituration of this residue with methanol (0.15 mL) gave, after filtration, 8 mg of B46. ¹H-NMR (400 MHz, CDCl₃), 2.34 (s, 3H), 4.94- 5.03 (m, 2H), 5.97 (d, J = 8.4, 1H), 6.67 (dd, J = 8.8, 2.4, 1H), 6.93 (s, 1H), 7.04 (dd, J= 8, 2 Hz, 1H), 7.1 (d, J = 2, 1H), 7.24- 7.33 (m, 4H), 7.45 (s,1H), 8.08 (br s, 1H). LC/MS (ESI-) 614: >80%

### Example 45. Preparation of B47.

Synthesis of 2-Methyl-2-allylcyclohexanone, I-42. To a solution of sodium hydride (1 eq.; 60% dispersion in mineral oil) in dimethoxyethylene glycol at 5 °C under a nitrogen atmosphere, was added 2-methylcyclohexanone (1 eq.) dropwise. The solution was allowed to warm to room temperature, after which it was heated to 80 °C for 1.5 h. The solution was then cooled to room temperature, and then to 5 °C. Allyl bromide (1 eq.) was added dropwise, after which, the reaction mixture was heated to 80 °C for 1.5 h. The reaction was cooled to room temperature and water (~14 eq.) was added dropwise. The aqueous layer was extracted twice with ethyl ether, and dried over sodium sulfate. After concentration, the crude product was purified via silica gel chromatography using 2.5 % ethyl ether in hexanes to obtain compound I-42 in 35% yield. ¹H NMR

Synthesis of (1-Methyl-2-oxo-cyclohexyl)-acetic acid, I-43. To biphasic solution of 1-methyl-1-allylcyclohexanone, I-42, in H₂O/CH₃CN/CCl₄ under nitrogen atmosphere was added NaIO₄ (20 eq), followed by RuCl_{3·}H₂O. The reaction was stirred at room temperature overnight. 2-Propanol (~88 eq) was added dropwise, causing the reaction mixture to blacken. The mixture was diluted with water and ethyl either, filtered through a Celite pad, and the pad was washed with ethyl ether. The aqueous layer was extracted with dichloromethane and ethyl acetate. The combined organics extracts were dried over sodium sulfate, and concentrated *in vacuo* to give compound I-43 in quantitative yield. ¹H NMR confirmed the structure.

General procedure (A-4) for preparation of hexahydro-indol-2-ones, I-44. A solution of (1-Methyl-2-oxo-cyclohexyl)-acetic acid, I-43 (1 eq), and the appropriate benzyl amine (1 eq) in m-xylene was heated under reflux at 145 °C for 3 h. The reaction was concentrated *in vacuo,* and the residue either taken through crude, or purified via silica gel chromatography, using hexanes in dichloromethane (10-20 %) as eluent to obtain the desired product, I-44. Product structure was verified by ¹H NMR.

General procedure (A-5) for bromination of hexahydro-indol-2-ones, I-45:
To a solution of the appropriate hexahydro-indol-2-one, I-44 in dichloromethane at 0 °C was added bromine (1 eq) dropwise. The reaction mixture was stirred until bromine color disappeared, and then for an additional 5 minutes.
Triethylamine (3 eq) was added in one portion and the reaction mixture was stirred at room temperature for 10 min. The reaction was washed with water (3x), and dried over magnesium sulfate. The dichloromethane solution was filtered and concentrated *in vacuo.* The residue was either taken through to the next step crude, or purified via silica gel chromatography, using dichloromethane as the eluent, to obtain the appropriate vinylic bromide, I-45. Product structure was verified by ¹H NMR.

Synthesis of 1-(3-Methoxy-benzyl)-3a-methyl-1,3,3a,4,5,6-hexahydro-indol-2-one, I-44: Following the general procedure A-4, (1-Methyl-2-oxo-cyclohexyl)-acetic acid (I-43) was converted to I-44. Consistent with ¹H-NMR.

Synthesis of 7-Bromo-1-(3-Methoxy-benzyl)-3a-methyl-1,3,3a,4,5,6-hexahydro-indol-2-one, I-45: Following the general procedure A-5,1-(3-Methoxy-benzyl)-3a-methyl-1,3,3a,4,5,6-hexahydro-indol-2-one, I-44 was converted to I-45. Consistent with ¹H-NMR.

Synthesis of 7-(1-Ethoxy-vinyl)-1-(3-methoxy-benzyl)-3a-methyl-1,3,3a,4,5,6-hexahydro-indol-2-one. I-46. To a solution of bromide I-45 (350 mg, 1 mmol) in dry dioxane (5 mL) were added tributyl(1-ethoxyvinyl)tin (390 mg, 1.05 mmol) and dichlorobis(triphenylphosphine) palladium (36 mg, 0.05 mmol). The reaction mixture was heated in a closed vial at 100 °C for 24 h. The reaction mixture was cooled to rt, concentrated *in vacuo,* diluted with methylene chloride (10 mL) and filtered through a short plug of celite. The plug was washed a few times with methylene chloride. The solvent was removed and the crude residue was purified by silica gel column chromatography using hexane and 2% ethyl acetate/hexane to provide 224 mg of I-46 (65.7% yield). ¹H-NMR (500 MHz, CDCl₃) confirmed the structure.

Synthesis of 7-Acetyl-1-(3-methoxy-benzyl)-3a-methyl-1,3,3a,4,5,6-hexahydro-indol-2-one, I-47. To a solution of I-46 (220 mg, 0.645 mmol) in THF (5 mL) was added of 2N aqueous HCl (2 mL) at rt. The reaction mixture was stirred at rt for 2 h. The reaction mixture was partitioned between water and ether (20 mL, 1:1). The mixture was transferred to a separatory funnel and organic layer was separated. The aqueous layer was extracted with ether (3 x 15 mL). The combined extracts were washed with water, brine, dried over MgSO₄, filtered and concentrated to afford 203 mg of I-47. ¹H-NMR (500 MHz, CDCl₃) confirmed the structure.

Synthesis of 7-(2-Bromo-acetyl)-1-(3-methoxy-benzyl)-3a-methyl-1,3,3a,4,5,6-hexahydro-indol-2-one, I-48. To a solution of 7-Acetyl-1-(3-methoxy-benzyl)-3a-methyl-1,3,3a,4,5,6-hexahydro-indol-2-one, I-47 (160 mg, 0.511 mmol) in a mixture of dioxane/chloroform (1:1, 2 mL) was added bromine (81.8 mg, 26 µL) at a rate of one drop every 3 s. The reaction mixture was stirred at rt for 2 h. The mixture was concentrated, diluted with ethyl acetate (10 mL), washed with water, brine, dried over MgSO₄, filtered and concentrated to afford 208 mg of I-48. This product was deemed of sufficient purity to be carried on to the next step. 1H-NMR (500 MHz, CDCl₃) confirmed the structure..

Synthesis of 7-(2-Amino-thiazol-4-yl)-1-(3-methoxy-benzyl)-3a-methyl-1,3,3a,4,5,6-hexahydro-indol-2-one, I-49. A mixture of 7-(2-bromo-acetyl)-1-(3-methoxy-benzyl)-3a-methyl-1,3,3a,4,5,6-hexahydro-indol-2-one, I-48 (200 mg, 0.51 mmol), thiourea (34 mg, 0.51 mmol) in ethanol (2 mL) was heated at 80 °C for 3 h. The mixture was concentrated, diluted with ethyl acetate (15 mL) and washed with 10% sodium acetate solution (3 mL). The organic layer was separated, washed with water, brine, dried over MgSO₄, filtered and concentrated to afford 150 mg of crude product. Trituration with ether afforded 75 mg of I-49. ¹H-NMR (500 MHz, CDCl₃)

Synthesis of 3,4-Difluoro-N-{4-[1-(3-methoxy-benzyl)-3a-methyl-2-oxo-2,3,3a,4,5,6-hexahydro-1H-indol-7-yl]-thiazol-2-yl}-benzenesulfonamide, B47. To a solution of 7-(2-Amino-thiazol-4-yl)-1-(3-methoxy-benzyl)-3a-methyl-1,3,3a,4,5,6-hexahydro-indol-2-one, I-49 (45 mg, 0.122 mmol) in pyridine (0.2 mL) was added DMAP (30 mg, 0.24 mmol). This mixture was heated at 70 °C and 3,4-difluorobenzene sulfonylchloride (52 mg, 0.24 mmol) was added. The solution became a suspension and the reaction was complete in 10 min. The mixture was cooled to rt and concentrated to dryness. The residue was diluted with ethyl acetate (4 mL) and washed with 10% aqueous HCl. The aqueous layer was extracted one more time with ethyl acetate. The combined extracts were washed with water, brine, dried over MgSO₄, filtered and concentrated to afford 70 mg of crude product. Purification by preparative silica gel TLC using ethyl acetate/hexane (1:1) as eluent gave 35 mg of B47 (53% yield). ¹H-NMR (400 MHz, CDCl₃), 1.21 (s, 3H), 1.61-1.67 (m, 2H), 1.77-1.83 (m, 2H), 2.18- 2.27 (m, 2H), 2.24 (dd, J =16, 4.8 Hz, 2H), 3.73 (s, 1H), 3.99 (d, J =16 Hz, 1H), 5.03 (d, J =16 Hz, 1H), 5.95 (s, 1H), 6.36 (d, J = 7.6 Hz, 1H), 6.38 (s, 1H), 6.68 (dd, J = 8.4, 2 Hz, 1H), 7 (t, J = 8 Hz, 1H), 7.23- 7.29 (m, 2H), 7.66- 7.76 (m, 2H). LC/MS (ESI+) 546: 93%.

### Example 46. Preparation of B09.

Synthesis of 4-(5-Fluoro-3-methyl-1H-indol-7-yl)-phenylamine, I-50. A mixture of I-10 (220 mg, 0.96 mmol), 4-(4,4,5,5-tetramethyl)-1,3,2-dioxaborane-2-yl) aniline (316 mg, 1.44 mmol), tetrakistriphenylphosphine palladium (56 mg, 0.048 mmol) and cesium carbonate (470 mg, 1.44 mmol) in DMF (4 mL) was heated at 110 °C for 2 h in a closed vial. Reaction mixture was cooled to rt, partitioned between water and EtOAc. The aqueous layer was extracted with EtOAc (2 x 20 mL). The combined organic layers were washed with water, brine, dried (MgSO₄) and concentrated to give 250 mg of crude product. This crude product was chromatographed on SiO₂ with 20% EtOAc/hexanes solvent mixture to afford 1-50 (120 mg, 52 % yield) as white foam.
¹H-NMR (400 MHz, CDCl₃) confirmed the structure.

Synthesis of N-[4-(5-Fluoro-3-methyl-1H-indol-7-yl)-phenyl]-methanesulfonamide, 1-51. To a solution of 4-(5-Fluoro-3-methyl-1H-indol-7-yl)-phenylamine, I-50 (120 mg, 0.5 mmol) in pyridine (0.3 mL) cooled to 0 °C, was added methanesulfonylchloride (114.55 mg, 2 eq.). The reaction mixture was stirred at rt for 3 h. The mixture was concentrated, 10% aqueous HCl was added and this aqueous mixture was extracted with EtOAc (2x10 mL). The combined organic layers were washed with water, brine, dried (MgSO₄), filtered and concentrated to give a residue. This residue was purified by column chromatography (SiO₂) using 20% EtOAc/hexanes solvent mixture and afforded 95.5 mg of I-51 (60% yield). ¹H-NMR (400 MHz, CDCl₃) confirmed the structure.

Synthesis of N-{4-[1-(2,4-Dichloro-benzyl)-5-fluoro-3-methyl-1H-indol-7-yl]-phenyl}-methanesulfonamide, B09. To a suspension of NaH (60% in mineral oil, 24 mg, 0.59 mmol, 2 equiv.) in DMF (2 mL) was added N-[4-(5-Fluoro-3-methyl-1H-indol-7-yl)-phenyl]-methanesulfonamide, I-51 (95 mg, 0.298 mmol, 1 equiv.) at -10 °C. The reaction mixture was allowed to warm to rt and stirred for 30 min at rt. The reaction mixture was cooled to 0 °C and 2,4-dichlorobenzyl chloride (71 mg, 0.36 mmol, 1.2 equiv.) was added gradually. The reaction mixture was allowed to warm to rt and stirred for 4 h. The reaction mixture was quenched with 10 % aqueous HCl (10 mL) and extracted with ether (3 x 20 mL). The combined organic extracts were washed with water, brine, dried over MgSO₄, filtered, and concentrated to afford a residue. This residue was purified by column chromatography utilizing 7% EtOAc/hexanes as eluent to provide 38 mg of B09 (30% yield). 1H-NMR (400 MHz, CDCl₃): 2.34 (s, 3H), 3.07 (s, 3H), 4.83 (s, 2H), 5.99 (d, J = 8 Hz, 1H), 6.37 (br s, 1H), 6.7 (dd, J = 9.6, 2.4 Hz, 1H), 6.86 (s, 1H), 6.99 (dd, J = 8.4, 2 Hz, 1H), 7.03 (s, 4H), 7.2 (d, J = 2 Hz, 1H), 7.26 (dd, J = 8.8, 2.4 Hz, 1H). LCMS (ESI-): 447, 99%.

The compounds of the invention were assayed for their binding on prostanoid EP3 receptors according to the method of Abramovitz et al. [Bioch. Biophys. Acta, 1473, 285-293 (2000)]. Chart 1 shows the activity in column 2. Compounds with IC₅₀ < 1µM are shown as +++; compounds with IC₅₀ 1-10 µM are shown as ++; and compounds with IC₅₀ > 10 µM are shown as +.

| **Comoun No B(X)** | **Activity** |
|---|---|
| B01 | +++ |
| B02 | ++ |
| B03 | ++ |
| B04 | ++ |
| B05 | ++ |
| B06 | ++ |
| B07 | + |
| B08 | ++ |
| B09 | + |
| B10 | ++ |
| B11 | ++ |
| B12 | +++ |
| B13 | +++ |
| B14 | ++ |
| B15 | ++ |
| B16 | + |
| B17 | ++ |
| B18 | +++ |
| B19 | +++ |
| B20 | +++ |
| B21 | + |
| B22 | + |
| B23 | ++ |
| B24 | ++ |
| B25 | + |
| B26 | +++ |
| B27 | ++ |
| B28 | ++ |
| B29 | +++ |
| B30 | +++ |
| B31 | +++ |
| B32 | +++ |
| B33 | +++ |
| B34 | +++ |
| B35 | +++ |
| B36 | +++ |
| B37 | +++ |
| B38 | +++ |
| B39 | +++ |
| B40 | +++ |
| B41 | +++ |
| B42 | +++ |
| B43 | ++ |
| B44 | ++ |
| B45 | +++ |
| B46 | +++ |
| B47 | +++ |

## Claims

1. A compound of formula 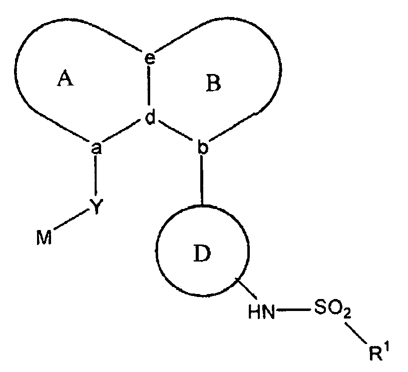 wherein
A and B represent a pair of fused 5-, 6- or 7-membered rings, said fused A/B ring system containing from zero to four heteroatoms chosen from nitrogen, oxygen and sulfur and said rings additionally substituted with from zero to four substituents chosen independently from halogen, -OH, (C₁-C₆)alkyl, (C₃-C₆)cycloalkyl, -O-(C₁-C₆)alkyl, -O-(C₃-C₆)cycloalkyl, fluoro(C₁-C₆)alkyl, fluoro(C₃-C₆)cycloalkyl, -O-(C₁-C₆)fluoroalkyl, -O-(C₃-C₆)cyclofluoroalkyl, methylenedioxy, ethylenedioxy, alkoxy-(C₁-C₆)alkyl, alkoxy-(C₃-C₆)cycloalkyl, hydroxy(C₁-C₆)alkyl, hydroxy(C₃-C₆)cycloalkyl, oxo, oxide, - CN, nitro, -S-(C₁-C₆)alkyl, -S-(C₃-C₆)cycloalkyl, amino, (C₁-C₆)alkylamino, (C₃-C₆)cycloalkylamino, di(C₁-C₆)alkylamino, di(C₃-C₆)cycloalkylamino, di(C₁-C₆)alkylaminoalkyl, di(C₁-C₆)alkylaminocycloalkyl, di(C₃-C₆)cycloalkylaminoalkyl, di(C₃-C₆)cycloalkylaminocycloalkyl, carboxy, carboalkoxy, acyl, carboxamido, (C₁-C₆)alkylsulfoxide, (C₃-C₆)cycloalkylsulfoxide, acylamino, phenyl, benzyl, *spiro*thiazolidinyl, phenoxy and benzyloxy;
a and b represent points of attachment of residues Y and D respectively and a and b are in a peri relationship to one another on said fused A/B ring system;
d and e represent points of fusion between ring A and ring B in said fused A/B ring system;
D is an aryl or heteroaryl ring system, said ring system additionally substituted with from zero to four substituents chosen independently from halogen, -OH, (C₁-C₆)alkyl, (C₃-C₆)cycloalkyl, -O-(C₁-C₆)alkyl, -O-(C₃-C₆)cycloalkyl, fluoro(C₁-C₆)alkyl, fluoro(C₃-C₆)cycloalkyl, -O-(C₁-C₆)fluoroalkyl, -O-(C₃-C₆)cyclofluoroalkyl, methylenedioxy, ethylenedioxy, alkoxy-(C₁-C₆)alkyl, alkoxy-(C₃-C₆)cycloalkyl, hydroxy(C₁-C₆)alkyl, hydroxy(C₃-C₆)cycloalkyl, -CN, nitro, -S-(C₁-C₆)alkyl, -S-(C₃-C₆)cycloalkyl, amino, (C₁-C₆)alkylamino, (C₃-C₆)cycloalkylamino, di(C₁-C₆)alkylamino, di(C₃-C₆)cycloalkylamino, di(C₁-C₆)alkylaminoalkyl, di(C₁-C₆)alkylaminocycloalkyl, di(C₃-C₆₎cycloalkylaminoalkyl, di(C₃-C₆)cycloalkylaminocycloalkyl, carboxy, carboalkoxy, acyl, carboxamido, (C₁-C₆)alkylsulfoxide, (C₃-C₆)cycloalkylsulfoxide, acylamino, phenyl, benzyl, phenoxy and benzyloxy;
Y is chosen from -CH₂-, -O-, -OCH₂-, -S-, -SO-, and -SO₂-, wherein the left-hand bond indicates the point of attachment to ring A or B;
M is chosen from aryl, substituted aryl, heterocyclyl and substituted heteroaryl;
R¹ is chosen from aryl, substituted aryl, heteroaryl, substituted heteroaryl and CF₃; and when Y is -CH₂-, -O-, -S-, -SO-, or -SO₂-, R¹ may additionally be (C₁-C₆)alkyl or (C₃-C₆)cycloalkyl;
wherein, in each substituted moiety M or R¹, up to three H atoms in each residue are replaced with halogen, lower alkyl, haloalkyl, hydroxy, loweralkoxy, carboxy, alkoxycarbonyl, alkylaminocarbonyl, cyano, carbonyl, nitro, amino, alkylamino, dialkylamino, mercapto, alkylthio, sulfoxide, sulfone, acylamino, amidino, phenyl, benzyl, heteroaryl, phenoxy, benzyloxy, heteroaryloxy, methylenedioxy or ethylenedioxy.

2. A compound according to claim 1 wherein D is phenyl substituted with from zero to four substituents.

3. A compound according to claim 1 or 2 wherein D is naphthyl substituted with from zero to four substituents.

4. A compound according to any of claims 1 to 3 wherein D is monocyclic heteroaryl substituted with from zero to four substituents.

5. A compound according to claim 1 wherein D is bicyclic heteroaryl substituted with from zero to four substituents.

6. A compound according to claim 1 wherein R¹ is chosen from phenyl, substituted phenyl, 5-membered ring heteroaryl, substituted 5-membered ring heteroaryl and CF₃.

7. A compound according to claim 1 wherein M is chosen from aryl, substituted aryl, heterocyclyl and substituted heteroaryl.

8. A compound according to claim 7 wherein M is chosen from phenyl, substituted phenyl, naphthyl, substituted naphthyl, heteroaryl and substituted heteroaryl.

9. A compound according to claim 1 wherein the A/B ring system is a pair of fused 5-membered rings:

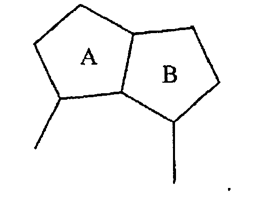

10. A compound according to claim 1 wherein the A/B ring system is a pair of fused 6-membered rings:

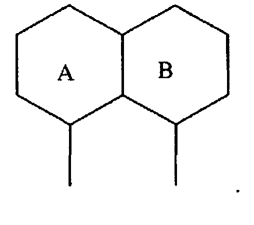

11. A compound according to claim 1 wherein the A/B ring system is a fused 5-and 6-membered ring pair:

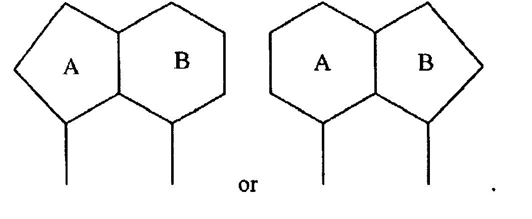

12. A compound according to claim 11 wherein the A/B ring system is an indole.

13. A compound according to claim 1 for use in the treatment or prophylaxis of a prostaglandin-mediated disease or condition in a mammal.

14. A compound for use according to claim 13 wherein said disease or condition is chosen from
pain, fever or inflammation associated with rheumatic fever, influenza or other viral infections, common cold, dysmenorrhea, headache, migraine, sprains and strains, myositis, neuralgia, synovitis, arthritis, including rheumatoid arthritis, degenerative joint diseases (osteoarthritis), gout and ankylosing spondylitis, bursitis, burns including radiation and corrosive chemical injuries, sunburns, immune and autoimmune diseases; cellular neoplastic transformations or metastic tumor growth;
diabetic retinopathy, tumor angiogenesis;
prostanoid-induced smooth muscle contraction associated with dysmenorrhea, premature labor, asthma or eosinophil related disorders;
Alzheimer's disease;
glaucoma;
bone loss;
osteoporosis;
Paget's disease;
peptic ulcers, gastritis, regional enteritis, ulcerative colitis, diverticulitis or other gastrointestinal lesions; GI bleeding;
coagulation disorders selected from hypoprothrombinemia, hemophilia and other bleeding problems;
kidney disease;
thrombosis, myocardial infarction, stroke; and
occlusive vascular disease.

15. A compound for use according to claim 14 wherein said disease is occlusive vascular disease.

16. A compound according to claim 1 for use in reducing plaque in the treatment of atherosclerosis in a mammal.

17. A compound according to claim 1 for use in the promotion of bone formation or for use in cytoprotection in a mammal.

18. A compound according to claim 1 for use in the treatment or prophylaxis of pain, inflammation, atherosclerosis, myocardial infarction, stroke or vascular occlusive disorder in a mammal.

19. A pharmaceutical composition comprising a pharmaceutically acceptable carrier and a compound according to claim 1.

20. A pharmaceutical formulation according to claim 19 comprising an additional therapeutic agent chosen from a platelet aggregation inhibitor, an HMG-CoA reductase inhibitor, an antihyperlipidemic agent and a cyclooxygenase inhibitor.

21. A pharmaceutical formulation according to claim 20 wherein said platelet aggregation inhibitor is chosen from tirofiban, dipyridamole, clopidogrel and ticlopidine.

22. A pharmaceutical formulation according to claim 20 wherein said HMG-CoA reductase inhibitor is chosen from lovastatin, simvastatin, pravastatin, rosuvastatin, mevastatin, atorvastatin, cerivastatin, pitavastatin and fluvastatin.

23. A pharmaceutical formulation according to claim 20 wherein said cyclooxygenase inhibitor is chosen from rofecoxib, meloxicam, celecoxib, etoricoxib, lumiracoxib, valdecoxib, parecoxib, cimicoxib, diclofenac, sulindac, etodolac, ketoralac, ketoprofen, piroxicam and LAS-34475.

24. A method for screening for selective prostanoid receptor ligands comprising bringing a labeled compound according to claim 1 into contact with a prostanoid receptor and measuring its displacement by a test compound.

25. A method according to claim 24 for screening for selective EP₃ ligands comprising bringing a labeled compound into contact with a cloned human EP₃ receptor and measuring its displacement by a test compound.

26. A compound of formula 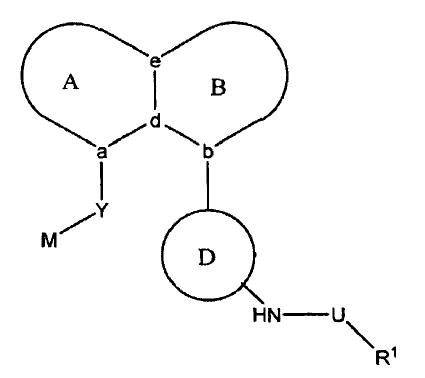 wherein
A and B represent a pair of fused 5-, 6- or 7-membered rings, said fused A/B ring system containing from zero to four heteroatoms chosen from nitrogen, oxygen and sulfur and said rings additionally substituted with from zero to four substituents chosen independently from halogen, -OH, (C₁-C₆)alkyl, (C₃-C₆)cycloalkyl, -O-(C₁-C₆)alkyl, -O-(C₃-C₆)cycloalkyl, fluoro(C₁-C₆)alkyl, fluoro(C₃-C₆)cycloalkyl, -O-(C₁-C₆)fluoroalkyl, -O-(C₃-C₆)cyclofluoroalkyl, methylenedioxy, ethylenedioxy, alkoxy-(C₁-C₆)alkyl, alkoxy-(C₃-C₆)cycloalkyl, hydroxy(C₁-C₆)alkyl, hydroxy(C₃-C₆)cycloalkyl, oxo, oxide, - CN, nitro, -S-(C₁-C₆)alkyl, -S-(C₃-C₆)cycloalkyl, amino, (C₁-C₆)alkylamino, (C₃-C₆)cycloalkylamino, di(C₁-C₆)alkylamino, di(C₃-C₆)cycloalkylamino, di(C₁-C₆)alkylaminoalkyl, di(C₁-C₆)alkylaminocycloalkyl, di(C₃-C₆)cycloalkylaminoalkyl, di(C₃-C₆)cycloalkylaminocycloalkyl, carboxy, carboalkoxy, acyl, carboxamido, (C₁-C₆)alkylsulfoxide, (C₃-C₆)cycloalkylsulfoxide, acylamino, phenyl, benzyl, *spiro*thiazolidinyl, phenoxy and benzyloxy;
a and b represent points of attachment of residues Y and D respectively and a and b are in a peri relationship to one another on said fused A/B ring system;
d and e represent points of fusion between ring A and ring B in said fused A/B ring system;
U is C=O;
D is an aryl or heteroaryl ring system, said ring system additionally substituted with from zero to four substituents chosen independently from halogen, -OH, (C₁-C₆)alkyl, (C₃-C₆)cycloalkyl, -O-(C₁-C₆)alkyl, -O-(C₃-C₆)cycloalkyl, fluoro(C₁-C₆)alkyl, fluoro(C₃-C₆)cycloalkyl, -O-(C₁-C₆)fluoroalkyl, -O-(C₃-C₆)cyclofluoroalkyl, methylenedioxy, ethylenedioxy, alkoxy-(C₁-C₆)alkyl, alkoxy-(C₃-C₆)cycloalkyl, hydroxy(C₁-C₆)alkyl, hydroxy(C₃-C₆)cycloalkyl, -CN, nitro, -S-(C₁-C₆)alkyl, -S-(C₃-C₆)cycloalkyl, amino, (C₁-C₆)alkylamino, (C₃-C₆)cycloalkylamino, di(C₁-C₆)alkylamino, di(C₃-C₆)cycloalkylamino, di(C₁-C₆)alkylaminoalkyl, di(C₁-C₆)alkylaminocyaoalkyl, di(C₃-C₆)cycloalkylaminoalkyl, di(C₃-C₆)cycloalkylaminocycloalkyl, carboxy, carboalkoxy, acyl, carboxamido, (C₁-C₆)alkylsulfoxide, (C₃-C₆)cycloalkylsulfoxide, acylamino, phenyl, benzyl, phenoxy and benzyloxy;
Y is -CH₂-;
M is chosen from aryl, substituted aryl, heterocyclyl and substituted heteroaryl;
R¹ is chosen from aryl, substituted aryl, heteroaryl, substituted heteroaryl, CF₃, (C₁-C₆)alkyl, and (C₃-C₆)cycloalkyl;
wherein, in each substituted moiety M or R¹, up to three H atoms in each residue are replaced with halogen, lower alkyl, haloalkyl, hydroxy, loweralkoxy, carboxy, alkoxycarbonyl, alkylaminocarbonyl, cyano, carbonyl, nitro, amino, alkylamino, dialkylamino, mercapto, alkylthio, sulfoxide, sulfone, acylamino, amidino, phenyl, benzyl, heteroaryl, phenoxy, benzyloxy, heteroaryloxy, methylenedioxy or ethylenedioxy.

27. A compound selected from:

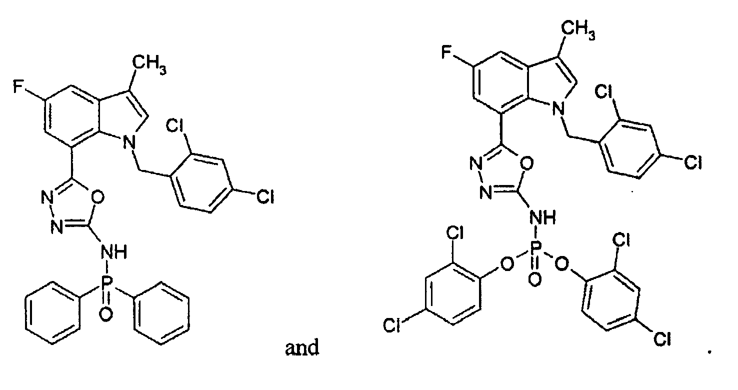

28. A compound according to claim 26 wherein the A/B ring system is an indole.

29. A compound according to claim 28 wherein M is aryl or substituted aryl.

30. A compound according to claim 28 wherein D is phenyl or oxadiazolyl.

31. A compound according to claim 30 wherein R¹ is chosen from phenyl, substituted phenyl, 5-membered ring heteroaryl, substituted 5-membered ring heteroaryl, CH₃ and CF₃.

## Patentansprüche

1. Verbindung der Formel 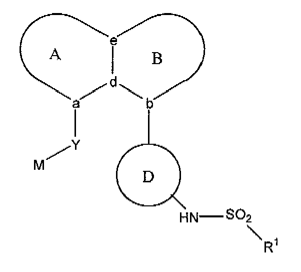 wobei
A und B für ein Paar kondensierte Ringe mit jeweils 5, 6 oder 7 Ringgliedern stehen, wobei genanntes kondensiertes A/B-Ringsystem null bis vier Heteroatome enthält, ausgewählt unter Stickstoff, Sauerstoff und Schwefel, und genannte Ringe zusätzlich durch null bis vier Substituenten substituiert sind, unabhängig ausgewählt unter Halogen, -OH, (C₁C₆)alkyl, (C₃-C₆)cycloalkyl, -O-(C₁-C₆)alkyl, -O-(C₃-C₆)cycloalkyl, Fluor(C₁-C₆)alkyl, Fluor(C₃-C₆)cycloalkyl, -O-(C₁-C₆)fluoralkyl, -O-(C₃-C₆)cyclofluoralkyl, Methylendioxy, Ethylendioxy, Alkoxy-(C₁-C₆)alkyl, Alkoxy-(C₃-C₈)cycloalkyl, Hydroxy(C₁-C₆)alkyl, Hydroxy(C₃-C₆)cycloalkyl, Oxo, Oxid, -CN, Nitro, -S-(C₁-C₆)alkyl, -S-(C₃-C₆)cycloalkyl, Amino, (C₁-C₆)alkylamino, (C₃-C₆)cycloalkylamino, Di(C₁-C₆)alkylamino, Di(C₃-C₆)cycloalkylamino, Di(C₁-C₆)alkylaminoalkyl, Di(C₁-C₆)alkylaminocycloalkyl, Di(C₃-C₆)cycloalkylaminoalkyl, Di(C₃-C₆)cycloalkylaminocycloalkyl, Carboxy, Carboalkoxy, Acyl, Carboxamido, (C₁-C₆)alkylsulfoxid, (C₃-C₆)cycloalkylsulfoxid, Acylamino, Phenyl, Benzyl, *spiro*thiazolidinyl, Phenoxy und Benzyloxy;
a und b für Bindungsstellen für Rest Y beziehungsweise D stehen und a und b in einer *peri-*Verbindung zueinander an genanntem kondensierten A/B-Ringsystem stehen;
d und e für Kondensationspunkte zwischen Ring A und Ring B in genanntem kondensierten A/B-Ringsystem stehen;
D ein Aryl- oder Heteroaryl-Ringsystem ist, wobei genanntes Ringsystem zusätzlich durch null bis vier Substituenten substituiert ist, unabhängig ausgewählt unter Halogen, -OH, (C₁-C₆)alkyl, (C₃-C₆)cycloalkyl, -O-(C₁-C₆)alkyl, -O-(C₃-C₆)cycloalkyl, Fluor(C₁-C₆)alkyl, Fluor(C₃-C₆)cycloalkyl, -O-(C₁-C₆)fluoralkyl, -O-(C₃-C₆)cyclofluoralkyl, Methylendioxy, Ethylendioxy, Alkoxy-(C₁-C₆)alkyl, Alkoxy-(C₃-C₅)cycloalkyl, Hydroxy(C₁-C₆)alkyl, Hydroxy(C₃-C₆)cycloalkyl, -CN, Nitro, -S-(C₁-C₆)alkyl, -S-(C₃-C₆)cycloalkyl, Amino, (C₁-C₆)alkylamino, (C₃-C₆)cycloalkylamino, Di(C₁-C₆)alkylamino, Di(C₃-C₆)cycloalkylamino, Di(C₁-C₆)alkylaminoalkyl, Di(C₁-C₆)alkylaminocycloalkyl, Di(C₃-C₆)cycloalkylaminoalkyl, Di(C₃-C₆)cycloalkylaminocycloalkyl, Carboxy, Carboalkoxy, Acyl, Carboxamido, (C₁-C₆)alkylsulfoxid, (C₃-C₆)cycloalkylsulfoxid, Acylamino, Phenyl, Benzyl, Phenoxy und Benzyloxy;
Y ausgewählt ist unter -CH₂-, -O-, -OCH₂-, -S-, -SO- und -SO₂-, wobei die linksseitige Bindung die Bindungsstelle an Ring A oder B anzeigt;
M ausgewählt ist unter Aryl, substituiertem Aryl, Heterocyclyl und substituiertem Heteroaryl;
R¹ ausgewählt ist unter Aryl, substituiertem Aryl, Heteroaryl, substituiertem Heteroaryl und CF₃; und
wenn Y -CH₂-, -O-, -S-, -SO- oder -SO₂- ist, R¹ zusätzlich (C₁-C₆)alkyl oder (C₃-C₆)cycloalkyl sein kann;
wobei in jedem substituierten Teil M oder R¹ bis zu drei H-Atome in jedem Rest durch Halogen, Niederalkyl, Haloalkyl, Hydroxy, Niederalkoxy, Carboxy, Alkoxycarbonyl, Alkylaminocarbonyl, Cyano, Carbonyl, Nitro, Amino, Alkylamino, Dialkylamino, Mercapto, Alkylthio, Sulfoxid, Sulfon, Acylamino, Amidino, Phenyl, Benzyl, Heteroaryl, Phenoxy, Benzyloxy, Heteroaryloxy, Methylendioxy oder Ethylendioxy ersetzt sind.

2. Verbindung nach Anspruch 1, wobei D Phenyl ist, substituiert durch null bis vier Substituenten.

3. Verbindung nach Anspruch 1 oder 2, wobei D Naphthyl ist, substituiert durch null bis vier Substituenten.

4. Verbindung nach einem der Anspruch 1 bis 3, wobei D monocyclisches Heteroaryl ist, substituiert durch null bis vier Substituenten.

5. Verbindung nach Anspruch 1, wobei D bicyclisches Heteroaryl ist, substituiert durch null bis vier Substituenten.

6. Verbindung nach Anspruch 1, wobei R¹ ausgewählt ist unter Phenyl, substituiertem Phenyl, 5-gliedrigem Ring-Heteroaryl, substituiertem 5-gliedrigen Ring-Heteroaryl und CF₃.

7. Verbindung nach Anspruch 1, wobei M ausgewählt ist unter Aryl, substituiertem Aryl, Heterocyclyl und substituiertem Heteroaryl.

8. Verbindung nach Anspruch 7, wobei M ausgewählt ist unter Phenyl, substituiertem Phenyl, Naphthyl, substituiertem Naphthyl, Heteroaryl und substituiertem Heteroaryl.

9. Verbindung nach Anspruch 1, wobei das A/B-Ringsystem ein kondensiertes Ringpaar mit jeweils 5 Ringgliedern ist:

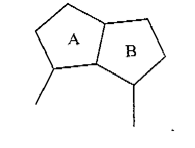

10. Verbindung nach Anspruch 1, wobei das A/B-Ringsystem ein Par kondensierte Ringe mit jeweils 6 Ringgliedern ist:

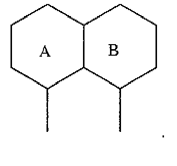

11. Verbindung nach Anspruch 1, wobei das A/B-Ringsystem ein Par kondensierte Ringe mit jeweils 5 und 6 Ringgliedern ist:

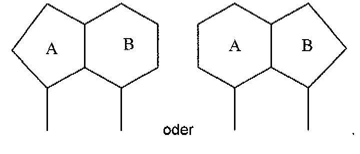

12. Verbindung nach Anspruch 11, wobei das A/B-Ringsystem ein Indol ist.

13. Verbindung nach Anspruch 1 zur Verwendung bei der Behandlung oder Prophylaxe von Prostaglandin-vermittelter Erkrankung oder Beschwerden in einem Säugetier.

14. Verbindung zur Verwendung nach Anspruch 13, wobei genannte Krankheit oder Beschwerden ausgewählt ist/sind unter Schmerzen, Fieber oder Entzündung in Verbindung mit rheumatischem Fieber, Influenza oder anderen Virusinfektionen, Erkältungskrankheit, Dysmenorrhoe, Kopfschmerzen, Migräne, Verstauchungen und Zerrungen, Myositis, Neuralgie, Synovitis, Arthritis, einschließlich rheumatoider Arthritis, degenerative Gelenkerkrankungen (Osteoarthritis), Gicht und Morbus Bechterew, Bursitis, Verbrennungen, einschließlich durch Strahlung und Verletzungen durch ätzende Chemikalien, Sonnenbrand, Immun- und Autoimmunkrankheiten;
neoplastische Transformationen von Zellen oder metastasierende Tumore;
diabetische Retinopathie, Tumorangiogenese;
Prostanoid-induzierte Kontraktionen der glatten Muskulatur in Verbindung mit Dysmenorrhoe, vorzeitige Wehen, Asthma oder durch Eosinophile ausgelöste Krankheiten; Morbus Alzheimer;
Glaukom;
Knochenverlust;
Osteoporose;
Morbus Paget:
peptisches Ulkus, Gastritis, regionale Enteritis, Colitis ulcerosa, Divertikulitis oder andere gastrointestinale Läsionen; gastrointestinale Blutungen;
Koagulationsstörungen ausgewählt unter Hypoprothrombinämie, Hämophilie und anderen Blutungsprobleme;
Nierenkrankheit;
Thrombose, Myokardinfarkt, Schlaganfall und
okklusive Gefäßkrankheit.

15. Verbindung zur Verwendung nach Anspruch 14, wobei es sich bei der genannten Krankheit um okklusive Gefäßkrankheit handelt.

16. Verbindung nach Anspruch 1 zur Verwendung für die Reduktion von Plaque bei der Behandung von Atherosklerose in einem Säugetier.

17. Verbindung nach Anspruch 1 zur Verwendung bei der Förderung von Knochenbildung oder zur Verwendung für den Zellschutz in einem Säugetier.

18. Verbindung nach Anspruch 1 zur Verwendung bei der Behandlung oder Prophylaxe von Schmerzen, Entzündung, Atherosklerose, Myokardinfarkt, Schlaganfall oder okklusiver Gefäßerkrankung in einem Säugetier.

19. Pharmazeutische Zusammensetzung umfassend einen pharmazeutisch akzeptablen Trägerstoff und eine Verbindung nach Anspruch 1.

20. Pharmazeutische Formulierung nach Anspruch 19 umfassend ein zusätzliches therapeutisches Mittel, ausgewählt unter einem Plättchenaggregationshemmer, einem HMG-CoA-Reduktasehemmer, einem Lipidsenker und einem Cyclooxygenasehemmer.

21. Pharmazeutische Formulierung nach Anspruch 20, wobei genannter Plättchenaggregationshemmer ausgewählt ist unter Tirofiban, Dipyridamol, Clopidogrel und Ticlopidin.

22. Pharmazeutische Formulierung nach Anspruch 20, wobei genannter HMG-CoA-Reduktasehemmer ausgewählt ist unter Lovastatin, Simvastatin, Pravastatin, Rosuvastatin, Mevastatin, Atorvastatin, Cerivastatin, Pitavastatin und Fluvastatin.

23. Pharmazeutische Formulierung nach Anspruch 20, wobei genannter Cyclooxygenasehemmer ausgewählt ist unter Rofecoxib, Meloxicam, Celecoxib, Etoricoxib, Lumiracoxib, Valdecoxib, Parecoxib, Cimicoxib, Diclofenac, Sulindac, Etodolac, Ketoralac, Ketoprofen, Piroxicam und LAS-34475.

24. Verfahren zum Screening auf selektive Prostanoidrezeptorliganden umfassend das in Kontakt bringen einer markierten Verbindung nach Anspruch 1 mit einem Prostanoidrezeptor und das Ermitteln der Verdrängung desselben durch eine Testverbindung.

25. Verfahren nach Anspruch 24 zum Screening auf selektive EP₃-Liganden umfassend das in Kontakt bringen einer markierten Verbindung mit einem geklonten humanen EP₃-Rezeptor und das Ermitteln der Verdrängung desselben durch eine Testverbindung.

26. Verbindung der Formel 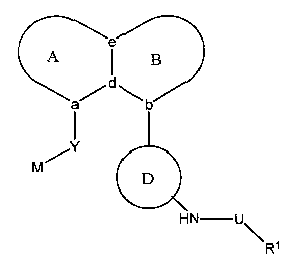 wobei
A und B für ein Paar kondensierte Ringe mit jeweils 5, 6 oder 7-Ringgliedern stehen, wobei genanntes kondensiertes A/B-Ringsystem null bis vier Heteroatome enthält, ausgewählt unter Stickstoff, Sauerstoff und Schwefel, und genannte Ringe zusätzlich substituiert sind durch null bis vier Substituenten, unabhängig ausgewählt unter Halogen, -OH, (C₁-C₆)alkyl, (C₉-C₆)cycloalkyl, -O-(C₁-C₆)alkyl, -O-(C₃-C₆)cycloalkyl, Fluor(C₁-C₆)alkyl, Fluor(C₃-C₆)cycloalkyl, -O-(C₁-C₆)fluoralkyl, -O-(C₃-C₆)cyclofluoralkyl, Methylendioxy, Ethylendioxy, Alkoxy-(C₁-C₆)alkyl, Alkoxy-(C₃-C₆)cycloalkyl, Hydroxy(C₁-C₆)alkyl, Hydroxy(C₃-C₆)cycloalkyl, Oxo, Oxid, -CN, Nitro, -S-(C₁-C₆)alkyl, -S-(C₃-C₆)cycloalkyl, Amino, (C₁-C₆)alkylamino, (C₃-C₆)cycloalkylamino, Di(C₁-C₆)alkylamino, Di(C₃-C₆)cycloalkylamino, Di(C₁-C₆)alkylaminoalkyl, Di(C₁-C₆)alkylaminocycloalkyl, Di(C₃-C₆)cycloalkylaminoalkyl, Di(C₃-C₆)cycloalkylaminocycloalkyl, Carboxy, Carboalkoxy, Acyl, Carboxamido, (C₁-C₆)alkylsulfoxid, (C₃-C₆)cycloalkylsulfoxid, Acylamino, Phenyl, Benzyl, S*piro*thiazolidinyl, Phenoxy und Benzyloxy;
a und b für Bindungsstellen von Rest Y beziehungsweise D stehen und a und b in einer *peri-*Verbindung zueinander an genanntem kondensierten A/B-Ringsystem stehen;
d und e für Kondensationspunkte zwischen Ring A und Ring B in genanntem kondensiertem A/B-Ringsystem stehen;
U C=O ist;
D ein Aryl- oder Heteroaryl-Ringsystem ist, wobei genanntes Ringsystem zusätzlich substituiert ist durch null bis vier Substituenten, unabhängig ausgewählt unter Halogen, - OH, (C₁-C₆)alkyl, (C₃-C₆)cycloalkyl, -O-(C₁-C₆)alkyl, -O-(C₃-C₆)cycloalkyl, Fluor(C₁-C₆)alkyl, Fluor(C₃-C₅)cycloalkyl, -O-(C₁-C₆)fluoralkyl, -O-(C₃-C₆)cyclofluoralkyl, Methylendioxy, Ethylendioxy, Alkoxy-(C₁-C₆)alkyl, Alkoxy-(C₃-C₆)cycloalkyl, Hydroxy(C₁-C₆)alkyl, Hydroxy(C₃-C₆)cycloalkyl, -CN, Nitro, -S-(C₁-C₆)alkyl, -S-(C₃-C₆)cycloalkyl, Amino, (C₁-C₆)alkylamino, (C₃-C₆)cycloalkylamino, Di(C₁-C₆)alkylamino, Di(C₃-C₆)cycloalkylamino, Di(C₁-C₆)alkylaminoalkyl, Di(C₁-C₆)alkylaminocycloalkyl, Di(C₃-C₆)cycloalkylaminoalkyl, Di(C₃-C₆)cycloalkylaminocycloalkyl, Carboxy, Carboalkoxy, Acyl, Carboxamido, (C₁-C₆)alkylsulfoxid, (C₃-C₆)cycloalkylsulfoxid, Acylamino, Phenyl, Benzyl, Phenoxy und Benzyloxy;
Y -CH₂- ist;
M ausgewählt ist unter Aryl, substituiertem Aryl, Heterocyclyl und substituiertem Heteroaryl;
R¹ ausgewählt ist unter Aryl, substituiertem Aryl, Heteroaryl, substituiertem Heteroaryl, CF₃, (C₁-C₆)alkyl und (C₃-C₆)cycloalkyl;
wobei in jeder substituierten Teil M oder R¹ bis zu drei H-Atome in jedem Rest durch Halogen, Niederalkyl, Haloalkyl, Hydroxy, Niederalkoxy, Carboxy, Alkoxycarbonyl, Alkylaminocarbonyl, Cyano, Carbonyl, Nitro, Amino, Alkylamino, Dialkylamino, Mercapto, Alkylthio, Sulfoxid, Sulfon, Acylamino, Amidino, Phenyl, Benzyl, Heteroaryl, Phenoxy, Benzyloxy, Heteroaryloxy, Methylendioxy oder Ethylendioxy ersetzt sind.

27. Verbindung ausgewählt unter:

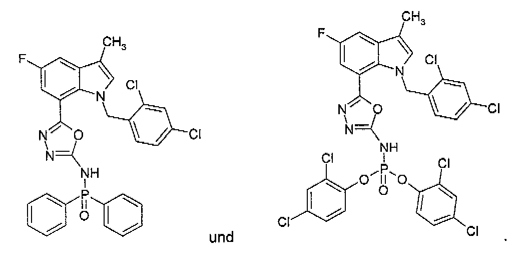

28. Verbindung nach Anspruch 26, wobei das A/B-Ringsystem ein Indol ist.

29. Verbindung nach Anspruch 28, wobei M Aryl oder substituiertes Aryl ist.

30. Verbindung nach Anspruch 28, wobei D Phenyl oder Oxadiazolyl ist.

31. Verbindung nach Anspruch 30, wobei R¹ ausgewählt ist unter Phenyl, substituiertem Phenyl, 5-gliedrigem Ring-Heteroaryl, substituiertem 5-gliedrigem Ring-Heteroaryl, CH₃ und CF₃.

## Revendications

1. Composé de formule 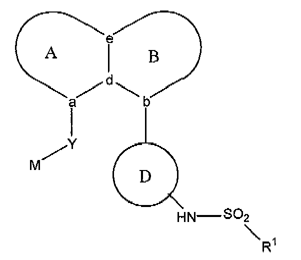 dans lequel
A et B représentent une paire de noyaux à 5, 6 ou 7 chaînons fusionnés, ledit système de noyaux A/B fusionnés contenant de zéro à quatre hétéroatomes sélectionnés parmi l'azote, l'oxygène et le soufre et lesdits noyaux étant de plus substitués par de zéro à quatre substituants indépendamment sélectionnés parmi les suivants : halogène, -OH, alkyle en (C₁-C₆), cycloalkyle en (C₃-C₆), -O-alkyle en (C₁-C₆), -O-cycloalkyle en (C₃-C₆), fluoroalkyle en (C₁-C₆), fluorocycloalkyle en (C₃-C₆), -O-fluoroalkyle en (C₁-C₆), -O-cyclofluoroalkyle en (C₃-C₆), méthylènedioxy, éthylenèdioxy, alkoxy-alkyle en (C₁-C₆), alkoxy-cycloalkyle en (C₃-C₆), hydroxyalkyle en (C₁-C₆), hydroxycycloalkyle en (C₃-C₆), oxo, oxyde, -CN, nitro, -S-alkyle en (C₁-C₆), -S-cycloalkyle en (C₃-C₆), amino, aminoalkyle en (C₁-C₆), aminocycloalkyle en (C₃-C₆), di[aminoalkyle en (C₁-C₆)], di[aminocycloalkyle en (C₃-C₆)], di[alkyle en (C₁-C₆)-aminoalkyle], di[alkyle en (C₁-C₆)-aminocycloalkyle], di[cycloalkyle en (C₃-C₆)-aminoalkyle], di[cycloalkyle en (C₃-C₆)-aminocycloalkyle], carboxy, carboalkoxy, acyle, carboxamido, alkyle en (C₁-C₆)-sulfoxyde, cycloalkyle en (C₃-C₆)-sulfoxyde, acylamino, phényle, benzyle, *spiro*thiazolidinyle, phénoxy et benzyloxy ;
a et b représentent des points d'attache des résidus Y et D, respectivement, et a et b sont en *peri* l'un par rapport à l'autre sur ledit système de noyaux A/B fusionnés ;
d et e représentent des points de fusion entre le noyau A et le noyau B dudit système de noyaux A/B fusionnés ;
D est un système aryle ou hétéroraryle cyclique, ledit système étant de plus substitué par de zéro à quatre substituants indépendamment sélectionnés parmi les suivants : halogène, - OH, alkyle en (C₁-C₆), cycloalkyle en (C₃-C₆), -O-alkyle en (C₁-C₆), -O-cycloalkyle en (C₃-C₆), fluoroalkyle en (C₁-C₆), fluorocycloalkyle en (C₃-C₆), -O-fluoroalkyle en (C₁-C₆), -O-cyclofluoroalkyle en (C₃-C₆), méthylènedioxy, éthylenèdioxy, alkoxy-alkyle en (C₁-C₆), alkoxy-cycloalkyle en (C₃-C₆), hydroxyalkyle en (C₁-C₆), hydroxycycloalkyle en (C₃-C₆), -CN, nitro, -S-alkyle en (C₁-C₆), -S-cycloalkyle en (C₃-C₆), amino, aminoalkyle en (C₁-C₆), aminocycloalkyle en (C₃-C₆), di[aminoalkyle en (C₁-C₆)], di[aminocycloalkyle en (C₃-C₆)], di[alkyle en (C₁-C₆)-aminoalkyle], di[alkyle en (C₁-C₆)-aminocycloalkyle], di[cycloalkyle en (C₃-C₆)-aminoalkyle], di[cycloalkyle en (C₃-C₆)-aminocycloalkyle], carboxy, carboalkoxy, acyle, carboxamido, alkyle en (C₁-C₆)-sulfoxyde, cycloalkyle en (C₃-C₆)-sulfoxyde, acylamino, phényle, benzyle, phénoxy et benzyloxy ;
Y est sélectionné parmi les suivants : -CH₂-, -O-, -OCH₂-, -S-, -SO- et -SO₂-, dans lequel la liaison de gauche indique le point d'attache au noyau A ou B ;
M est sélectionné parmi aryle, aryle substitué, hétérocyclyle et hétéroaryle substitué ;
R¹ est sélectionné parmi aryle, aryle substitué, hétéroaryle, hétéroaryle substitué et CF₃ ; et quand Y est -CH₂-, -O-, -S-, -SO- ou -SO₂-, R¹ peut également être alkyle en (C₁-C₆) ou un cycloalkyle en (C₃-C₆) ;
dans lequel, dans chaque fragment M ou R¹ substitué, jusqu'à trois des atomes d'hydrogène sur chaque résidu sont remplacés par halogène, alkyle inférieur, haloalkyle, hydroxy, alkoxy inférieur, carboxy, alkoxycarbonyle, alkylaminocarbonyle, cyano, carbonyle, nitro, amino, alkylamino, dialkylamino, mercapto, alkylthio, sulfoxyde, sulfone, acylamino, amidino, phényle, benzyle, hétéroaryle, phénoxy, benzyloxy, hétéroaryloxy, méthylènedioxy ou éthylènedioxy.

2. Composé selon la revendication 1, dans lequel D est phényle substitué par de zéro à quatre substituants.

3. Composé selon la revendication 1 ou 2, dans lequel D est naphtyle substitué par de zéro à quatre substituants.

4. Composé selon l'une quelconque des revendications 1 à 3, dans lequel D est hétéroaryle monocyclique substitué par de zéro à quatre substituants.

5. Composé selon la revendication 1, dans lequel D est hétéroaryle bicyclique substitué par de zéro à quatre substituants.

6. Composé selon la revendication 1, dans lequel R¹ est sélectionné parmi phényle, phényle substitué, hétéroaryle cyclique à 5 chaînons, hétéroaryle cyclique à 5 chaînons substitué et CF₃.

7. Composé selon la revendication 1, dans lequel M est sélectionné parmi aryle, aryle substitué, hétérocyclyle et hétéroaryle substitué.

8. Composé selon la revendication 7, dans lequel M est sélectionné parmi phényle, phényle substitué, naphtyle, naphtyle substitué, hétéroaryle et hétéroaryle substitué.

9. Composé selon la revendication 1, dans lequel le système de noyaux A/B est une paire de noyaux à 5 chaînons fusionnés :

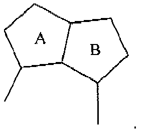

10. Composé selon la revendication 1, dans lequel le système de noyaux A/B est une paire de noyaux à 6 chaînons fusionnés :

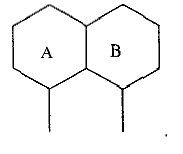

11. Composé selon la revendication 1, dans lequel le système de noyaux A/B est une paire de noyaux à 5 et 6 chaînons fusionnés :

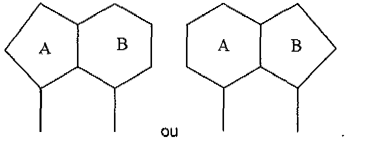

12. Composé selon la revendication 11, dans lequel le système de noyaux A/B est un indole.

13. Composé selon la revendication 1, pour un usage dans le traitement ou la prophylaxie d'une maladie ou affection sous la médiation de prostaglandines chez un mammifère.

14. Composé pour un usage selon la revendication 13, dans lequel ladite maladie ou affection est sélectionnée parmi les suivantes :
douleur, fièvre ou inflammation associée à un rhumatisme articulaire aigu, la grippe ou d'autres infections virales, rhume banal, dysménorrhée, céphalées, migraine, entorses et foulures, myosite, névralgie, synovite, arthrite, y compris la polyarthrite rhumatoïde, affections articulaires dégénératives (arthrose), goutte et spondylarthrite ankylosante, bursite, brûlures, y compris les lésions dues à des irradiations et à des produits chimiques corrosifs, érythèmes solaires, maladies immunes et autoimmunes ;
transformations cellulaires néoplasiques ou croissance tumorale métastique ;
rétinopathie diabétique, angiogenèse tumorale ;
contractions des muscles lisses induites par des prostanoïdes et associées à une dysménorrhée, un déclenchement prématuré du travail, l'asthme ou des maladies à éosinophiles ;
maladie d'Alzheimer ;
glaucome ;
déperdition osseuse ;
ostéoporose ;
maladie de Paget ;
ulcères gastroduodénaux, gastrite, entérite régionale, rectocolite hémorragique, diverticulite ou autres lésions gastro-intestinales ; hémorragie digestive ;
troubles de la coagulation sélectionnés parmi l'hypoprothrombinémie, l'hémophilie et d'autres troubles hémorragiques ;
maladie rénale ;
thrombose, infarctus du myocarde, accident vasculaire cérébral ; et
maladie vasculaire occlusive.

15. Composé pour un usage selon la revendication 14, dans lequel ladite maladie est une maladie vasculaire occlusive.

16. Composé selon la revendication 1, pour un usage visant à réduire la plaque dans le traitement de l'athérosclérose chez un mammifère.

17. Composé selon la revendication 1, pour un usage visant à favoriser la formation osseuse ou la cytoprotection chez un mammifère.

18. Composé selon la revendication 1 pour un usage dans le traitement ou la prophylaxie de la douleur, de l'inflammation, de l'athérosclérose, d'un infarctus du myocarde, d'un accident vasculaire cérébral ou d'une maladie vasculaire occlusive chez un mammifère.

19. Composition pharmaceutique qui comprend un véhicule pharmaceutiquement acceptable et un composé selon la revendication 1.

20. Formulation pharmaceutique selon la revendication 19, qui comprend un agent thérapeutique additionnel sélectionné parmi un antiagrégant plaquettaire, un inhibiteur de la HMG-CoA-réductase, un antihyperlipidémiant ou un inhibiteur de la cyclo-oxygénase.

21. Formulation pharmaceutique selon la revendication 20, dans lequel ledit antiagrégant plaquettaire est sélectionné parmi le tirofiban, le dipyridamole, le clopidogrel et la ticlopidine.

22. Formulation pharmaceutique selon la revendication 20, dans lequel ledit inhibiteur de la HMG-CoA-réductase est sélectionné parmi la lovastatine, la simvastatine, la pravastatine, la rosuvastatine, la mévastatine, l'atorvastatine, la cérivastatine, la pitavastatine et la fluvastatine.

23. Formulation pharmaceutique selon la revendication 20, dans lequel ledit inhibiteur de la cyclo-oxygénase est sélectionné parmi le rofécoxib, le méloxicam, le célécoxib, l'étoricoxib, le lumiracoxib, le valdécoxib, le parécoxib, le cimicoxib, le diclofénac, le sulindac, l'étodolac, le kétorolac, le kétoprofène, le piroxicam et LAS-34475.

24. Méthode de triage de ligands sélectifs des récepteurs aux prostanoïdes, qui comprend la mise en contact d'un composé marqué selon la revendication 1 avec un récepteur aux prostanoïdes et la mesure de son déplacement par un composé à l'essai.

25. Méthode selon la revendication 24 pour le triage de ligands sélectifs des récepteurs de type EP₃, qui comprend la mise en contact d'un composé marqué avec un récepteur de type EP₃ humain cloné et la mesure de son déplacement par un composé à l'essai.

26. Composé de formule 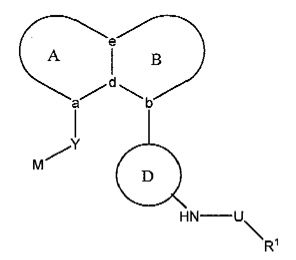 dans lequel
A et B représentent une paire de noyaux à 5, 6 ou 7 chaînons fusionnés, ledit système de noyaux A/B fusionnés contenant de zéro à quatre hétéroatomes sélectionnés parmi l'azote, l'oxygène et le soufre et lesdits noyaux étant de plus substitués par de zéro à quatre substituants indépendamment sélectionnés parmi les suivants : halogène, -OH, alkyle en (C₁-C₆), cycloalkyle en (C₃-C₆), -O-alkyle en (C₁-C₆), -O-cycloalkyle en (C₃-C₆), fluoroalkyle en (C₁-C₆), fluorocycloalkyle en (C₃-C₆), -O-fluoroalkyle en (C₁-C₆), -O-cyclofluoroalkyle en (C₃-C₆), méthylènedioxy, éthylenèdioxy, alkoxy-alkyle en (C₁-C₆), alkoxy-cycloalkyle en (C₃-C₆), hydroxyalkyle en (C₁-C₆), hydroxycycloalkyle en (C₃-C₆), oxo, oxyde, -CN, nitro, -S-alkyle en (C₁-C₆), -S-cycloalkyle en (C₃-C₆), amino, aminoalkyle en (C₁-C₆), aminocycloalkyle en (C₃-C₆), di[aminoalkyle en (C₁-C₆)], di[aminocycloalkyle en (C₃-C₆)], di[alkyle en (C₁-C₆)-aminoalkyle], di[alkyle en (C₁-C₆)-aminocycloalkyle], di[cycloalkyle en (C₃-C₆)-aminoalkyle], di[cycloalkyle en (C₃-C₆)-aminocycloalkyle], carboxy, carboalkoxy, acyle, carboxamido, alkyle en (C₁-C₆)-sulfoxyde, cycloalkyle en (C₃-C₆)-sulfoxyde, acylamino, phényle, benzyle, *spiro*thiazolidinyle, phénoxy et benzyloxy ;
a et b représentent des points d'attache des résidus Y et D, respectivement, et a et b sont en *peri* l'un par rapport à l'autre sur ledit système de noyaux A/B fusionnés ;
d et e représentent des points de fusion entre le noyau A et le noyau B dudit système de noyaux A/B fusionnés ;
U est C=O ;
D représente un système aryle ou hétéroraryle cyclique, ledit système étant de plus substitué par de zéro à quatre substituants indépendamment sélectionnés parmi les suivants : halogène, -OH, alkyle en (C₁-C₆), cycloalkyle en (C₃-C₆), -O-alkyle en (C₁-C₆), -O-cycloalkyle en (C₃-C₆), fluoroalkyle en (C₁-C₆), fluorocycloalkyle en (C₃-C₆), -O-fluoroalkyle en (C₁-C₆), -O-cyclofluoroalkyle en (C₃-C₆), méthylènedioxy, éthylenèdioxy, alkoxy-alkyle en (C₁-C₆), alkoxy-cycloalkyle en (C₃-C₆), hydroxyalkyle en (C₁-C₆), hydroxycycloalkyle en (C₃-C₆), -CN, nitro, -S-alkyle en (C₁-C₆), -S-cycloalkyle en (C₃-C₆), amino, aminoalkyle en (C₁-C₆), aminocycloalkyle en (C₃-C₆), di[aminoalkyle en (C₁-C₆)], di[aminocycloalkyle en (C₃-C₆)], di[alkyle en (C₁-C₆)-aminoalkyle], di[alkyle en (C₁-C₆)-aminocycloalkyle], di[cycloalkyle en (C₃-C₆)-aminoalkyle], di[cycloalkyle en (C₃-C₆)-aminocycloalkyle], carboxy, carboalkoxy, acyle, carboxamido, alkyle en (C₁-C₆)- sulfoxyde, cycloalkyle en (C₃-C₆)-sulfoxyde, acylamino, phényle, benzyle, phénoxy et benzyloxy ;
Y est -CH₂- ;
M est sélectionné parmi aryle, aryle substitué, hétérocyclyle et hétéroaryle substitué ;
R¹ est sélectionné parmi aryle, aryle substitué, hétéroaryle, hétéroaryle substitué, CF₃, alkyle en (C₁-C₆) et cycloalkyle en (C₃-C₆) ;
dans lequel, dans chaque fragment M ou R¹ substitué, jusqu'à trois des atomes d'hydrogène sur chaque résidu sont remplacés par halogène, alkyle inférieur, haloalkyle, hydroxy, alkoxy inférieur, carboxy, alkoxycarbonyle, alkylaminocarbonyle, cyano, carbonyle, nitro, amino, alkylamino, dialkylamino, mercapto, alkylthio, sulfoxyde, sulfone, acylamino, amidino, phényle, benzyle, hétéroaryle, phénoxy, benzyloxy, hétéroaryloxy, méthylènedioxy ou éthylènedioxy.

27. Composé sélectionné entre :

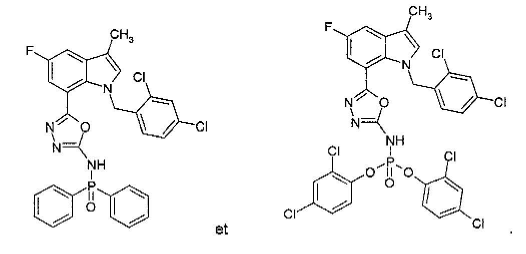

28. Composé selon la revendication 26, dans lequel le système de noyaux A/B est un indole.

29. Composé selon la revendication 28, dans lequel M est aryle ou aryle substitué.

30. Composé selon la revendication 28, dans lequel D est phényle ou oxadiazolyle.

31. Composé selon la revendication 30, dans lequel R¹ est sélectionné parmi phényle, phényle substitué, hétéroaryle cyclique à 5 chaînons, hétéroaryle cyclique à 5 chaînons substitué, CH₃ et CF₃.
